# EUROPEAN PATENT SPECIFICATION

(11) **EP 2 010 496 B1**
(45) Date of publication and mention of the grant of the patent: **25.08.2010**
(21) Application number: 07732381.4
(22) Date of filing: 12.04.2007
(51) Int. Cl.: C07D 215/54

(54) **4-ANILINOQUINOLINE-3-CARBOXAMIDES AS CSF-1R KINASE INHIBITORS**
4-ANILINOCHINOLIN-3-CARBONSÄUREAMIDE ALS CSF-1R-KINASEINHIBITOREN
4-ANILINOQUINOLEINE-3-CARBOXAMIDES EN TANT QU'INHIBITEURS DE LA CSF-IR KINASE

(30) Priority: 14.04.2006 US 744857 P; 09.11.2006 US 865090 P
(43) Date of publication of application: 07.01.2009
(73) Proprietor: AstraZeneca AB, 151 85 Södertälje (SE)
(72) Inventor: COOK, Donald, Waltham, Massachusetts 02451 (US); DAKIN, Leslie, Waltham, Massachusetts 02451 (US); DEL VALLE, David, Austin, TX 78759 (US); GERO, Thomas, Waltham, Massachusetts 02451 (US); SCOTT, David, Waltham, Massachusetts 02451 (US); ZHENG, Xiaolan, Waltham, Massachusetts 02451 (US)
(86) International application number: PCT/GB2007/001338
(87) International publication number: WO 2007/119046

(56) References cited:
- EP-A1- 1 535 910
- WO-A-95/15758
- WO-A-02/092571
- WO-A-2005/034955
- WO-A1-2005/075429

## Description

### BACKGROUND OF THE INVENTION

The invention relates to chemical compounds, or pharmaceutically acceptable salts thereof, which possess colony stimulating factor 1 receptor (CSF-1R) kinase inhibitory activity and are accordingly useful for their anti-cancer activity and thus in methods of treatment of the human or animal body. The invention also relates to processes for the manufacture of said chemical compounds, to pharmaceutical compositions containing them and to their use in the manufacture of medicaments of use in the production of an anti-cancer effect in a warm-blooded animal such as man.

Receptor tyrosine kinases (RTK's) are a sub-family of protein kinases that play a critical role in cell signalling and are involved in a variety of cancer related processes including cell proliferation, survival, angiogenesis, invasion and metastasis. There are believed to be at least 96 different RTK's including CSF-1R.

CSF-1R or c-fms was originally identified as the oncogene v-fms from the feline sarcoma virus. CSF-1R is a member of the class III RTK's along with c-Kit, fms-related tyrosine kinase 3 (Flt3) and Platelet-derived growth factor receptor α and β (PDGFRα and PDGFRβ). All of these kinases have been implicated in the process of tumorigenesis. CSF-1R is normally expressed as an immature 130 kDa transmembrane protein and ultimately results in a mature 145-160 kDa cell surface N-linked glycosylated protein. Macrophage colony stimulating factor (M-CSF or CSF-1), the ligand for CSF-1R, binds to the receptor resulting in dimerization, auto-phosphorylation of the receptor and subsequent activation of downstream signal transduction cascades (C.J. Sherr, Biochim Biophys Acta, 1988, 948: 225-243).

CSF-1R is normally expressed in myeloid cells of the mononuclear phagocytic lineage and their bone-marrow progenitors as well as the epithelial cells of the ducts and alveoli in the lactating, but not normal resting, breast tissue. CSF-1R activation stimulates the proliferation, survival, motility and differentiation of cells of the monocyte/macrophage lineage. The mature macrophage plays a key role in normal tissue development and immune defence (F.L. Pixley and E.R. Stanley, Trends in Cell Biology, 2004, 14(11): 628-638). For example, osteoblasts secrete CSF-1 and activate the receptor on osteoclastic progenitors resulting in differentiation into mature osteoclasts (S.L. Teitelbaum, Science, 2000, 289: 1504-1508). The CSF-1R axis plays an important role in placental development, embryonic implantation, mammary gland ductal and lobuloalveolar development and lactation (E. Sapi, Exp Biol Med, 2004,229:1-11).

Transfection of CSF-1R with or without CSF-1 induces transformation and *in vivo* tumorigenicity of NIH3T3 (Rat2 and ovarian granulosa cells. Autocrine and/or paracrine signaling mechanisms have been implicated in the activation of CSF-1R in the tumour epithelium and tumour associated macrophage. Aberrant expression and activation of CSF-1R and/or its ligand have been found in human myeloid leukaemia, prostate, breast, ovarian, endometrial and a variety of other cancers. A number of studies have demonstrated that the overexpression of CSF-1R is associated with poor prognosis in several of these cancers. In addition, the CSF-1/CSF-1R axis plays a key role in the regulation of tumour-associated macrophage, which have been postulated to play a significant role in tumour angiogenesis, invasion and progression (E. Sapi, Exp Biol Med, 2004, 229:1-11).

### SUMMARY AND DETAILED DESCRIPTION OF THE INVENTION

Accordingly, the present invention provides a compound of formula IA or IB: 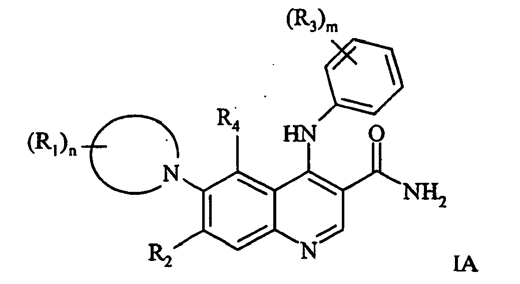 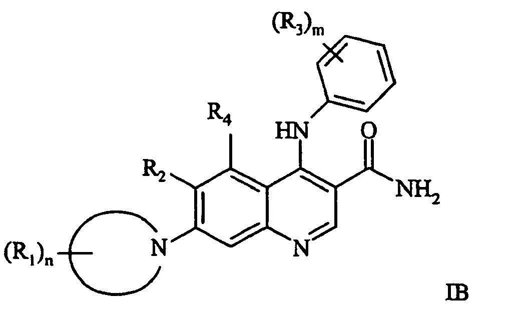 or a pharmaceutically acceptable salt thereof, wherein: 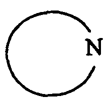 is a 3-10 membered, nitrogen linked, heterocycle or heteroaryl; wherein if said heterocycle or heteroaryl contains an -NH- moiety that nitrogen may be optionally substituted by a group selected from R₅;
**R₁** at each occurrence is independently halo, hydroxy, nitro, formyl, cyano, -CO₂H, -SH, (C₁-C₆)alkyl, (C₂-C₆)alkenyl, (C₂-C₆)alkynyl, (C₃-C₆)cycloalkyl, (C₁-C₆)alkoxy, -OC(O)-(C₁-C₆)akyl, -C(O)-(C₁-C₆)alkyl, -CO₂(C₁-C₆)alkyl, -NR'R", -NR'-C(O)-(C₁-C₆)alkyl, (C₁-C₆)alkylS(O)ₐ- wherein a is 0 to 2, -NR'-C(O)-O(C₁-C₆)alkyl, -NR'-SO₂-(C₁-C₆)alkyl, -NR'-C(O)NR'R", -SO₂-NR'R", -C(O)-NR'R", carbocyclyl, heterocyclo, heteroaryl or oxo;
**R₂** is hydrogen, halo, hydroxy, nitro, formyl, -CO₂H, -SH, cyano, (C₁-C₆)alkyl, (C₂-C₆)alkenyl, (C₂-C₆)alkynyl, (C₃-C₆)cycloalkyl, -O-(C₃-C₆)cycloalkyl, -OC(O)-(C₁-C₆)alkyl, -C(O)-(C₁-C₆)alkyl, -CO₂(C₁-C₆)alkyl, -NR'R", -NR'-C(O)-(C₁-C₆)alkyl, (C₁-C₆)alkylS(O)ₐ- wherein a is 0 to 2, -NR'-C(O)-O(C₁-C₆)allkyl, -NR'-SO₂-(C₁-C₆)alkyl, -NR'-C(O)NR'R", -SO₂-NR'R", -C(O)-NR'R", -OC(O)-NR'R", carbocyclyl, heterocyclo, heteroaryl or (C₁-C₆)alkoxy;
**R₃** at each occurrence is independently halo, nitro, formyl, cyano, hydroxy, -NR'R", -CO₂H, -C(O)-(C₁-C₆)alkyl, -CO₂(C₁-C₆)alkyl, -C(O)-NR'R", -NR'-C(O)-(C₁-C₆)alkyl, -N'R'-C(O)NR'R", -NR'-C(O)-O(C₁-C₆)alkyl, -O-C(O)-(C₁-C₆)alkyl, -SH, -SO₂-NR'R", (C₁-C₆)alkyl, (C₂-C₆)alkenyl, (C₂-C₆)alkynyl, (C₁-C₆)alkoxy, (C₁-C₆)alkylS(O)ₐ- wherein a is 0 to 2, -NR'-SO₂-(C₁-C₆)alkyl, carbocyclyl, heterocyclo, or heteroaryl, wherein if said heterocyclo or heteroaryl contains an -NH- moiety that nitrogen may be optionally substituted by (C₁-C₆)alkyl; or
two R₃ groups on adjacent carbons may optionally form a 5- or 6-membered saturated, partially unsaturated, unsaturated and/or aromatic ring optionally containing 0, 1, or 2 heteroatoms selected from S, O, or NRₐ wherein Rₐ is absent, H or (C₁-C₆)alkyl;
**R₄** is hydrogen or halo;
**m** is 0-3; wherein the values of R₃ may be the same or different;
**n** is 0-3; wherein the values of R₁ may be the same or different;
**p** is independently 1 or 2 at each occurrence; and
**R₅** is selected from (C₁-C₆)alkyl, (C₃-C₆)cycloalkyl, -C(O)-(C₁-C₆)alkyl, -S(O)ₚ(C₁-C₆)alkyl, -CO₂(C₁-C₆)alkyl, -C(O)-NR'R", benzyl, benzyloxycarbonyl, benzoyl and phenylsulphonyl;
**R'** and **R"** independently at each occurrence are H, optionally substituted (C₁-C₆)alkyl, or optionally substituted aryl, or taken together with the nitrogen to which they are attached form an optionally substituted 3-6 membered ring saturated or partially unsaturated containing 0 or 1 additional heteroatom selected from NRₐ; wherein said optional substituents may be selected from one or more R₆;
**R₆** may be independently (C₁-C₆)alkyl, halo(C₁-C₆)alkyl, halo, nitro, cyano, hydroxy, (C₁-C₆)alkoxy, -NR^{x}R^{y}, -COOR^{x} or -CONR^{x}R^{y}; and
**R^{x}** and **R^{y}** are independently of each other hydrogen or (C₁-C₆)alkyl; and wherein
each **Rₐ, R₁, R₂, R₃** and **R₅** may be optionally substituted on carbon by one or more formyl, -SH, azido, halo, nitro, cyano, hydroxy, trifluoromethoxy, -OC(O)-(C₁-C₆)alkyl, -NR'R", -CO₂H, -C(O)-(C₁-C₆)alkyl, -CO₂(C₁-C₆)alkyl, -C(O)-NR'R", -S-(C₁-C₆)alkyl, -SOₚ-(C₁-C₆)alkyl, -SOₚNR'R", (C₁-C₆)alkyl, (C₃-C₆)cycloalkyl, -NR'-C(O)-(C₁-C₆)alkyl, -NR'-C(O)-O(C₁-C₆)alkyl, -NR'-SO₂-(C₁-C₆)alkyl, -NR'-C(O)NR'R", (C₂-C₆)alkenyl, (C₂-C₆)alkynyl, or (C₁-C₆)alkoxy.

According to a further aspect of the present invention there is provided a compound of formula IA or IB or a pharmaceutically acceptable salt thereof, wherein: 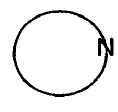 is a 3-8 membered heterocycle or heteroaryl;
**R₁** at each occurrence is independently halo, hydroxyl, (C₁-C₆)alkyl, (C₃-C₆)cycloalkyl, (C₁-C₆)alkoxy, -OC(O)-(C₁-C₆)alkyl, -C(O)-(C₁-C₆)alkyl, -CO₂(C₁-C₆)alkyl, -C(O)-NR'R", or oxo;
**R₂** is hydrogen, halo, hydroxyl, cyano, (C₁-C₆)alkyl, (C₃-C₆)cycloalkyl, or (C₁-C₆)alkoxy;
**R₃** at each occurrence is independently halo, nitro, cyano, hydroxy, trifluoromethoxy, NR'R", CO₂H, -C(O)-(C₁-C₆)alkyl, -CO₂(C₁-C₆)alkyl, -C(O)-NR'R", -NR-C(O)-(C₁-C₆)alkyl, -NR'-C(O)NR'R", -NR'-C(O)-O(C₁-C₆)alkyl, -O-C(O)-(C₁-C₆)alkyl, SH, -SO₂₋NR'R", (C₁-C₆)alkyl, (C₂-C₆)alkenyl, (C₂-C₆)alkynyl, (C₁-C₆)alkoxy, (C₁-C₆)alkylS(O)ₐ wherein a is 0 to 2, -NR'-SO₂-(C₁-C₆)alkyl, -(C₃-C₆)cycloalkyl, heterocyclo, or heteroaryl, wherein if said heterocyclo or heteroaryl contains an -NH- moiety that nitrogen may be optionally substituted by (C₁-C₆)alkyl; and
wherein if two R₃ groups are on adjacent carbons, they may optionally form a 5- or 6-membered saturated, partially unsaturated or aromatic ring optionally containing 0, 1, or 2 heteroatoms selected from S, O, or NRₐ wherein Rₐ is H or (C₁-C₆)alkyl, S, or O;
**R₄** is hydrogen or halo;
**m** is 0-3;
**n** is 0-3;
**p** is independently 1 or 2 at each occurrence; and **R'** and **R"** independently at each occurrence are H, (C₁-C₆)alkyl, or aryl, or taken together with the nitrogen to which they are attached form an optionally substituted 3-6 membered ring saturated or partially unsaturated containing 0 or 1 additional heteroatom selected from NRₐ; and

each Rₐ, R₁, R₂, and R₃ may be optionally substituted on carbon by azido, halo, nitro, cyano, hydroxy, trifluoromethoxy, -OC(O)-(C₁-C₆)alkyl, NR'R", -CO₂H; C(O)-(C₁-C₆)alkyl, -CO₂(C₁-C₆)alkyl, -C(O)-NR'R", S(C₁-C₆), SOₚ(C₁-C₆)alkyl, SOₚNH(C₁-C₆)alkyl, SOₚNR'R", (C₂-C₆)alkenyl, (C₂-C₆)alkynyl, or (C₁-C₆)alkoxy.

According to a further aspect of the present invention there is provided a compound of formula IA or IB or a pharmaceutically acceptable salt thereof, wherein: 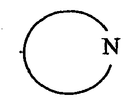 is a 3-8 membered, nitrogen linked, heterocycle or heteroaryl; wherein if said heterocycle or heteroaryl contains an -NH- moiety that nitrogen may be optionally substituted by a group selected from R⁵;
**R₁** at each occurrence is independently halo, hydroxy, nitro, formyl, cyano, -CO₂H, -SH, (C₁-C₆)alkyl, (C₂-C₆)alkenyl, (C₂-C₆)alkynyl, (C₃-C₆)cycloalkyl, (C₁-C₆)alkoxy, -OC(O)-(C₁-C₆)alkyl, -C(O)-(C₁-C₆)alkyl, -CO₂(C₁-C₆)alkyl, -NR'R", -NR'-C(O)-(C₁-C₆)alkyl, (C₁-C₆)alkylS(O)ₐ- wherein a is 0 to 2, -NR'-C(O)-O(C₁-C₆)alkyl, -NR'-SO₂-(C₁-C₆)alkyl, -NR'-C(O)NR'R", -SO₂-NR'R", -C(O)-NR'R", carbocyclyl, heterocyclo, heteroaryl or oxo;
**R₂** is hydrogen, halo, hydroxy, nitro, formyl, -CO₂H, -SH, cyano, (C₁-C₆)alkyl, (C₂-C₆)alkenyl, (C₂-C₆)alkynyl, (C₃-C₆)cycloalkyl, -O-(C₃-C₆)cycloalkyl, -OC(O)-(C₁-C₆)alkyl, -C(O)-(C₁-C₆)alkyl, -CO₂(C₁-C₆)alkyl, -NR'R", -NR'-C(O)-(C₁-C₆)alkyl; (C₁-C₆)alkylS(O)ₐ- wherein a is 0 to 2, -NR'-C(O)-O(C₁-C₆)alkyl, -NR'-SO₂-(C₁-C₆)alkyl, -NR'-C(O)NR'R", -SO₂-NR'R", -C(O)-NR'R", -OC(O)-NR'R", carbocyclyl, heterocyclo, heteroaryl or (C₁-C₆)alkoxy;
**R₃** at each occurrence is independently halo, nitro, formyl, cyano, hydroxy, -NR'R", -CO₂H, -C(O)-(C₁-C₆)alkyl, -CO₂(C₁-C₆)alkyl, -C(O)-NR'R", -NR'-C(O)-(C₁-C₆)alkyl, -NR'-C(O)NR'R", -NR'-C(O)-O(C₁-C₆)alkyl, -O-C(O)-(C₁-C₆)alkyl, -SH, -SO₂-NR'R", (C₁-C₆)alkyl, (C₂-C₆)alkenyl, (C₂-C₆)alkynyl, (C₁-C₆)alkoxy, (C₁-C₆)alkylS(O)ₙ- wherein a is 0 to 2, -NR'-SO₂-(C₁-C₆)alkyl, carbocyclyl, heterocyclo, or heteroaryl, wherein if said heterocyclo or heteroaryl contains an -NH- moiety that nitrogen may be optionally substituted by (C₁-C₆)alkyl; or
two **R₃** groups on adjacent carbons may optionally form a 5- or 6-membered saturated, partially unsaturated, unsaturated and/or aromatic ring optionally containing 0, 1, or 2 heteroatoms selected from S, O, or NRₐ wherein Rₐ is absent, H or (C₁-C₆)alkyl;
**R₄** is hydrogen or halo;
**m** is 0-3; wherein the values of R₃ may be the same or different;
**n** is 0-3; wherein the values of R₁ may be the same or different;
**p** is independently 1 or 2 at each occurrence; and
**R₅** is selected from (C₁-C₆)akyl, (C₃-C₆)cycloalkyl, -C(O)-(C₁-C₆)alkyl, -S(O)ₚ(C₁-C₆)alkyl, -CO₂(C₁-C₆)alkyl, -C(O)-NR'R", benzyl, benzyloxycarbonyl, benzoyl and phenylsulphonyl;
**R'** and **R"** independently at each occurrence are H, optionally substituted (C₁-C₆)alkyl, or optionally substituted aryl, or taken together with the nitrogen to which they are attached form an optionally substituted 3-6 membered ring saturated or partially unsaturated containing 0 or 1 additional heteroatom selected from NRₐ; wherein said optional substituents may be selected from one or more R₆;
**R₆** may be independently (C₁-C₆)alkyl, halo(C₁-C₆)alkyl, halo, nitro, cyano, hydroxy, (C₁-C₆)akoxy, -NR^{x}R^{y}, -COOR^{x} or -CONR^{x}R^{y}; and
**R^{x}** and **R^{y}** are independently of each other hydrogen or (C₁-C₆)alkyl; and wherein
each **Rₐ, R₁, R₂, R₃** and **R₅** may be optionally substituted on carbon by one or more formyl, -SH, azido, halo, nitro, cyano, hydroxy, trifluoromethoxy, -OC(O)-(C₁-C₆)alkyl, -NR'R", -CO₂H, -C(O)-(C₁-C₆)alkyl, -CO₂(C₁-C₆)alkyl, -C(O)-NR'R", -S-(C₁-C₆)alkyl, -SOₚ-(C₁-C₆)alkyl, -SOₚNR'R", (C₁-C₆)alkyl, (C₃-C₆)cycloalkyl, -NR'-C(O)-(C₁-C₆)alkyl, -NR'-C(O)-O(C₁-C₆)alkyl, -NR'-SO₂₋(C₁-C₆)alkyl, -NR'-C(O)NR'R", (C₂-C₆)alkenyl, (C₂-C₆)alkynyl, or (C₁-C₆)alkoxy.

What is also provided is a compound of formula IA-1 or a pharmaceutically acceptable salt thereof:

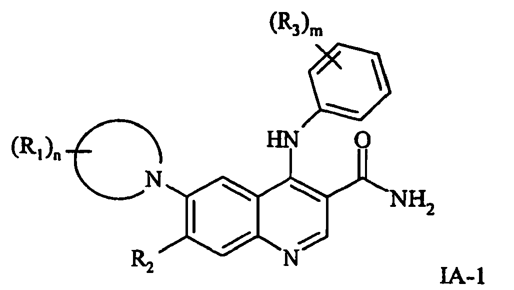

What is also provided is a compound of formula IA-2 or a pharmaceutically acceptable salt thereof: 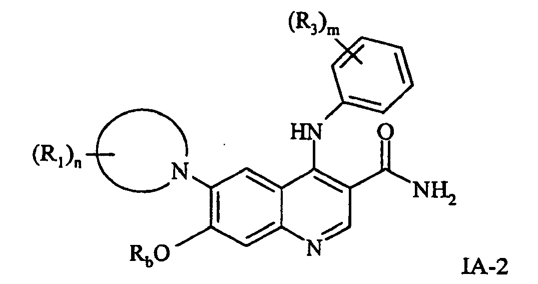 wherein R_{b} at each occurrence is independently H, (C₁-C₆)alkyl, (C₃-C₆)cycloalkyl, -C(O)-(C₁-C₆)alkyl, -C(O)-NR'R", wherein each R_{b} may be optionally substituted on carbon by halo, cyano, hydroxy, trifluoromethoxy, -OC(O)-(C₁-C₆)alkyl, -NR'R", -CO₂H, -C(O)-(C₁-C₆)akyl, -CO₂(C₁-C₆)alkyl, -C(O)-NR'R", -S(C₁-C₆)alkyl, -SOₚ(C₁-C₆)alkyl, -SOₚNR'R", (C₂-C₆)akenyl, (C₂-C₆)alkynyl, or (C₁-C₆)alkoxy, wherein R', R", and p are as defined above.

What is also provided is a compound of formula IA-3 or a pharmaceutically acceptable salt thereof: 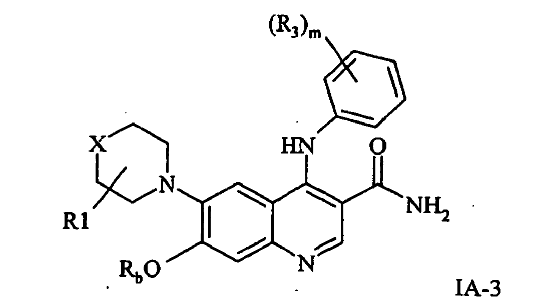 wherein
R₁ and R_{b} are as defined above; and
X is O, S, SO, SO₂, or N-R_{c}, wherein R_{c} is hydrogen, (C₁-C₆)alkyl, (C₃-C₆)cycloalkyl, -C(O)-(C₁-C₆)alkyl, -CO₂(C₁-C₆)alkyl, -C(O)-NR'R", or -SOₚNH(C₁-C₆)alkyl,
wherein R_{c} may be optionally substituted on carbon by azido, halo, nitro, cyano, hydroxy, trifluoromethoxy, -OC(O)-(C₁-C₆)alkyl, -NR'R", -CO₂H, -C(O)-(C₁-C₆)alkyl, -CO₂(C₁-C₆)alkyl, -C(O)-NR'R", -S(C₁-C₆)alkyl, -SOₚ(C₁-C₆)alkyl, -SOₚNR'R", (C₂-C₆)alkenyl, (C₂-C₆)alkynyl, or (C₁-C₆)akoxy, wherein R', R", and p are as defined above.

What is also provided is a compound of formula IA-3 or a pharmaceutically acceptable salt thereof wherein:
R₁ and R_{b} is as defined above; and
X is O, S, SO, SO₂, or N-R_{c}, wherein R_{c} is hydrogen, (C₁-C₆)alkyl, (C₃-C₆)cycloalkyl, (C₁-C₆)alkoxy, -OC(O)-(C₁-C₆)alkyl, -C(O)-(C₁-C₆)alkyl, -CO₂(C₁-C₆)alkyl, -C(O)-NR'R", or SOₚNH(C₁-C₆)alkyl,
wherein R_{c} may be optionally substituted on carbon by azido, halo, nitro, cyano, hydroxy, trifluoromethoxy, -OC(O)-(C₁-C₆)alkyl, NR'R", -CO₂H, C(O)-(C₁-C₆)alkyl, -CO₂(C₁-C₆)alkyl, -C(O)-NR'R", S(C₁-C₆), SOₚ(C₁-C₆)alkyl, SOₚNH(C₁-C₆)alkyl, SOpNR'R", (C₂-C₆)alkenyl, (C₂-C₆)alkynyl, or (C₁-C₆)alkoxy, wherein R', R", and p are as defined above.

Particular values of variable groups are as follows. Such values may be used where appropriate with any of the definitions, claims or embodiments defined hereinbefore or hereinafter.

A compound of formula IA.

A compound of formula IB. 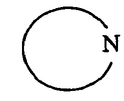 is a 5-7 membered, nitrogen linked, heterocycle; wherein if said heterocycle contains an -NH- moiety that nitrogen may be optionally substituted by a group selected from R₅; wherein
R₅ is selected from (C₁-C₆)alkyl, (C₃-C₆)cycloalkyl, -C(O)-(C₁-C₆)alkyl and CO₂(C₁-C6)alkyl; and
each R₅ may be optionally substituted on carbon by one or more cyano, hydroxy, -OC(O)-(C₁-C₆)alkyl, -NR'R", (C₃-C₆)cycloalkyl or (C₁-C₆)alkoxy; wherein
R' and R" independently at each occurrence are (C₁-C₆)alkyl. 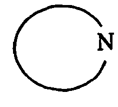 is piperazin-1-yl, piperidin-1-yl, morpholino, homopiperazin-1-yl and pyrrolidin-1-yl; wherein said piperazin-1-yl or homopiperazin-1-yl may be optionally substituted on nitrogen by a group selected from R₅; wherein
R₅ is selected from methyl, ethyl, isopropyl, cyclopropyl, acetyl, propionyl and t-butoxycarbonyl; and
each R₅ may be optionally substituted on carbon by one or more cyano, hydroxy, acetoxy, -NR'R", cyclopropyl or methoxy; wherein
R' and R" are methyl. 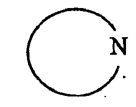 is piperazin-1-yl, *N*-methylpiperazin-1-yl, *N*-ethylpiperazin-1-yl, *N*-isopropylpiperazin-1-yl, *N*-acetylpiperazin-1-yl, *N*-(2-hydroxyacetyl)piperazin-1-yl, *N*-(2-dimethylaminoethyl)piperazin-1-yl, *N*-(2-methoxyethyl)piperazin-1-yl, *N*-(2-cyanoethyl)piperazin-1-yl, *N*-(2-hydroxyethyl)piperazin-1-yl, *N*-(cyclopropylmethyl)piperazin-1-yl, *N*-(cyclopropyl)piperazin-1-yl, *N*-((R)-2-hydroxypropionyl)piperazin-1-yl, *N*-((S)-2-hydroxypropionyl)piperazin-1-yl, *N*-(*t*-butoxycarbonyl)piperazin-1-yl, *N*-(acetoxyacetyl)piperazin-1-yl, piperidin-1-yl, morpholino, homopiperazin-1-yl, *N*-methylhomopiperazin-1-yl, *N*-ethylhomopiperazin-1-yl, *N*-acetylhomopiperazinl-1-yl, *N*-isopropylhomopiperazin-1-yl, *N*-cyclopropylhomopiperazin-1-yl and pyrrolidin-1-yl. 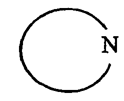 is *N*-methylpiperazin-1-yl.

R₁ is hydroxy.

R₁ at each occurrence is hydroxy, -NR'R" or oxo; wherein R' and R" independently at each occurrence are (C₁-C₆)alkyl.

R₁ at each occurrence is hydroxy, -NMe₂ or oxo.

n is 0 or 1.

n is 0.

n is 1.

n is 0 or 1 and R₁ is hydroxy.

n is 0 or 1 and R₁ is hydroxy, -NMe₂ or oxo.

R₂ is hydrogen, halo or (C₁-C₆)alkoxy; wherein R₂ may be optionally substituted on carbon by one or more (C₁-C₆)alkoxy.

R₂ is hydrogen, halo or (C₁-C₆)alkoxy; wherein R₂ may be optionally substituted on carbon by one or more (C₁-C₆)alkoxy or hydroxy.

R₂ is hydrogen, halo or (C₁-C₆)alkoxy.

R₂ is hydrogen, fluoro, methoxy, ethoxy or isopropoxy; wherein R₂ may be optionally substituted on carbon by one or more methoxy.

R₂ is hydrogen, fluoro, methoxy, ethoxy or isopropoxy; wherein R₂ may be optionally substituted on carbon by one or more methoxy or hydroxy.

R₂ is hydrogen, fluoro, methoxy, ethoxy or isopropoxy.

R₂ is hydrogen, fluoro, methoxy, ethoxy, 2-(methoxy)ethoxy, 2-hydroxyethoxy or isopropoxy.

R₂ is hydrogen.

R₂ is fluoro.

R₂ is methoxy.

R₂ is ethoxy.

R₂ is isopropoxy.

R₂ is 2-(methoxy)ethoxy.

R₂ is 2-hydroxyethoxy.

R₃ at each occurrence is independently halo, (C₁-C₆)alkyl or (C₁-C₆)alkoxy; wherein R₃ may be optionally substituted on carbon by halo.

R₃ at each occurrence is independently fluoro, chloro, methyl, ethyl or methoxy; wherein R₃ may be optionally substituted on carbon by fluoro.

R₃ at each occurrence is independently fluoro, chloro, methyl, trifluoromethyl, ethyl, methoxy or trifluoromethoxy.

m is 1-3; wherein the values of R₃ may be the same or different.

m is 1.

m is 2; wherein the values of R₃ may be the same or different.

m is 3; wherein the values of R₃ may be the same or different.

(R₃)ₘ and the phenyl to which it is attached form 2,4-difluorophenyl, 2-fluoro-3-chlorophenyl or 2-fluoro-4-methylphenyl.

R₄ is hydrogen or fluoro.

R₄ is hydrogen.

R₄ is fluoro.

Therefore in a further aspect of the invention there is provided a compound of formula IA or IB (as depicted above) wherein: 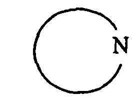 is a 5-7 membered, nitrogen linked, heterocycle; wherein if said heterocycle contains an -NH- moiety that nitrogen may be optionally substituted by a group selected from R₅;
R₁ is hydroxy;
n is 0 or 1;
R₂ is hydrogen, halo or (C₁-C₆)alkoxy;
R₃ at each occurrence is independently halo, (C₁-C₆)alkyl or (C₁-C₆)alkoxy; wherein R₃ may be optionally substituted on carbon by halo;
m is 1-3; wherein the values of R₃ may be the same or different;
R₄ is hydrogen or fluoro;
R₅ is selected from (C₁-C₆)alkyl, (C₃-C₆)cycloalkyl, -C(O)-(C₁-C₆)alkyl and -CO₂(C₁-C₆)alkyl; wherein R₅ may be optionally substituted on carbon by one or more cyano, hydroxy, -OC(O)-(C₁-C₆)alkyl, -NR'R", (C₃-C₆)cycloalkyl or (C₁-C₆)alkoxy; and
R' and R" independently at each occurrence are (C₁-C₆)alkyl;
or a pharmaceutically acceptable salt thereof.

Therefore in a further aspect of the invention there is provided a compound of formula IA or IB (as depicted above) wherein: 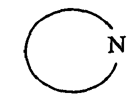 is a 5-7 membered, nitrogen linked, heterocycle; wherein if said heterocycle contains an -NH- moiety that nitrogen may be optionally substituted by a group selected from R₅;
R₁ at each occurrence is hydroxy, -NR'R" or oxo;
n is 0 or 1;
R₂ is hydrogen, halo or (C₁-C₆)alkoxy; wherein R₂ may be optionally substituted on carbon by one or more (C₁-C₆)alkoxy or hydroxy;
R₃ at each occurrence is independently halo, (C₁-C₆)alkyl or (C₁-C₆)alkoxy; wherein R₃ may be optionally substituted on carbon by halo;
m is 1-3; wherein the values of R₃ may be the same or different;
R₄ is hydrogen or fluoro;
R₅ is selected from (C₁-C₆)alkyl, (C₃-C₆)cycloalkyl, -C(O)-(C₁-C₆)alkyl and -CO₂(C₁-C₆)alkyl; wherein R₅ may be optionally substituted on carbon by one or more cyano, hydroxy, -OC(O)-(C₁-C₆)alkyl, -NR'R", (C₃-C₆)cycloalkyl or (C₁-C₆)alkoxy; and
R' and R" independently at each occurrence are (C₁-C₆)alkyl;
or a pharmaceutically acceptable salt thereof.

Therefore in a further aspect of the invention there is provided a compound of formula IA or IB (as depicted above) wherein: 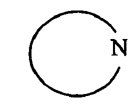 is piperazin-1-yl, *N*-methylpiperazin-1-yl, *N*-ethylpiperazin-1-yl, *N*-isopropylpiperazin-1-yl, *N*-acetylpiperazin-1-yl, *N*-(2-hydroxyacetyl)piperazin-1-yl, *N*-(2-dimethylaminoethyl)piperazin-1-yl, *N*-(2-methoxyethyl)piperazin-1-yl, *N*-(2-cyanoethyl)piperazin-1-yl, *N*-(2-hydroxyethyl)piperazin-1-yl, *N*-(cyclopropylmethyl)piperazin-1-yl, *N*-(cyclopropyl)piperazin-1-yl, *N*-((R)2-hydroxypropionyl)piperazin-1-yl, *N*-((S)-2-hydroxypropionyl)piperazin-1-yl, *N*-(*t*-butoxycarbonyl)piperazin-1-yl, *N*-(acetoxyacetyl)piperazin-1-yl, piperidin-1-yl, morpholino, homopiperazin-1-yl, *N*-methylhomopiperazin-1-yl, *N*-ethylhomopiperazin-1-yl, *N*-acetylhomopiperazin-1-yl, *N*-isopropylhomopiperazin-1-yl, *N*-cyclopropylhomopiperazin-1-yl and pyrrolidin-1-yl;
R₁ is hydroxy;
n is 0 or 1;
R₂ is hydrogen, fluoro, methoxy, ethoxy or isopropoxy;
R₃ at each occurrence is independently fluoro, chloro, methyl, trifluoromethyl, ethyl, methoxy or trifluoromethoxy;
m is 1-3; wherein the values of R₃ may be the same or different;
R₄ is hydrogen or fluoro;
or a pharmaceutically acceptable salt thereof.

Therefore in a further aspect of the invention there is provided a compound of formula IA or IB (as depicted above) wherein: 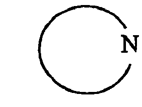 is piperazin-1-yl, *N*-methylpiperazin-1-yl, *N*-ethylpiperazin-1-yl, *N*-isopropylpiperazin-1-yl, *N*-acetylpiperazin-1-yl, *N*-(2-hydroxyacetyl)piperazin-1-yl, *N*-(2-dimethylaminoethyl)piperazin-1-yl, *N*-(2-methoxyethyl)piperazin-1-yl, *N*-(2-cyanoethyl)piperazin-1-yl, *N*-(2-hydroxyethyl)piperazin-1-yl, *N*-(cyclopropylmethyl)piperazin-1-yl, *N*-(cyclopropyl)piperazin-1-yl, *N*-((R)-2-hydroxypropionyl)piperazin-1-yl, *N*-((S)-2-hydroxypropionyl)piperazin-1-yl, *N*-(*t*-butoxycarbonyl)piperazin-1-yl, *N*-(acetoxyacetyl)piperazin-1-yl, piperidin-1-yl, morpholino, homopiperazin-1-yl, *N*-methylhomopiperazin-1-yl, *N*-ethylhomopiperazin-1-yl, *N*-acetylhomopiperazin-1-yl, *N*-isopropylhomopiperazin-1-yl, *N*-cyclopropylhomopiperazin-1-yl and pyrrolidin-1-yl;
R₁ at each occurrence is hydroxy, -NMe₂ or oxo;
n is 0 or 1;
R₂ is hydrogen, fluoro, methoxy, ethoxy, 2-(methoxy)ethoxy, 2-hydroxyethoxy or isopropoxy;
R₃ at each occurrence is independently fluoro, chloro, methyl, trifluoromethyl, ethyl, methoxy or trifluoromethoxy;
m is 1-3; wherein the values of R₃ may be the same or different;
R₄ is hydrogen or fluoro;
or a pharmaceutically acceptable salt thereof.

In another aspect of the invention, preferred compounds of the invention are any one of the Examples or a pharmaceutically acceptable salt thereof.

In another aspect of the invention, preferred compounds of the invention are any one of Examples 11, 12, 48, 70, 83, 88, 165, 166, 168 or 172 or a pharmaceutically acceptable salt thereof.

What is also provided is a compound which is one of the Examples.

What is also provided is a pharmaceutical composition which comprises a compound of formula IA or IB, or a pharmaceutically acceptable salt thereof, in association with a pharmaceutically-acceptable diluent or carrier.

What is also provided is a compound of formula IA or IB, or a pharmaceutically acceptable salt thereof, for use as a medicament.

What is also provided is the use of a compound of the formula IA or IB, or a pharmaceutically acceptable salt thereof, in the manufacture of a medicament for use in the production of a CSF-1R kinase inhibitory effect in a warm-blooded animal such as man.

What is also provided is the use of a compound of the formula IA or IB, or a pharmaceutically acceptable salt thereof, in the manufacture of a medicament for use in the production of an anti-cancer effect in a warm-blooded animal such as man.

What is also provided is the use of a compound of the formula IA or IB, or a pharmaceutically acceptable salt thereof, in the manufacture of a medicament for use in the treatment of melanoma, papillary thyroid tumours, cholangiocarcinomas, colon cancer, ovarian cancer, lung cancer, leukemias, lymphoid malignancies, carcinomas and sarcomas in the liver, kidney, bladder, prostate, breast and pancreas, and primary and recurrent solid tumours of the skin, colon, thyroid, lungs and ovaries.

What is also provided is the use of a compound of the formula IA or IB, or a pharmaceutically acceptable salt thereof, in the manufacture of a medicament for use in the treatment of breast, ovarian, bladder, cervical, endometrial, prostate, lung, kidney and pancreatic tumors; haematological malignancies including myelodysplastic syndrome, acute myelogenous leukemia, chronic myelogenous leukemia, non Hodgkin's lymphoma, Hodgkin's disease, multiple myeloma and chronic lymphocytic leukemia; and glioma, squamous cell carcinoma of the esophagus, malignant uveal melanoma and follicular lymphoma.What is also provided is the use of a compound of the formula IA or IB, or a pharmaceutically acceptable salt thereof, in the manufacture of a medicament for use in the treatment of tumor-associated osteolysis, osteoporosis including ovariectomy-induced bone loss, orthopedic implant failure, autoimmune disorders including systemic lupus erythematosus, arthritis including rheumatoid arthritis, osteoarthritis, renal inflammation and glomerulonephritis; inflammatory bowel disease; transplant rejection including renal and bone marrow allografts and skin xenograft, atherosclerosis, obesity, Alzheimer's Disease and Langerhans cell histiocytosis.

What is also provided is the use of a compound of the formula IA or IB, or a pharmaceutically acceptable salt thereof, in the manufacture of a medicament for use in the treatment of chronic obstructive pulmonary disease, diabetes and chronic skin disorders including psoriasis.

What is also provided is a pharmaceutical composition which comprises a compound of the formula IA or IB, or a pharmaceutically acceptable salt thereof, in association with a pharmaceutically-acceptable diluent or carrier for use in the production of a CSF-1R kinase inhibitory effect in a warm-blooded animal such as man.

What is also provided is a pharmaceutical composition which comprises a compound of the formula IA or IB, or a pharmaceutically acceptable salt thereof, in association with a pharmaceutically-acceptable diluent or carrier for use in the production of an anti-cancer effect in a warm-blooded animal such as man.

What is also provided is a pharmaceutical composition which comprises a compound of the formula IA or IB or a pharmaceutically acceptable salt thereof, in association with a pharmaceutically-acceptable diluent or carrier for use in the treatment of melanoma, papillary thyroid tumors, cholangiocarcinomas, colon cancer, ovarian cancer, lung cancer, leukemias, lymphoid malignancies, carcinomas and sarcomas in the liver, kidney, bladder, prostate, breast and pancreas, and primary and recurrent solid tumours of the skin, colon, thyroid, lungs and ovaries in a warm-blooded animal such as man.

What is also provided is a pharmaceutical composition which comprises a compound of the formula IA or IB or a pharmaceutically acceptable salt thereof, in association with a pharmaceutically-acceptable diluent or carrier for use in the treatment of breast, ovarian, bladder, cervical, endometrial, prostate, lung, kidney and pancreatic tumors; haematological malignancies including myelodysplastic syndrome, acute myelogenous leukemia, chronic myelogenous leukemia, non Hodgkin's lymphoma, Hodgkin's disease, multiple myeloma and chronic lymphocytic leukemia; and glioma, squamous cell carcinoma of the esophagus, malignant uveal melanoma and follicular lymphoma in a warm-blooded animal such as man.

What is also provided is a pharmaceutical composition which comprises a compound of the formula IA or IB or a pharmaceutically acceptable salt thereof, in association with a pharmaceutically-acceptable diluent or carrier for use in the treatment of tumor-associated osteolysis, osteoporosis including ovariectomy-induced bone loss, orthopedic implant failure, autoimmune disorders including systemic lupus erythematosus, arthritis including rheumatoid arthritis, osteoarthritis, renal inflammation and glomerulonephritis; inflammatory bowel disease; transplant rejection including renal and bone marrow allografts and skin xenograft, atherosclerosis, obesity, Alzheimer's Disease and Langerhans cell histiocytosis in a warm-blooded animal such as man.

What is also provided is a pharmaceutical composition which comprises a compound of the formula IA or IB or a pharmaceutically acceptable salt thereof, in association with a pharmaceutically-acceptable diluent or carrier for use in the treatment of chronic obstructive pulmonary disease, diabetes and chronic skin disorders including psoriasis in a warm-blooded animal such as man.

What is also provided is a process for preparing a compound of formula IA or IB or a pharmaceutically acceptable salt thereof, as provided in the schemes 1 and 2, *infra.*

### Definitions

"Alkyl" means a linear saturated monovalent hydrocarbon radical of, unless otherwise specified, one to six carbon atoms or a branched saturated monovalent hydrocarbon radical of, unless otherwise specified, three to six carbon atoms, e.g., methyl, ethyl, propyl, 2-propyl, pentyl, and the like. References to individual alkyl groups such as "propyl" are specific for the straight chain version only and references to individual branched chain alkyl groups such as 'isopropyl' are specific for the branched chain version only. For example, "(C₁-C₆)alkyl" includes methyl, propyl, isopropyl and t-butyl.

The term "halo" refers to fluoro, chloro, bromo and iodo.

"Alkenyl" means a linear monovalent hydrocarbon radical of, unless otherwise specified, two to six carbon atoms or a branched monovalent hydrocarbon radical of, unless otherwise specified, three to six carbon atoms, containing at least one double bond, e.g., ethenyl, propenyl, and the like. Examples of "(C₂-C₆)alkenyl" are vinyl, allyl and 1-propenyl.

"Alkynyl" means an alkyl group, as defined above, having one or more carbon-carbon triple bonds, e.g., ethynyl. Examples of "(C₂-C₆)alkynyl" are ethynyl, 1-propynyl and 2-propynyl.

"Cycloalkyl" means a saturated monovalent cyclic hydrocarbon radical of, unless otherwise specified, three to six ring carbons, e.g., cyclopropyl, cyclohexyl, and the like. Examples of "(C₃-C₆)cycloalkyl" include cyclopropyl and cyclohexyl.

"Aryl" means a monovalent monocyclic or bicyclic aromatic or totally unsaturated hydrocarbon radical of 6 to 10 ring atoms.

"Heterocycle" or "heterocyclo" means a saturated or partially unsaturated cyclic radical of 3 to 8 ring atoms in which one or two ring atoms are heteroatoms selected from NRₐ wherein Rₐ is as defined above, O, S, SO, or SO₂, which may be carbon or nitrogen linked unless otherwise specified.

"Heteroaryl" means an monovalent monocyclic radical, which is totally unsaturated and/or aromatic ring of 5 or 6 ring atoms containing one, two, or three ring heteroatoms selected from N, O, or S, the remaining ring atoms being C, which may be carbon or nitrogen linked unless otherwise specified. The term heteroaryl includes, but is not limited to pyridyl, pyrrolyl, thienyl, pyrazolyl, thiazolyl, imidazolyl, pyrimidinyl, thiadiazolyl and derivatives thereof.

A "carbocyclyl" is a saturated, partially saturated or unsaturated, mono or bicyclic carbon ring that contains 3-12 atoms; wherein a -CH₂- group can optionally be replaced by a -C(O)-. Particularly "carbocyclyl" is a monocyclic ring containing 5 or 6 atoms or a bicyclic ring containing 9 or 10 atoms. Suitable values for "carbocyclyl" include cyclopropyl, cyclobutyl, 1-oxocyclopentyl, cyclopentyl, cyclopentenyl, cyclohexyl, cyclohexenyl, phenyl, naphthyl, tetralinyl, indanyl or 1-oxoindanyl. A particular example of "carbocyclyl" is phenyl.

"Two R₃ groups on adjacent carbons may optionally form a 5- or 6-membered saturated, partially unsaturated, unsaturated and/or aromatic ring optionally containing 0, 1, or 2 heteroatoms selected from S, O, or NRₐ wherein Rₐ is absent, H or (C₁-C₆)alkyl", said ring forms a bicyclic ring with the phenyl to which it is attached. For the avoidance of doubt the 5-or 6-members of the ring include two from the phenyl ring to which it is attached. Suitable examples of two R₃ groups on adjacent carbons forming a 5- or 6-membered ring together with the phenyl to which they are attached include naphthyl, indol-6-yl, isoindole-5-yl, benzofuran-4-yl, quinolin-8-yl and 1H-indazol-7-yl.

R' and R" "taken together with the nitrogen to which they are attached form a 3-6 membered ring saturated or partially unsaturated containing 0 or 1 additional heteroatom selected from NRₐ". Examples and suitable values of this ring are azetidin-1-yl, piperidin-1-yl, piperazin-1-yL *N*-methylpiperazin-1-yl and pyrrolidin-1-yl. 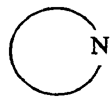 is a 3-10 membered, nitrogen linked, heterocycle or heteroaryl. Said ring may be mono- or bicyclic and/or bridged. Examples and suitable values of this ring include are azetidin-1-yl, morpholino, piperidin-1-yl, piperazin-1-yl, pyrrolidin-1-yl, thiomorpholino, homopiperazin-1-yl, pyrrol-1-yl, pyrazol-1-yl, imidazole-1-yl and triazol-1-yl. Additional examples, where 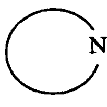 is a bicyclic ring include hexahydropyrrolo[1,2-*a*]pyrazin-2(1*H*)-yl and 3-methyl-5,6-dihydroimidazo[1,2-*a*]pyrazin-7(8*H*)-yl. Additional examples, where 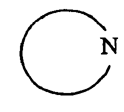 is a bridged ring include 2,5-diazabicyclo[2.2.1]hept-2-yl. Particularly 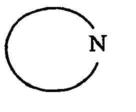 is a 3-8 membered, nitrogen linked, heterocycle or heteroaryl. Examples of "(C₁-C₆)alkoxy" include methoxy, ethoxy and isopropoxy. An example of "-OC(O)-(C₁-C₆)alkyl" is acetoxy. "-CO₂H" is carboxy. Examples of "-C(O)-(C₁-C₆)alkyl" include propionyl and acetyl. Examples of "-C(O)-NR'R"" wherein R' and R" are as defined above include carbamoyl, methylcarbamoyl, ethylcarbamoyl, dimethylcarbamoyl and *N*-phenyl-*N*-ethylcarbamoyl. Examples of "-CO₂(C₁-C₆)alkyl" include methoxycarbonyl, ethoxycarbonyl, n- and t-butoxycarbonyl. Examples of "-NR'R"" wherein R' and R" are as defined above include amino, methylamino, ethylamino, dimethylamino, diisopropylamino and N-ethyl-N-phenylamino. Examples of "-NR'-C(O)-(C₁-C₆)alkyl" wherein R' is as defined above include formamido, acetamide, propionylamino and *N*-acetyl-*N*-phenylamino. Examples of "-NR'-C(O)-O(C₁-C₆)alkyl" wherein R' is as defined above are methoxycarbonylamino and *N*-(*t*-butoxycarbonyl)-*N*-phenylamino. Examples of "-NR'-C(O)NR'R"" wherein R' and R" are as defined above include ureido, *N,N*'-dimethylureido, *N*-methyl-*N*'-propylureido, *N',N'*-diethylureido, *N*'-methyl-*N*'-propylureido, *N*-(methyl)-*N*'-ethyl-*N*'-isopropylureido and *N*-ethyl-*N',N'*-diethylureido. Examples of "-NR'-SO₂-(C₁-C₆)alkyl" wherein R' is as defined above include mesylamino, *N*-ethylsulphonyl-*N*-phenylamino and isopropylsulphonylamino. Examples of "-SO₂-NR'R'''' wherein R' and R" are as defined above include sulphamoyl, *N*-(methyl)sulphamoyl, *N*-(isopropyl)sulphamoyl, *N,N-*(dimethyl)sulphamoyl and *N*-(methyl)-*N*-(phenyl)sulphamoyl. Examples of "-SOₚ-(C₁-C₆)alkyl" wherein p is as defined above include mesyl, ethylsulphinyl and isopropylsulphonyl. Examples of "-SOₚNR'R'''' wherein p, R' and R" are as defined above include amino(oxido)sulfanyl, sulphamoyl, *N*-(methyl)sulphamoyl, *N*-(isopropyl)sulphamoyl, *N*-(isopropyl)amino(oxido)sulfanyl, *N,N-*(dimethyl)sulphamoyl and *N*-(methyl)-*N*-(phenyl)sulphamoyl. Examples of "-S(O)₂(C₁-C₆)alkyl" include mesyl, ethylsulphonyl and isopropylsulphonyl. Examples of "-S-(C₁-C₆)alkyl" include methylthio, ethylthio and isopropylthio. Examples of "(C₁-C₆)alkylS(O)ₐ- wherein a is 0 to 2" include methylthio, ethylthio, methylsulphinyl, ethylsulphinyl, mesyl and ethylsulphonyl. Examples f "halo(C₁-C₆)alkyl" include trifluoromethyl, 1-chloropropyl and 3-bromo-3-methylbutyl. Examples of "-O-(C₃-C₆)cycloalkyl" include cyclopropyloxy and cyclohexyloxy. Examples of -OC(O)-NR'R" include carbamoyloxy, methylcarbamoyloxy, ethylcarbamoyloxy, dimethylcarbamoyloxy and *N*-phenyl-*N*-ethylcarbamoyloxy.

A suitable pharmaceutically acceptable salt of a compound of the invention is, for example, an acid-addition salt of a compound of the invention which is sufficiently basic, for example, an acid-addition salt with, for example, an inorganic or organic acid, for example hydrochloric, hydrobromic, sulphuric, phosphoric, trifluoroacetic, citric or maleic acid. In addition a suitable pharmaceutically acceptable salt of a compound of the invention which is sufficiently acidic is an alkali metal salt, for example a sodium or potassium salt, an alkaline earth metal salt, for example a calcium or magnesium salt, an ammonium salt or a salt with an organic base which affords a physiologically-acceptable cation, for example a salt with methylamine, dimethylamine, trimethylamine, piperidine, morpholine or tris-(2-hydroxyethyl)amine.

Some compounds of the formula IA or IB may have chiral centres and/or geometric isomeric centres (E- and Z- isomers), and it is to be understood that the invention encompasses all such optical, diastereoisomers and geometric isomers that possess CSF-1R kinase inhibitory activity. The invention further relates to any and all tautomeric forms of the compounds of the formula IA or 1B that possess CSF-1R kinase inhibitory activity.

It is also to be understood that certain compounds of the formula IA or IB can exist in solvated as well as unsolvated forms such as, for example, hydrated forms. It is to be understood that the invention encompasses all such solvated forms that possess CSF-1R kinase inhibitory activity.

Another aspect of the present invention provides a process for preparing a compound of formula IA or IB or a pharmaceutically acceptable salt thereof which process (wherein variable groups are, unless otherwise specified, as defined in formula IA or IB) comprises of:
*Process a)* reacting a compound of formula IIA or IIB: 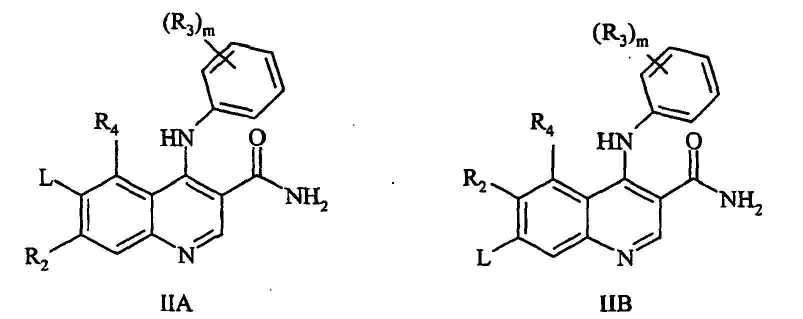 wherein L is a displaceable atom or group; with a compound of formula III: 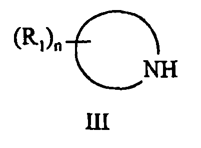 or
*Process b)* reacting a compound of formula IVA or IVB: 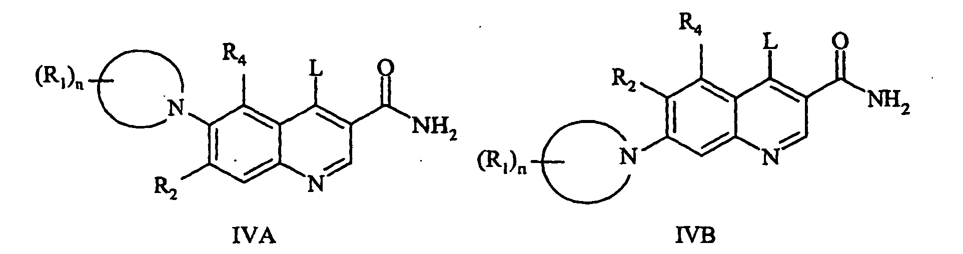 wherein L is a displaceable atom or group; with a compound of formula V: 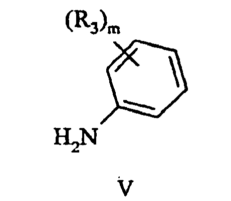 or
*Process c)* reacting a compound of formula VIA or VIB: 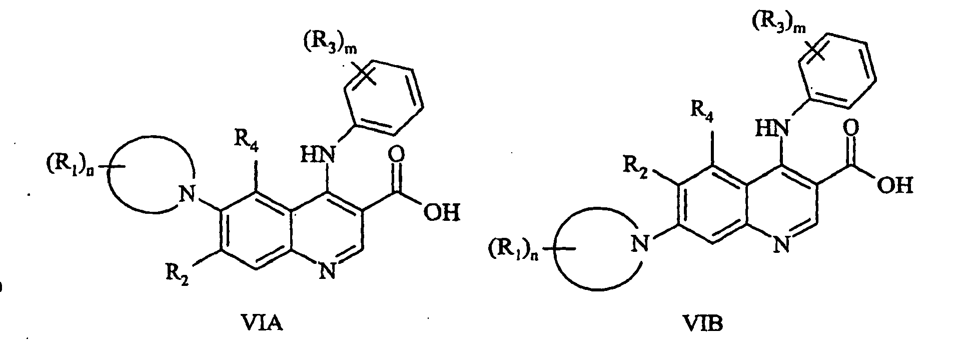 or an activated derivative thereof; with ammonia;
   or
*Process d)* reacting a compound of formula VIIA or VIIB: 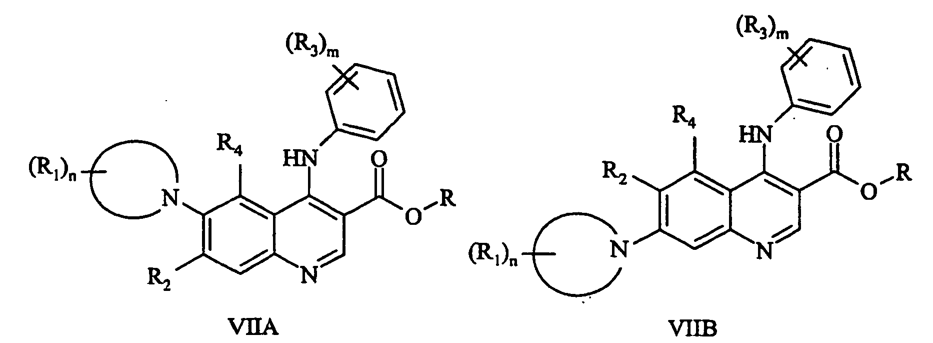 wherein R is (C₁-C₆)alkyl, in particular methyl and ethyl; with formamide and a base;
   or
*Process e)* hydrolysis of a compound of formula VIIIA or VIIIB: 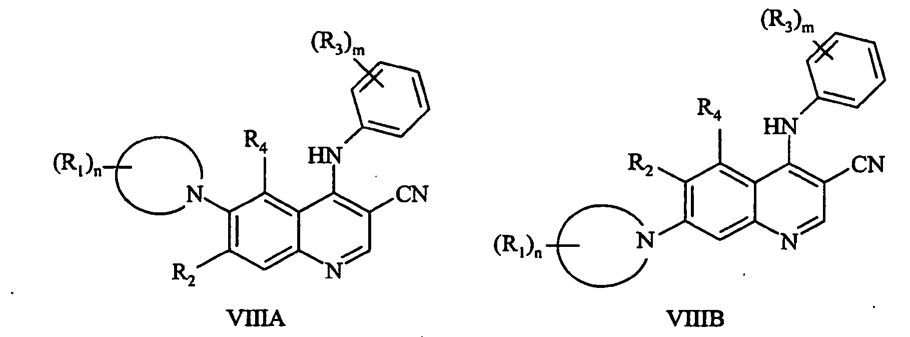 and thereafter if necessary:
   i) converting a compound of the formula IA or IB into another compound of the formula IA or IB;
   ii) removing any protecting groups;
   iii) forming a pharmaceutically acceptable salt.

L is a displaceable group, suitable values for L include chloro, bromo, tosyl and trifluoromethylsulphonyloxy.

Specific reaction conditions for the above reactions are as follows.
*Process a)* Compounds of formula IIA or IIB can be reacted with compounds of formula III by coupling chemistry utilizing an appropriate catalyst and ligand such as Pd₂(dba)₃ and BINAP respectively and a suitable base such as sodium *tert*-butoxide or caesium carbonate. The reaction usually requires thermal conditions often in the range of 80 °C to 100 °C.
   Compounds of formula IIA may be prepared according to the conditions of *Scheme I:* 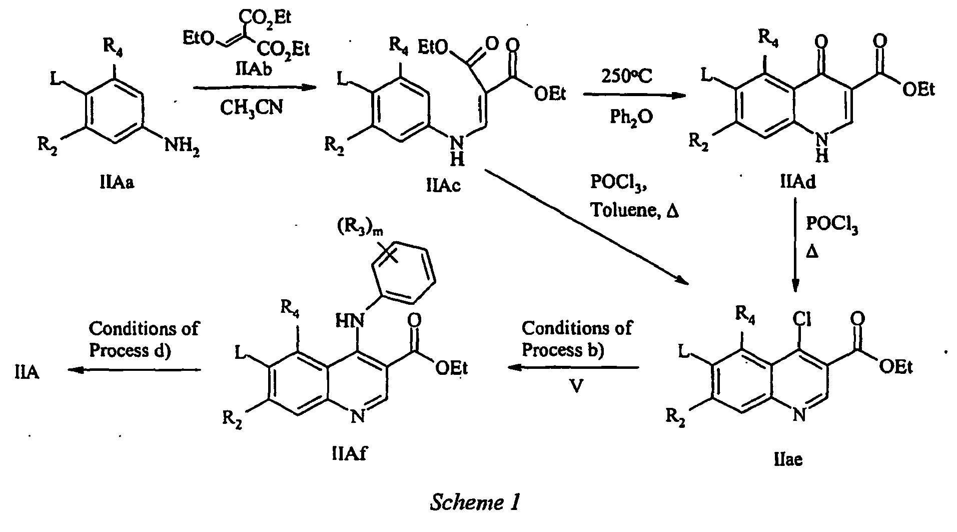
   Compounds of formula IIB may be prepared by a modification of *Scheme 1.*
   Compounds of formula IIAa, IIAb and III are commercially available compounds or they are literature compounds or they are readily prepared by processes known to the person skilled in the art.
*Process b)* Compounds of formula IVA or IVB can be reacted with compounds of formula V in a solvent such as ethanol or dimethylformamide, usually under thermal conditions often in the range of 70 °C to 100 °C, and in some cases catalysed by the addition of acetic acid.
   Alternatively, compounds of formula IVA or IVB can be reacted with compounds of formula V using coupling chemistry utilizing an appropriate catalyst and ligand such as Pd₂(dba)₃ and BINAP respectively and a suitable base such as sodium *tert*-butoxide or caesium carbonate. The reaction usually requires thermal conditions often in the range of 80 °C to 100 °C.
   Compounds of formula IVA and IVB may be prepared by a modification of *Scheme 1.*
   Compounds of formula V are commercially available compounds or they are literature compounds or they are readily prepared by processes known to the person skilled in the art.
*Process c)* Acids of formula VIA or VIB and ammonia may be coupled together in the presence of a suitable coupling reagent. Standard peptide coupling reagents known in the art can be employed as suitable coupling reagents, for example carbonyldiimidazole and dicyclohexyl-carbodiimide, optionally in the presence of a catalyst such as dimethylaminopyridine or 4-pyrrolidinopyridine, optionally in the presence of a base for example triethylamine, pyridine, or 2,6-di-*alkyl*-pyridines such as 2,6-lutidine or 2,6-di-*tert-*butylpyridine. Suitable solvents include dimethylacetamide, dichloromethane, benzene, tetrahydrofuran and dimethylformamide. The coupling reaction may conveniently be performed at a temperature in the range of -40 to 40 °C.
   Suitable activated acid derivatives include acid halides, for example acid chlorides, and active esters, for example pentafluorophenyl esters. The reaction of these types of compounds with amines is well known in the art, for example they may be reacted in the presence of a base, such as those described above, and in a suitable solvent, such as those described above. The reaction may conveniently be performed at a temperature in the range of -40 to 40 °C.
   Compounds of formula VIA or VIB may be prepared by a modification of *Schemes 1,* for example by adding a hydrolysis step and the procedure outlined in *Process a).*
*Process d)* Esters of formula VIIA or VIIB may be reacted together with formamide and a base. Preferably this reaction occurs sequentially, addition of the formamide first, followed by the base. Suitable bases are alkoxide bases, for example methoxide and ethoxide bases, eg sodium methoxide. The reaction is typically performed at a temperature of 100 °C in a suitable solvent such as DMF.Compounds of formula VIIA or VIIB may be prepared by a modification of *Scheme 1.*
*Process e)* Compounds of formula VIIIA or VIIIB can be hydrolysed under standard acidic or basic conditions.

Compounds of formula VIIIA or VIIIB may be prepared by a modification of *Scheme 1*.

It will be appreciated that certain of the various ring substituents in the compounds of the present invention may be introduced by standard aromatic substitution reactions or generated by conventional functional group modifications either prior to or immediately following the processes mentioned above, and as such are included in the process aspect of the invention. Such reactions and modifications include, for example, introduction of a substituent by means of an aromatic substitution reaction, reduction of substituents, alkylation of substituents and oxidation of substituents. The reagents and reaction conditions for such procedures are well known in the chemical art. Particular examples of aromatic substitution reactions include the introduction of a nitro group using concentrated nitric acid, the introduction of an acyl group using, for example, an acyl halide and Lewis acid (such as aluminium trichloride) under Friedel Crafts conditions; the introduction of an alkyl group using an alkyl halide and Lewis acid (such as aluminium trichloride) under Friedel Crafts conditions; and the introduction of a halo group. Particular examples of modifications include the reduction of a nitro group to an amino group by for example, catalytic hydrogenation with a nickel catalyst or treatment with iron in the presence of hydrochloric acid with heating; oxidation of alkylthio to alkylsulphinyl or alkylsulphonyl.

It will also be appreciated that in some of the reactions mentioned herein it may be necessary/desirable to protect any sensitive groups in the compounds. The instances where protection is necessary or desirable and suitable methods for protection are known to those skilled in the art. Conventional protecting groups may be used in accordance with standard practice (for illustration see T.W. Green, Protective Groups in Organic Synthesis, John Wiley and Sons, 1991). Thus, if reactants include groups such as amino, carboxy or hydroxy it may be desirable to protect the group in some of the reactions mentioned herein.

A suitable protecting group for an amino or alkylamino group is, for example, an acyl group, for example an alkanoyl group such as acetyl, an alkoxycarbonyl group, for example a methoxycarbonyl, ethoxycarbonyl or t-butoxycarbonyl group, an arylmethoxycarbonyl group, for example benzyloxycarbonyl, or an aroyl group, for example benzoyl. The deprotection conditions for the above protecting groups necessarily vary with the choice of protecting group. Thus, for example, an acyl group such as an alkanoyl or alkoxycarbonyl group or an aroyl group may be removed for example, by hydrolysis with a suitable base such as an alkali metal hydroxide, for example lithium or sodium hydroxide. Alternatively an acyl group such as a t-butoxycarbonyl group may be removed, for example, by treatment with a suitable acid as hydrochloric, sulphuric or phosphoric acid or trifluoroacetic acid and an arylmethoxycarbonyl group such as a benzyloxycarbonyl group may be removed, for example, by hydrogenation over a catalyst such as palladium-on-carbon, or by treatment with a Lewis acid for example boron tris(trifluoroacetate). A suitable alternative protecting group for a primary amino group is, for example, a phthaloyl group which may be removed by treatment with an alkylamine, for example dimethylaminopropylamine, or with hydrazine.

A suitable protecting group for a hydroxy group is, for example, an acyl group, for example an alkanoyl group such as acetyl, an aroyl group, for example benzoyl, or an arylmethyl group, for example benzyl. The deprotection conditions for the above protecting groups will necessarily vary with the choice of protecting group. Thus, for example, an acyl group such as an alkanoyl or an aroyl group may be removed, for example, by hydrolysis with a suitable base such as an alkali metal hydroxide, for example lithium or sodium hydroxide. Alternatively an arylmethyl group such as a benzyl group may be removed, for example, by hydrogenation over a catalyst such as palladium-on-carbon.

A suitable protecting group for a carboxy group is, for example, an esterifying group, for example a methyl or an ethyl group which may be removed, for example, by hydrolysis with a base such as sodium hydroxide, or for example a *t-*butyl group which may be removed, for example, by treatment with an acid, for example an organic acid such as trifluoroacetic acid, or for example a benzyl group which may be removed, for example, by hydrogenation over a catalyst such as palladium-on-carbon.

The protecting groups may be removed at any convenient stage in the synthesis using conventional techniques well known in the chemical art.

Certain intermediates described herein are novel and are thus provided as a further feature of the invention. For example compounds of formula VIIA and VIIB are provided as a further feature of the invention: 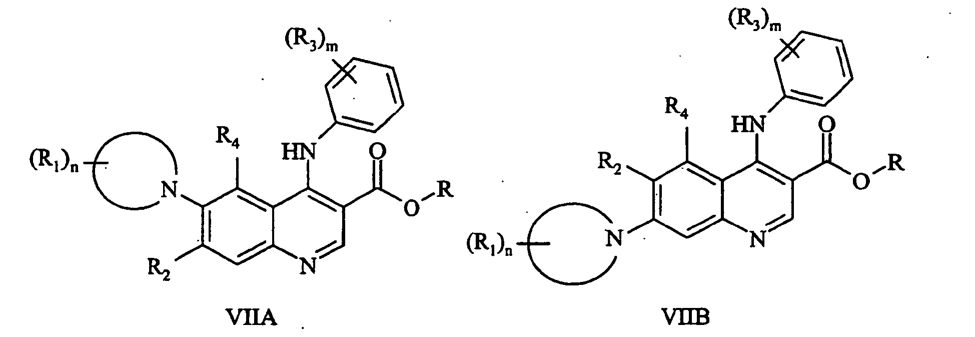 wherein variable groups are as defined herein above.

Likewise compounds of formula VIA and VIB are provided as a further feature of the invention: 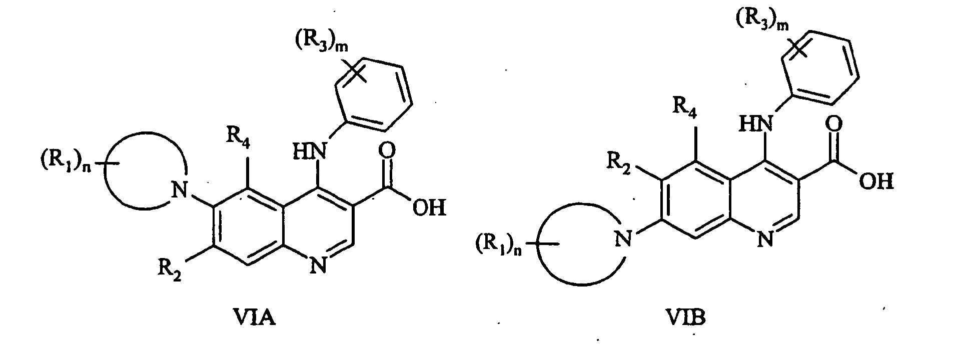 wherein variable groups are as defined herein above.

Likewise, novel compounds of formula IIA, IIB, IVA, IVB, VIIIA and VIIIB are considered further features of the invention.

As stated hereinbefore the compounds defined in the present invention possess anti-cancer activity which is believed to arise from the CSF-1R kinase inhibitory activity of the compounds. These properties may be assessed, for example, using the procedure set out below.

### Biological Activity

### CSF-1R in vitro AlphaScreen assay

Activity of purified CSF-1R was determined *in vitro* using an Amplified Luminescent Proximity Homogeneous Assay (ALPHA)(Perkin Elmer), which measures phosphorylation of the CSF-1R substrate, biotinylated poly-glutamine-tyrosine peptide (pEY-HTRF CisBio 61GTOBLD), as described below. The His-tagged kinase domain of CSF-1R (i.e., amino acids 568-912, GeneBank ID NM_005211; (see page 25 lines 13-19 of WO 2006/067445 for the sequence listing)) was purified from baculovirus infected SF+Express insect cells (1.4 x 106 cells/ml), French pressed and chromatographed through subsequent Qiagen Ni-NTA, Superflow Mono Q HR 10/10, and Superdex 200 SEC columns. Typical yield was 245µg/l of cell pellet at >95% purity.

The phosphorylation of the CSF-1R substrate in the presence and absence of the compound of interest was determined. Briefly, 0.57 nM of purified GSF-1R, 5nM pEY substrate, and compound were preincubated in 1x buffer for 30 minutes at 25 °C. Reactions were initiated with addition of 90µM adenosine triphosphate (ATP) in 1x buffer and incubated at 25 °C for 60 minutes and reactions stopped by addition of 5µl of detection mix consisting of 136mM NaCl, 102mM ethylenediamine tetraacetic acid, 1.65mg/ml BSA, 40ug/ml Streptavidin donor beads (Perkin Elmer 6760002), 40ug/ml pTyr100 acceptor beads (pekin Elmer 6760620). Plates were incubated at 25°C for 18 hours in the dark. Phosphorylated substrate was detected by an EnVision plate reader (Perkin Elmer) 680nm excitation, 520-620nm emission. Data was graphed and IC₅₀s calculated using Excel Fit (Microsoft).

When tested in the above *in vitro* assay, the compounds of the present invention exhibited activity less than 30 µM. For example the following results were obtained:

| **Example No** | **IC₅₀ (nM)** |
|---|---|
| 3 | 26 |
| 9 | 23 |
| 18 | 154 |

### Pharmaceutical Formulations

According to a further aspect of the invention there is provided a pharmaceutical composition which comprises a compound of the formula IA or IB, or a pharmaceutically acceptable salt thereof, as defined herein, in association with a pharmaceutically-acceptable diluent or carrier.

The composition may be in a form suitable for oral administration, for example as a tablet or capsule, for parenteral injection (including intravenous, subcutaneous, intramuscular, intravascular or infusion) as a sterile solution, suspension or emulsion, for topical administration as an ointment or cream or for rectal administration as a suppository.

In general the above compositions may be prepared in a conventional manner using conventional excipients.

The compound of formula IA or IB will normally be administered to a warm-blooded animal at a unit dose within the range 1-1000 mg/kg, and this normally provides a therapeutically-effective dose. Preferably a daily dose in the range of 10-100 mg/kg is employed. However the daily dose will necessarily be varied depending upon the host treated, the particular route of administration, and the severity of the illness being treated. Accordingly the optimum dosage may be determined by the practitioner who is treating any particular patient.

### Uses

According to a further aspect of the present invention there is provided a compound of the formula IA or IB, or a pharmaceutically acceptable salt thereof, as defined hereinbefore for use in a method of treatment of the human or animal body by therapy.

We have found that the compounds defined in the present invention, or a pharmaceutically acceptable salt thereof, are effective anti-cancer agents which property is believed to arise from their CSF-1R kinase inhibitory properties. Accordingly the compounds of the present invention are expected to be useful in the treatment of diseases or medical conditions mediated alone or in part by CSF-1R kinase, i.e. the compounds may be used to produce a CSF-1R kinase inhibitory effect in a warm-blooded animal in need of such treatment.

Thus the compounds of the present invention provide a method for treating cancer characterised by inhibition of CSF-IR kinase, i.e. the compounds may be used to produce an anti-cancer effect mediated alone or in part by the inhibition of CSF-1R kinase.

Such a compound of the invention is expected to possess a wide range of anti-cancer properties as aberrant expression of CSF1R and/or CSF1 has been observed in multiple human cancers and derived cell lines, including but not limited to, breast, ovarian, endometrial, prostate, lung, kidney and pancreatic tumors as well as haematological malignancies including, but not limited to, myelodysplastic syndrome, acute myelogenous leukemia, chronic myelogenous leukemia, non Hodgkin's lymphoma, Hodgkin's disease, multiple myeloma and chronic lymphocytic leukemia. Activating mutations have also been reported in haematopoietic and lymphoid tissue and lung cancer. Further, tumor associated macrophages have been associated with poor prognosis in multiple tumor types including, but not limited to, breast, endometrial, kidney, lung, bladder and cervical cancers, glioma, squamous cell carcinoma of the esophagus, malignant uveal melanoma and follicular lymphoma. It is expected that a compound of the invention will possess anticancer activity against these cancers through direct effect on the tumor and/or indirectly through effect on tumor associated macrophages. Alternatively particular cancers include melanoma, papillary thyroid tumours, cholangiocarcinomas, colon cancer, ovarian cancer, lung cancer, leukemias, lymphoid malignancies, carcinomas and sarcomas in the liver, kidney, bladder, prostate, breast and pancreas, and primary and recurrent solid tumours of the skin, colon, thyroid, lungs and ovaries.

In a further aspect of the invention, compounds of formula IA or IB may be also be of value in the treatment of certain additional indications. These indications include, but are not limited to tumor-associated osteolysis, osteoporosis including ovariectomy-induced bone loss, orthopedic implant failure, autoimmune disorders including systemic lupus erythematosus, arthritis including rheumatoid arthritis, osteoarthritis, renal inflammation and glomerulonephritis; inflammatory bowel disease; transplant rejection including renal and bone marrow allografts and skin xenograft, atherosclerosis, obesity, Alzheimer's Disease and Langerhans cell histiocytosis. A further aspect of the present invention therefore includes the treatment of one of more of these diseases, particularly arthritis including rheumatoid arthritis and osteoarthritis. These indications also include, but are not limited to chronic obstructive pulmonary disease, diabetes and chronic skin disorders including psoriasis.

Thus according to this aspect of the invention there is provided a compound of the formula IA or IB, or a pharmaceutically acceptable salt thereof, as defined hereinbefore for use as a medicament.

According to a further aspect of the invention there is provided the use of a compound of the formula IA or IB, or a pharmaceutically acceptable salt thereof, as defined hereinbefore in the manufacture of a medicament for use in the production of a CSF-1R kinase inhibitory effect in a warm-blooded animal such as man.

According to this aspect of the invention there is provided the use of a compound of the formula IA or IB, or a pharmaceutically acceptable salt thereof, as defined hereinbefore in the manufacture of a medicament for use in the production of an anti-cancer effect in a warm-blooded animal such as man.

According to a further feature of the invention, there is provided the use of a compound of the formula IA or IB, or a pharmaceutically acceptable salt thereof, as defined herein before in the manufacture of a medicament for use in the treatment of melanoma, papillary thyroid tumours, cholangiocarcinomas, colon cancer, ovarian cancer, lung cancer, leukemias, lymphoid malignancies, carcinomas and sarcomas in the liver, kidney, bladder, prostate, breast and pancreas, and primary and recurrent solid tumours of the skin, colon, thyroid, lungs and ovaries.

According to a further feature of the invention, there is provided the use of a compound of the formula IA or IB, or a pharmaceutically acceptable salt thereof, as defined herein before in the manufacture of a medicament for use in the treatment of breast, ovarian, bladder, cervical, endometrial, prostate, lung, kidney and pancreatic tumors; haematological malignancies including myelodysplastic syndrome, acute myelogenous leukemia, chronic myelogenous leukemia, non Hodgkin's lymphoma, Hodgkin's disease, multiple myeloma and chronic lymphocytic leukemia; and glioma, squamous cell carcinoma of the esophagus, malignant uveal melanoma and follicular lymphoma.

According to a further feature of the invention, there is provided the use of a compound of the formula IA or IB, or a pharmaceutically acceptable salt thereof, as defined herein before in the manufacture of a medicament for use in the treatment of tumor-associated osteolysis, osteoporosis including ovariectomy-induced bone loss, orthopedic implant failure, autoimmune disorders including systemic lupus erythematosus, arthritis including rheumatoid arthritis, osteoarthritis, renal inflammation and glomerulonephritis; inflammatory bowel disease; transplant rejection including renal and bone marrow allografts and skin xenograft, atherosclerosis, obesity, Alzheimer's Disease, chronic obstructive pulmonary disease, diabetes and chronic skin disorders including psoriasis and Langerhans cell histiocytosis.

According to a further feature of this aspect of the invention there is provided a method for producing a CSF-1R kinase inhibitory effect in a warm-blooded animal, such as man, in need of such treatment which comprises administering to said animal an effective amount of a compound of formula IA or IB, or a pharmaceutically acceptable salt thereof, as defined above.

According to a further feature of this aspect of the invention there is provided a method for producing an anti-cancer effect in a warm-blooded animal, such as man, in need of such treatment which comprises administering to said animal an effective amount of a compound of formula IA or IB, or a pharmaceutically acceptable salt thereof, as defined above.

According to an additional feature of this aspect of the invention there is provided a method of treating melanoma, papillary thyroid tumours, cholangiocarcinomas, colon cancer, ovarian cancer, lung cancer, leukemias, lymphoid malignancies, carcinomas and sarcomas in the liver, kidney, bladder, prostate, breast and pancreas, and primary and recurrent solid tumours of the skin, colon, thyroid, lungs and ovaries, in a warm-blooded animal, such as man, in need of such treatment which comprises administering to said animal an effective amount of a compound of formula IA or IB or a pharmaceutically acceptable salt thereof as defined herein before.

According to an additional feature of this aspect of the invention there is provided a method of treating breast, ovarian, bladder, cervical, endometrial, prostate, lung, kidney and pancreatic tumors; haematological malignancies including myelodysplastic syndrome, acute myelogenous leukemia, chronic myelogenous leukemia, non Hodgkin's lymphoma, Hodgkin's disease, multiple myeloma and chronic lymphocytic leukemia; and glioma, squamous cell carcinoma of the esophagus, malignant uveal melanoma and follicular lymphoma in a warm-blooded animal, such as man, in need of such treatment which comprises administering to said animal an effective amount of a compound of formula IA or IB or a pharmaceutically acceptable salt thereof as defined herein before.

According to an additional feature of this aspect of the invention there is provided a method of treating tumor-associated osteolysis, osteoporosis including ovariectomy-induced bone loss, orthopedic implant failure, autoimmune disorders including systemic lupus erythematosus, arthritis including rheumatoid arthritis, osteoarthritis, renal inflammation and glomerulonephritis; inflammatory bowel disease; transplant rejection including renal and bone marrow allografts and skin xenograft, atherosclerosis, obesity, Alzheimer's Disease, chronic obstructive pulmonary disease, diabetes and chronic skin disorders including psoriasis and Langerhans cell histiocytosis in a warm-blooded animal, such as man, in need of such treatment which comprises administering to said animal an effective amount of a compound of formula IA or IB or a pharmaceutically acceptable salt thereof as defined herein before.

In a further aspect of the invention there is provided a pharmaceutical composition which comprises a compound of the formula IA or IB, or a pharmaceutically acceptable salt thereof, as defined herein before in association with a pharmaceutically-acceptable diluent or carrier for use in the production of a CSF-1R kinase inhibitory effect in a warm-blooded animal such as man.

In a further aspect of the invention there is provided a pharmaceutical composition which comprises a compound of the formula IA or IB, or a pharmaceutically acceptable salt thereof, as defined herein before in association with a pharmaceutically-acceptable diluent or carrier for use in the production of an anti-cancer effect in a warm-blooded animal such as man.

In a further aspect of the invention there is provided a pharmaceutical composition which comprises a compound of the formula IA or IB, or a pharmaceutically acceptable salt thereof, as defined herein before in association with a pharmaceutically-acceptable diluent or carrier for use in the treatment of melanoma, papillary thyroid tumours, cholangiocarcinomas, colon cancer, ovarian cancer, lung cancer, leukemias, lymphoid malignancies, carcinomas and sarcomas in the liver, kidney, bladder, prostate, breast and pancreas, and primary and recurrent solid tumours of the skin, colon, thyroid, lungs and ovaries in a warm-blooded animal such as man. '

In a further aspect of the invention there is provided a pharmaceutical composition which comprises a compound of the formula IA or IB, or a pharmaceutically acceptable salt thereof, as defined herein before in association with a pharmaceutically-acceptable diluent or carrier for use in the treatment of breast, ovarian, bladder, cervical, endometrial, prostate, lung, kidney and pancreatic tumors; haematological malignancies including myelodysplastic syndrome, acute myelogenous leukemia, chronic myelogenous leukemia, non Hodgkin's lymphoma, Hodgkin's disease, multiple myeloma and chronic lymphocytic leukemia; and glioma, squamous cell carcinoma of the esophagus, malignant uveal melanoma and follicular lymphoma in a warm-blooded animal such as man.

In a further aspect of the invention there is provided a pharmaceutical composition which comprises a compound of the formula IA or IB, or a pharmaceutically acceptable salt thereof, as defined herein before in association with a pharmaceutically-acceptable diluent or carrier for use in the treatment of tumor-associated osteolysis, osteoporosis including ovariectomy-induced bone loss, orthopedic implant failure, autoimmune disorders including systemic lupus erythematosus, arthritis including rheumatoid arthritis, osteoarthritis, renal inflammation and glomerulonephritis; inflammatory bowel disease; transplant rejection including renal and bone marrow allografts and skin xenograft, atherosclerosis, obesity, Alzheimer's Disease, chronic obstructive pulmonary disease, diabetes and chronic skin disorders including psoriasis and Langerhans cell histiocytosis in a warm-blooded animal such as man.

According to a further aspect of the invention there is provided the use of a compound of the formula IA or IB, or a pharmaceutically acceptable salt thereof, as defined hereinbefore in the production of a CSF-1R kinase inhibitory effect in a warm-blooded animal such as man.

According to this aspect of the invention there is provided the use of a compound of the formula IA or IB, or a pharmaceutically acceptable salt thereof, as defined hereinbefore in the production of an anti-cancer effect in a warm-blooded animal such as man.

According to a further feature of the invention, there is provided the use of a compound of the formula IA or IB, or a pharmaceutically acceptable salt thereof, as defined herein before in the treatment of melanoma, papillary thyroid tumours, cholangiocarcinomas, colon cancer, ovarian cancer, lung cancer, leukemias, lymphoid malignancies, carcinomas and sarcomas in the liver, kidney, bladder, prostate, breast and pancreas, and primary and recurrent solid tumours of the skin, colon, thyroid, lungs and ovaries.

According to a further feature of the invention, there is provided the use of a compound of the formula IA or IB, or a pharmaceutically acceptable salt thereof, as defined herein before in the treatment of breast, ovarian, bladder, cervical, endometrial, prostate, lung, kidney and pancreatic tumors; haematological malignancies including myelodysplastic syndrome, acute myelogenous leukemia, chronic myelogenous leukemia, non Hodgkin's lymphoma, Hodgkin's disease, multiple myeloma and chronic lymphocytic leukemia; and glioma, squamous cell carcinoma of the esophagus, malignant uveal melanoma and follicular lymphoma.

According to a further feature of the invention, there is provided the use of a compound of the formula IA or IB, or a pharmaceutically acceptable salt thereof, as defined herein before in the treatment of tumor-associated osteolysis, osteoporosis including ovariectomy-induced bone loss, orthopedic implant failure, autoimmune disorders including systemic lupus erythematosus, arthritis including rheumatoid arthritis, osteoarthritis, renal inflammation and glomerulonephritis; inflammatory bowel disease; transplant rejection including renal and bone marrow allografts and skin xenograft, atherosclerosis, obesity, Alzheimer's Disease, chronic obstructive pulmonary disease, diabetes and chronic skin disorders including psoriasis and Langerhans cell histiocytosis.

The CSF-1R kinase inhibitory treatment defined hereinbefore may be applied as a sole therapy or may involve, in addition to the compound of the invention, conventional surgery or radiotherapy or chemotherapy. Such chemotherapy may include one or more of the following categories of anti-tumour agents :-
(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas); antimetabolites (for example antifolates such as fluoropyrimidines like 5-fluorouracil and tegafur, raltitrexed, methotrexate, cytosine arabinoside and hydroxyurea; antitumour antibiotics (for example anthracyclines like adriamycin, bleomycin, doxorubicin, daunomycin, epirubicin, idarubicin, mitomycin-C, dactinomycin and mithramycin); antimitotic agents (for example vinca alkaloids like vincristine, vinblastine, vindesine and vinorelbine and taxoids like taxol and taxotere); and topoisomerase inhibitors (for example epipodophyllotoxins like etoposide and teniposide, amsacrine, topotecan and camptothecin);
(ii) cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of Sa-reductase such as fmasteride;
(iii) Agents which inhibit cancer cell invasion (for example metalloproteinase inhibitors like marimastat and inhibitors of urokinase plasminogen activator receptor function);
(iv) inhibitors of growth factor function, for example such inhibitors include growth factor antibodies, growth factor receptor antibodies (for example the anti-erbb2 antibody trastuzumab [Herceptin™] and the anti-erbb1 antibody cetuximab [C225]), farnesyl transferase inhibitors, MEK inhibitors, tyrosine kinase inhibitors and serine/threonine kinase inhibitors, for example inhibitors of the epidermal growth factor family (for example EGFR family tyrosine kinase inhibitors such as *N-*(3-chloro-4-fluorophenyl)-7-methoxy-6-(3-morpholinopropoxy)quinazolin-4-amine (gefitinib, AZD1839), *N-*(3-ethynylphenyl)-6,7-bis(2-methoxyethoxy)quinazolin-4-amine (erlotinib, OSI-774) and 6-acrylamido-N (3-chtoro-4-fluorophenyl)-7-(3-morpholinopropoxy)quinazolin-4-amine (CI 1033)), for example inhibitors of the platelet-derived growth factor family and for example inhibitors of the hepatocyte growth factor family;
(v) antiangiogenic agents such as those which inhibit the effects of vascular endothelial growth factor, (for example the anti-vascular endothelial cell growth factor antibody bevacizumab [Avastin™], compounds such as those disclosed in International Patent Applications WO 97/22596, WO 97/30035, WO 97/32856 and WO 98/13354) and compounds that work by other mechanisms (for example linomide, inhibitors of integrin αvβ3 function and angiostatin);
(vi) vascular damaging agents such as Combretastatin A4 and compounds disclosed in International Patent Applications WO 99/02166, WO00/40529, WO 00/41669, WO01/92224, WO02/0443 and WO02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy;
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies;
(x) Cell cycle inhibitors including for example CDK inhibitors (eg flavopiridol) and other inhibitors of cell cycle checkpoints (eg checkpoint kinase); inhibitors of aurora kinase and other kinases involved in mitosis and cytokinesis regulation (eg mitotic kinesins); and histone deacetylase inhibitors; and
(xi) endothelin antagonists, including endothelin A antagonists, endothelin B antagonists and endothelin A and B antagonists; for example ZD4054 and ZD1611 (WO 96 40681), atrasentan and YM598.

Such conjoint treatment may be achieved by way of the simultaneous, sequential or separate dosing of the individual components of the treatment. Such combination products employ the compounds of this invention within the dosage range described hereinbefore and the other pharmaceutically-active agent within its approved dosage range.

In addition to their use in therapeutic medicine, the compounds of formula IA or IB and their pharmaceutically acceptable salts are also useful as pharmacological tools in the development and standardisation of in vitro and *in vivo* test systems for the evaluation of the effects of inhibitors of CSF-1R kinase in laboratory animals such as cats, dogs, rabbits, monkeys, rats and mice, as part of the search for new therapeutic agents.

In the above other pharmaceutical composition, process, method, use and medicament manufacture features, the alternative and preferred embodiments of the compounds of the invention described herein also apply.

### Processes for Making

Compounds of formula 1A can be prepared as provided in Schemes 1 and 2. The routes depicted in the schemes can be readily adapted for preparation of compounds of formula IB, by choice of starting material; that, is by using 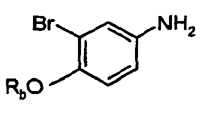 as a starting material in Scheme 1 or 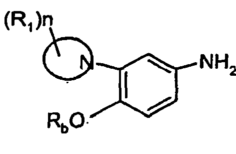 as a starting material in Scheme 2, compounds of formula IB may be obtained.

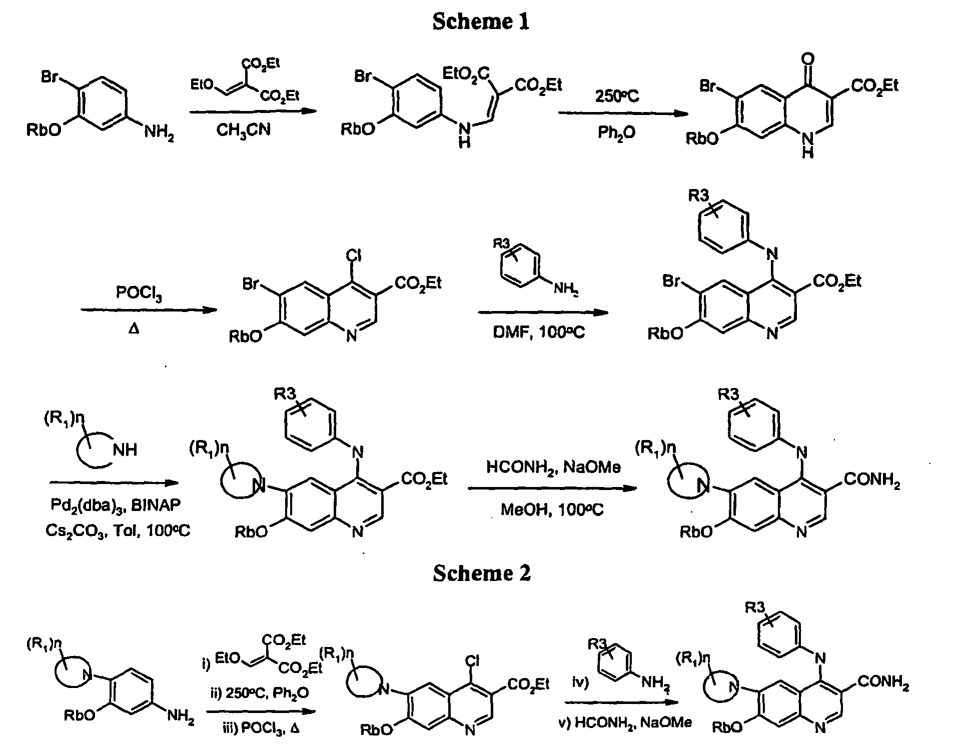

### Examples

The invention will now be illustrated by the following non limiting examples in which, unless stated otherwise:
(i) temperatures are given in degrees Celsius (°C); operations were carried out at room or ambient temperature unless otherwise stated, that is, at a temperature in the range of 18-25°C;
(ii) organic solutions were dried over anhydrous sodium sulphate or magnesium sulphate; evaporation of solvent was carried out using a rotary evaporator under reduced pressure (600-4000 Pascals; 4.5-30 mmHg) with a bath temperature of up to 60 °C;
(iii) in general, the course of reactions was followed by TLC and reaction times are given for illustration only;
(iv) final products had satisfactory-proton nuclear magnetic resonance (NMR) spectra and/or mass spectral data;
(v) yields are given for illustration only and are not necessarily those which can be obtained by diligent process development; preparations were repeated if more material was required;
(vii) when given, NMR data is in the form of delta values for major diagnostic protons, given in parts per million (ppm) relative to tetramethylsilane (TMS) as an internal standard, determined at 400 MHz using perdeuterio dimethyl sulphoxide (DMSO-d₆) as solvent unless otherwise indicated;
(vii) chemical symbols have their usual meanings; SI units and symbols are used;
(viii) solvent ratios are given in volume:volume (v/v) terms; and
(ix) mass spectra were run with an electron energy of 70 electron volts in the chemical ionization (CI) mode using a direct exposure probe; where indicated ionization was effected by electron impact (EI), fast atom bombardment (FAB) or electrospray (ESP); values for m/z are given; generally, only ions which indicate the parent mass are reported; and unless otherwise stated, the mass ion quoted is (MH)⁺;
(x) where a synthesis is described as being analogous to that described in a previous example the amounts used are the millimolar ratio equivalents to those used in the previous example;
(xi) the following abbreviations have been used:

| | |
|---|---|
| DMF | *N,N* dimethylformamide; |
| EtOAc | ethyl acetate; |
| MeOH | methanol; |
| DIEA | *N,N-*diisopropylethylamine; |
| HATU | *O-*(7-azabenzotriazol-1-yl)-*N,N*,*N',N'*-tetramethyluronium hexafluorophosphate; |
| MP-carbonate | macroporous triethylammonium methylpolystyrene carbonate; |
| THF | tetrahydrofuran; |
| DMSO | dimethylsulphoxide; |

(xii) "ISCO" refers to normal phase flash column chromatography using 12 g and 40 g prepacked silica gel cartridges used according to the manufacturers instruction obtained from ISCO, Inc, 4700 superior street Lincoln, NE, USA.; and
(xiii) "Gilson HPLC" refers to a YMC-AQC18 reverse phase HPLC Column with dimension 20 mm/100 and 50 mm/250 in water/MeCN with 0.1% TFA as mobile phase.

### Example 1

### 4-[(2,3-Dichlorophenyl)amino]-7-methoxy-6-(4-methylpiperazin-1-yl)quinoline-3-carboxamide

To a solution of ethyl 4-[(2,3-dichlorophenyl)amino]-7-methoxy-6-(4-methylpiperazin-1-yl)quinoline-3-carboxylate (Intermediate 1; 117 mg, 0.24 mmol) and formamide (47 µL, 1.2 mmol) in anhydrous DMF under N₂ at 100°C was added dropwise over 10 minutes a solution of NaOMe in MeOH (0.5 M, 1.44 mL, 0.72 mmol). After 2 hours at 100°C, the reaction mixture was cooled and poured into water. The aqueous layer was extracted with EtOAc, dried (Na₂SO₄), filtered, and concentrated to give 38 mg of a solid. ¹H NMR (CD₃OD): 8.92 (s, 1 H), 7.60 (dd, 1 H), 7.39 (m, 3 H), 7.01 (s, 1 H), 4.07 (s, 3 H), 3.50 (m, 2 H), 3.38 (m,2 H), 3.23 (m, 2 H), 2.92 (s, 3 H), 2.73 (m, 2H); *m*/*z*: 460.

### Examples 2-173

The following Examples were prepared by a similar method to Example 1 using the appropriate starting materials wherein purification of intermediates and final compounds was in some cases performed using an ISCO column chromatography or a Gilson HPLC system.

| **Ex.** | **Compound** | **NMR** | **M/z** | **SM** |
|---|---|---|---|---|
| **2** | 4-[(2,4-Dichlorophenyl) amino]-7-methoxy-6-(4-methylpiperazin-1-yl)quinoline-3-carboxamide | CD₃OD 8.82 (s, 1 H), 7.52 (d, 1 H), 7.28 (s, 1 H), 7.12 (dd, 1 H), 6.84 (s, 1 H), 6.71 (d, 1 H), 3.97 (s, 3 H), 2.97 (m, 4 H), 2.86 (m, 4 H), 2.52 (s, 3 H) | 460 | Intermediate 2 |
| **3** | 4-[(3,4-Dichlorophenyl) amino]-7-methoxy-6-(4-methylpiperazin-1-yl)quinoline-3-carboxamide | CD₃OD 8.79 (s, 1 H), 7.60 (d, 1 H), 7.53 (s, 1 H), 7.44 (d, 2 H), 7.27 (d, 1 H), 4.1 (s, 3 H), 3.61 (m, 4 H), 3.27 (m, 2 H), 2.98 (m, 5 H) | 460 | Intermediate 3 |
| **4** | 4-[(2,4-Difluorophenyl) amino]-7-methoxy-6-(4-methylpiperazin-1-yl)quinoline-3-carboxamide | CD₃OD 8.81 (s, 1 H), 7.52 (m, 1 H), 7.44 (s, 1H), 7.35 (s, 1H), 7.19 (m, 1 H), 7.12 (t, 1 H), 4.09 (s, 3 H), 3.55 (m, 4 H), 3.29 (m, 2 H), 2.96 (s, 3 H), 2.90 (m, 2 H) | 428 | Intermediate 4 |
| **5** | 4-[(2,3-Dichlorophenyl) amino]-7-methoxy-6-morpholin-4-ylquinoline-3-carboxamide | CD₃OD 8.89 (s, 1H), 7.63 (d, 1 H), 7.42 (m, 2 H), 7.27 (s, 1 H), 6.91 (s, 1 H), 4.06 (s, 3 H), 3.70 (m, 4 H), 2.73 (m, 4 H) | 447 | Intermediate 5 |
| **6** | 4-[(2,4-Difluorophenyl) amino]-7-methoxy-6-morpholin-4-ylquinoline-3-carboxamide | CD₃OD 8.80 (s, 1 H), 7.51 (d, 1 H), 7.31 (s, 1 H), 7.19 (m, 1 H), 7.18 (s, 1 H), 7.14 (m, 1 H), 4.06 (s, 3 H), 3.74 (m, 4 H), 2.85 (m, 4 H) | 415 | Intermediate 6 |
| **7** | 4-[(3,4-Dichlorophenyl) amino]-7-methoxy-6-morpholin-4-ylquinoline-3-carboxamide | CD₃OD 8.78 (s, 1 H), 7.62 (d, 1 H), 7.52 (d, 1 H), 7.29 (m, 2 H), 7.20 (s, 1 H), 4.07 (s, 3 H), 3.76 (m, 4 H), 2.90 (m, 4 H) | 447 | Intermediate 7 |
| **8** | 4-[(3,4-Dichlorophenyl) amino]-7-methoxy-6-piperidin-1-ylquinoline-3-carboxamide | CD₃OD 8.79 (s, 1 H), 7.62 (d, 1 H), 7.51 (d, 1 H), 7.30 (s, 1 H), 7.28 (dd, 1 H), 7.13 (s, 1 H), 4.05 (s, 3 H), 2.81 (m, 4 H), 1.63 (m; 4 H), 1.55 (m, 2 H) | 445 | Intermediate 8 |
| **9** | 4-[(2,3-Dichlorophenyl) amino]-6-methoxy-7-(4-methylpiperazin-1-yl)quinoline-3-carboxamide | CD₃OD 8.91 (s, 1 H), 7.78 (s, 1 H), 7.58 (dd, 1 H), 7.41 (m, 3 H), 6.95 (s, 1 H), 4.08 (m, 2 H), 3.67 (m, 2 H), 3.53 (s, 3 H), 3.33 (m, 4 H), 3.02 (s, 3 H) | 460 | Intermediate 9 |
| **10** | 4-[(3,4-Dichlorophenyl) amino]-6-methoxy-7-(4-methylpiperazin-1-yl)quinoline-3-carboxamide | CD₃OD 8.74 (s, 1 H), 7.57 (d, 1 H), 7.51 (d, 1 H), 7.37 (s,1H), 7.34 (s, 1 H), 7.25 (dd, 1 H), 4.03 (m, 2 H), 3.75 (s, 3 H), 3.64 (m, 2 H), 3.30 (m, 4 H), 2.99 (s, 3 H) | 460 | Intermediate 10 |
| **11** | 4-[(2,4-Difluorophenyl) amino]-7-ethoxy-6-(4-methylpiperazin-1-yl)quinoline-3-carboxamide | CD₃OD 8.80 (s,1H), 7.51 (m, 1 H), 7.34 (s, 2 H), 7.20 (m, 1 H), 7.13 (d, 1 H), 4.32 (q, 2 H), 3.54 (m, 4 H), 3.29 (m, 2 H), 2.94 (s, 3 H), 2.85 (m, 2 H), 1.56 (t, 3 H) | 441 | Intermediate 11 |
| **12** | 4-[(2,3-Dichlorophenyl) amino]-7-ethoxy-6-(4-methylpiperazin-1-yl)quinoline-3-carboxamide | CD₃OD 8.87 (s, 1 H), 7.53 (dd, 1 H), 7.35 (m, 3 H), 6.93 (s, 1 H), 4.27 (q, 2 H), 3.43 (m, 4 H), 3.20 (m, 2 H), 2.89 (s, 3 H), 2.71 (m, 2 H), 1.49 (t, 3 H) | 474 | Intermediate 12 |
| **13** | 4-[(2,4-Difluorophenyl) amino]-7-ethoxy-6-morpholin-4-ylquinoline-3-carboxamide | CD₃OD 8.78 (s, 1 H), 7.52 (d, 1 H), 7.24 (m, 3 H), 7.14 (m, 1 H), 4.30 (t, 2 H), 3.75 (m, 4 H), 2.86 (m, 4 H), 1.52 (t, 3 H) | 429 | Intermediate 13 |
| **14** | 4-[(3,4-Dichlorophenyl) amino]-7-ethoxy-6-morpholin-4-ylquinoline-3-carboxamide | CD₃OD 8.68 (s, 1 H), 7.53 (d, 1 H), 7.43 (d, 1 H), 7.19 (m, 2 H), 7.10 (s, 1 H), 4.21 (q, 2 H), 3.65 (m, 4 H), 2.83 (m, 4 H), 1.44 (t, 3 H) | 461 | Intermediate 14 |
| **15** | tert-Butyl 4-{3-(aminocarbonyl)-4-[(2,3-dichlorophenyl)amino]-7-methoxyquinolin-6-yl}piperazine-1-carboxylate | 10.79 (s, 1 H), 8.94 (s, 1 H), 8.35 (s, 1 H), 7.75 (s, 1 H), 7.34 (s, 1 H), 7.25 (dd, 1 H), 7.14 (1, 1 H), 6.70 (s, 1 H), 6.59 (d, 1 H), 3.95 (s, 3 H), 3.33 (m, 4 H), 2.68 (m, 4 H), 1.39 (s, 9H) | 545 | Intermediate 15 |
| **16** | 4-[(2,4-Difluorophenyl) amino]-7-fluoro-6-(4-methylpiperazin-1-yl)quinoline-3-carboxamide | 10.63 (s, 1 H), 8.86 (s, 1H), 8.29 (s, 1 H), 7.71 (s, 1 H), 7.63 (d, 1 H), 7.38 (m, 1 H), 7.11 (m, 3 H), 2.82 (m, 4 H), 2.48 (m, 4 H), 2.26 (s, 3 H) | 416 | Intermediate 16 |
| **17** | 4-[(2,3-Dichlorophenyl) amino]-7-fluoro-6-(4-methylpiperazin-1-yl)quinoline-3-carboxamide | 10.84 (s, 1 H), 8.98 (s, 1 H), 8.44 (s, 1H), 7.86 (s, 1 H), 7.70 (d, 1 H), 7.32 (d, 1 H), 7.19 (t, 1 H), 6.86 (d, 1 H), 6.69 (d, 1 H), 2.82 (m, 4 H), 2.48 (m, 4 H), 2.25 (s, 3 H) | 448 | Intermediate 17 |
| **18** | 4-[(2,4-Difluorophenyl) amino]-7-fluoro-6-morpholin-4-ylquinoline-3-carboxamide | CD₃OD 8.84 (s, 1 H), 7.66 (d, 1 H), 7.52 (m, 1H), 7.45 (m, 1H), 7.22 (m, 1 H), 7.14 (m, 1 H), 3.78 (m, 4 H), 2.95 (m, 4 H) | 403 | Intermediate 18 |
| **19** | 4-[(2,3-Dichlorophenyl) amino]-5-fluoro-6-(4-methylpiperazin-1-yl)quinoline-3-carboxamide | 10.90 (s, 1 H), 8.95 (s, 1 H), 8.46 (s, 1 H), 7.92 (s, 1 H), 7.79 (m, 1 H), 7.64 (t, 1 H), 7.19 (m, 1 H), 7.04 (t, 1 H), 6.47 (d, 1 H), 2.94 (m, 4 H), 2.38 (m, 4 H), 2.18 (s, 3 H) | 448 | Intermediate 19 |
| **20** | 7-Ethoxy-4-[(4-ethylphenyl)amino]-6-(4-methylpiperazin-1-yl)quinoline-3-carboxamide | 10.78 (s, 1 H), 8.82 (s, 1 H), 8.21 1 (s, 1 H), 7.57 (s, 1 H), 7.17 (s, 1 H), 7.12 (d, 2 H), 6.89 (d, 2 H), 6.80 (s, 1 H), 4.14 (q, 2 H), 2.63 (s, 4 H), 2.55 (q, 2 H), 2.30 (s, 4 H), 2.14 (s, 3 H), 1.38 (t, 3 H), 1.13 (t, 3 H) | 434 | Intermediate 20 |
| **21** | 4-[(3-Chloro-4-fluorophenyl)amino]-7-ethoxy-6-(4-methylpiperazin-1-yl)quinoline-3-carboxamide | 10.35 (s, 1 H), 8.80 (s, 1 H), 8.17 (s, 1 H), 7.59 (s, 1 H), 7.22 - 7.34 (m, 2 H), 7.09 (dd, 1 H), 6.85 - 6.96 (m, 2 H), 4.18 (q, 2 H), 2.81 (s, 4 H), 2.37 (s, 4 H), 2.17 (s, 3 H), 1.40 (t, 3 H) | 458 | Intermediate 21 |
| **22** | 4-[(3,4-Dichlorophenyl) amino]-7-ethoxy-6-(4-methylpiperazin-1-yl)quinoline-3-carboxamide | 8.78 (s, 1H), 8.12 (s,1H), 7.59 (s, 1 H), 7.44 (d, 1 H), 7.28 (s, 1 H), 7.09 (d, 1 H), 6.96 (s, 1 H), 6.85 (dd, 1 H), 4.19 (q, 2 H), 2.88 (s, 4 H), 2.43 (s, 4 H), 2.19 (s, 3 H), 1.41 (t, 3 H) | 474 | Intermediate 22 |
| **23** | 4-[(2,3-Dichlorophenyl) amino]-7-ethoxy-6-(4-ethylpiperazin-1-yl)quinoline-3-carboxamide | 10.80 (s, 1 H), 8.91 (s, 1 H), 8.37 (s, 1 H), 7.76 (s, 1 H), 7.29 (s, 1 H), 7.26 (d, 1 H), 7.14 (t, 1 H), 6.64 (s, 1 H), 6.57 (d, 1 H), 4.19 (q, 2 H), 2.75 (s, 4 H), 2.38 (s, 4 H), 2.35 (q, 2 H), 1.40 (t, 3 H), 0.97 (t, 3 H) | 488 | Intermediate 23 |
| **24** | 4-[(3-Chloro-2-fluorophenyl)amino]-7-ethoxy-6-morpholin-4-ylquinoline-3-carboxamide | CD₂Cl₂ 10.42 (s, 1 H), 8.77 (s, 1 H), 7.30 (s, 1 H), 7.06 (m, 1 H), 6.88 (m, 2 H), 6.73 (m, 1 H), 6.05 (br s, 2 H), 4.21 (q, 2 H), 3.72 (m, 4 H), 2.80 (m, 4 H), 1.50 (t, 3 H) | 445 | Intermediate 24 |
| **25** | 7-Ethoxy-4-[(2-fluoro-5-methylphenyl)amino]-6-morpholin-4-ylquinoline-3-carboxamide | CD₂Cl₂ 10.42 (s, 1 H), 8.74 (s, 1 H), 7.26 (s, 1 H), 7.01 (dd, 1 H), 6.95 (s, 1 H), 6.83 (m, 1 H), 6.77 (dd, 1 H), 6.02 (br s, 2 H), 4.20 (q, 2 H), 3.70 (m, 4 H), 2.74 (m, 4 H), 2.18(s,3H),1.49(t,3H) | 426 | Intermediate 25 |
| **26** | 4-[(3-Chloro-4-fluorophenyl)amino]-7-ethoxy-6-morpholin-4-ylquinoline-3-carboxamide | CD₂Cl₂ 10.51 (s,1H), 8.75 (s, 1 H), 7.27 (s, 1 H), 7.06 (m, 2 H), 6.92 (m, 1H), 6.85 (s, 1 H), 6.08 (br s, 2 H), 4.20 (q, 2 H), 3.673 (m, 4 H), 2.79 (m, 4 H), 1.49 (t, 3 H) | 447 | Intermediate 26 |
| **27** | 7-Ethoxy-4-[(4-ethylphenyl)amino]-6-morpholin-4-ylquinoline-3-carboxamide | CD₂Cl₂ 10.60 (s,1H), 8.69 (s, 1 H), 7.21 (s, 1 H), 7.11 (d, 2 H), 6.97 (d, 2 H), 6.90 (s, 1 H), 5.92 (br s, 2 H), 4.18 (q, 2 H), 3.66 (m, 4 H), 2.67 (m, 4 H), 2.59 (q, 2 H), 1.47 (t, 3H), 1.18 (t, 3H) | 421 | Intermediate 27 |
| **28** | 4-[(2,3-Dichlorophenyl) amino]-7-ethoxy-6-morpholin-4-ylquinoline-3-carboxamide | CD₂Cl₂ 10.36 (s, 1 H), 8.80 (s, 1 H), 7.32 (s, 1 H), 7.11 (d, 1 H), 6.97 (dd, 1 H), 6.79 (s, 1 H), 6.60 (d, 1 H), 6.04 (br s, 2 H), 4.23 (q, 2 H), 3.72 (m, 4 H), 2.82 (m, 4 H), 1.50 (t, 3 H) | 462 | Intermediate 28 |
| **29** | 4-[(2,3-Dichlorophenyl) amino]-7-ethoxy-6-(4-isopropylpiperazin-1-yl)quinoline-3-carboxamide | 11.10 (s, 1 H), 9.10 (s, 1 H), 8.55 (s, 1 H), 7.95 (s, 1 H), 7.70 (m, 2 H), 7.50 (m, 3 H), 7.36 (s, 1 H), 4.40 (q, 2 H), 3.60 (m, 4 H), 3.30 (m, 1 H), 3.20 (m, 4 H), 1.60 (t, 3 H), 1.50 (d, 6 H) | 501 | Intermediate 29 |
| **30** | 4-[(2,3-Dichlorophenyl) amino]-7-ethoxy-6-(4-methyl-1,4-diazepan-1-yl)quinoline-3-carboxamide | 10.69 (s, 1 H), 8.85 (s, 1 H), 8.35 (s, 1 H), 7.74 (s, 1 H), 7.24 (m, 2 H), 7.12 (m, 1 H), 6.54 (m, 2 H), 4.17 (q, 2 H), 3.14 (m, 2 H), 2.99 (m, 2 H), 2.50 (m, 2 H), 2.42 (m, 2 H), 2.19 (s, 3 H), 1.69 (m, 2 H), 1.41 (t, 3 H) | 487 | Intermediate 30 |
| **31** | 4-[(2,3-Dichlorophenyl) amino]-7-ethoxy-6-(3-hydroxypyrrolidin-1-yl)quinoline-3-carboxamide | 11.99 (s, 1 H), 8.83 (s, 1 H), 8.40 (s, 1 H), 7.90 (s, 1 H), 7.50 (d, 1 H); 7.33 (m, 1 H), 7.28 (s, 1 H), 7.23 (m, 1 H), 6.35 (s, 1 H), 4.12 (m, 3 H), 3.10 (m, 2 H), 2.90 (m, 2 H), 1.79 (m, 1 H), 1.69 (m, 1 H), 1.39 (t, 3 H) | 460 | Intermediate 31 |
| **32** | 4-[(2,3-Dichlorophenyl) amino]-6-{4-[2-(dimethylamino)ethyl] piperazin-1-yl}-7-ethoxyquinoline-3-carboxamide | 10.80 (s, 1 H), 9.00 (s, 1 H), 8.42 (s, 1 H), 7.75 (s, 1 H), 7.60 (m, 2 H), 7.35 (m, 2 H), 7.20 (s, 1 H), 4.25 (q, 2 H), 3.80-3.00 (m, 12 H), 2.85 (s, 6 H), 1.48 (t, 3 H) | 530 | Intermediate 32 |
| **33** | 4-[(2,3-Dichlorophenyl) amino]-7-ethoxy-6-[4-(2-methoxyethyl)piperazin-1-yl]quinoline-3-carboxamide | 11.10 (s, 1 H), 9.00 (s, 1 H), 8.45 (s, 1 H), 7.80 (s, 1 H), 7.60 (m, 2 H), 7.35 (m, 2 H), 7.22 (s, 1 H), 4.20 (q, 2 H), 3.71 (s, 3 H), 3.49-3.10 (m, 10 H), 2.95 (t, 2 H), 1.45 (t, 3 H) | 517 | Intermediate 33 |
| **34** | 4-[(3,4-Dichlorophenyl) amino]-7-ethoxy-6-(4-ethylpiperazin-1-yl)quinoline-3-carboxamide | CD₂Cl₂ 10.42 (s, 1 H), 8.75 (s, 1 H), 7.31 (d, 1 H), 7.27 (s, 1 H), 7.03 (s, 1 H), 6.86 (s, 1 H), 6.83 (d, 1 H), 6.05 (br s, 2 H), 4.21 (q, 2 H), 2.86 (m, 4 H), 2.48 (m, 4 H), 2.39 (q, 2 H), 1.51 (t, 3 H), 1.05 (t, 3 H) | 488 | Intermediate 34 |
| **35** | 4-[(2,4-Difluorophenyl) amino]-7-ethoxy-6-(4-ethylpiperazin-1-yl)quinoline-3-carboxamide | 10.72 (s, 1 H), 8.84 (s, 1 H), 8.27 (s, 1 H), 7.64 (s, 1 H), 7.36 (m, 1 H), 7.23 (s, 1 H), 7.00 (m, 2 H), 6.79 (s, 1 H), 4.16 (q, 2 H), 2.72 (m, 4 H), 2.38 (m, 4 H), 2.32 (q, 2 H), 1.40 (t, 3 H), 0.97 (t, 3 H) | 456 | Intermediate 35 |
| **36** | 4-[(3-Chloro-2-fluorophenyl)amino]-7-ethoxy-6-(4-ethylpiperazin-1-yl)quinoline-3-carboxamide | 10.69 (s, 1 H), 8.86 (s, 1 H), 8.30 (s, 1 H), 7.71 (s, 1 H), 7.18 - 7.29 (m, 2 H), 7.06 (s, 1 H), 6.76 - 6.84 (m, 2 H), 4.18 (q, 2 H), 2.77 (s, 4 H), 2.35 (m, 6 H), 1.40 (t, 3 H), 0.97 (t, 3 H) | 472 | Intermediate 36 |
| **37** | 7-Ethoxy-6-(4-ethylpiperazin-1-yl)-4-[(2-fluoro-5-methylphenyl)amino]qui noline-3-carboxamide | 10.78 (s, 1 H), 8.85 (s, 1 H), 8.29 (s, 1 H), 7.66 (s, 1 H), 7.17 (m, 2 H), 6.87 (m, 2 H), 6.69 (s, 1 H), 4.17 (q, 2 H), 2.71 (m, 4 H), 2.34 (m, 6 H), 2.13 (s, 3 H), 1.40 (t, 3 H), 0.97 (t, 3 H) | 452 | Intermediate 37 |
| **38** | 4-[(3-Chloro-4-fluorophenyl)amino]-7-ethoxy-6-(4-ethylpiperazin-1-yl)quinoline-3-carboxamide | CD₂Cl₂ 10.47 (s, 1 H), 8.74 (s, 1 H), 7.26 (s, 1 H), 7.06 (d, 1 H), 7.04 (dd, 1 H), 6.88 (m, 1 H), 6.85 (s, 1 H), 6.08 (br s, 2 H), 4.20 (q, 2 H), 2.82 (m, 4 H), 2.47 (m, 4 H), 2.38 (q, 2 H), 1.50 (t, 3 H), 1.05 (t, 3 H) | 472 | Intermediate 38 |
| **39** | 7-Ethoxy-4-[(4- ethylphenyl)amino]-6-(4-ethylpiperazin-1-yl)quinoline-3-carboxamide | CD₂Cl₂ 10.56 (s, 1 H), 8.69 (s, 1 H), 7.19 (s, 1 H), 7.10 (d, 2 H), 6.97 (d, 2 H), 6.91 (s, 1 H), 6.01 (br s, 2 H), 4.17 (q, 2 H), 2.70 (m, 4 H), 2.60 (q, 2 H), 2.39 (m, 4 H), 2.36 (q, 2 H), 1.47 (t, 3 H), 1.19 (t, 3 H), 1.04 (t, 3 H) | 448 | Intermediate 39 |
| **40** | 6-[4-(2-Cyanoethyl) piperazin-1-yl]-4-[(2,3-dichlorophenyl)amino]-7-ethoxyquinoline-3-carboxamide | 10.80 (s, 1 H), 8.90 (s, 1 H), 8.35 (s, 1 H), 7.72 (s, 1 H), 7.30 (s, 1 H), 7.25 (d, 1 H), 7.15 (t, 1 H), 6.65 (s, 1 H), 6.52 (d, 1 H), 4.20 (q, 2 H), 2.80-2.45 (m, 12 H), 1.40 (t, 3 H) | 512 | Intermediate 40 |
| **41** | 4-[(2,3-Dichlorophenyl) amino]-7-ethoxy-6-(4-hydroxypiperidin-1-yl)quinoline-3-carboxamide | 12.40 (s, 1 H), 9.11 (s, 1 H), 8.68 (s, 1 H), 8.00 (s, 1 H), 7.61 (d, 1 H), 7.50 (s, 1 H), 7.40 (m, 2 H), 6.86 (s, 1 H), 4.20 (q, 2 H), 3.55 (m, 1 H), 3.00 (m, 2 H), 2.45 (m, 2 H), 1.70 (m, 2 H), 1.32 (m, 5 H) | 474 | Intermediate 41 |
| **42** | 7-Ethoxy-4-[(4-ethylphenyl)amino]-6-(4-methyl-1,4-diazepan-1-yl)quinoline-3-carboxamide | 10.71 (s, 1 H), 8.77 (s, 1 H), 8.20 (s, 1 H), 7.56 (s, 1 H), 7.14 (s, 1 H), 7.11 (d, 2 H), 6.86 (d, 2 H), 6.72 (s, 1 H), 4.13 (q, 2 H), 3.03 (m, 2 H), 2.86 (m, 2 H), 2.555 (m, 2 H), 2.41 (m, 2 H), 2.18 (s, 3 H), 1.65 (m, 2 H), 1.39 (t, 3 H), 1.13 (t, 3 H) | 448 | Intermediate 42 |
| **43** | 4-[(2,4-Difluorophenyl) amino]-7-ethoxy-6-(3-hydroxypyrrolidin-1-yl)quinoline-3-carboxamide | 10.60 (s, 1 H), 8.80 (s, 1 H), 8.30 (s, 1 H), 7.69 (s, 1 H), 7.40 (m, 1 H), 7.25 (s, 1 H), 7.05 (m, 1 H), 6.97 (m, 1 H), 6.45 (s, 1 H), 4.87 (d, 1 H), 4.29 (m, 1 H), 4.20 (q, 2 H), 3.45 (m, 1 H), 3.22 (m, 1 H), 3.00 (m, 2 H), 1.90 (m, 1 H), 1.75 (m, 1 H), 1.46 (t, 3 H) | 428 | Intermediate 43 |
| **44** | 4-[(2,4-Difluorophenyl) amino]-7-ethoxy-6-(4-hydroxypiperidin-1-yl)quinoline-3-carboxamide | 10.78 (s, 1 H), 8.90 (s, 1 H), 8.31 (s, 1 H), 7.70 (s, 1 H), 7.43 (m, 1 H), 7.28 (s, 1 H), 7.05 (m, 2 H), 6.88 (s, 1 H), 4.67 (d, 1 H), 4.21 (q, 2 H), 3.55 (m, 1 H), 3.10 (m, 2 H), 2.46 (m, 2 H), 1.75 (m, 2 H), 1.45 (m, 5 H) | 442 | Intermediate 44 |
| **45** | 4-[(3,4-Dichlorophenyl) amino]-7-ethoxy-6-(4-isopropylpiperazin-1-yl)quinoline-3-carboxamide | CD₃OD 8.79 (s, 1 H), 7.37 (d, 1 H), 7.26 (s, 1 H), 7.07 (s, 1 H), 7.04 (d, 1 H), 6.88 (dd, 1 H), 4.24 (q, 2 H), 2.96 (m, 4 H), 2.65 (m, 5 H), 1.51 (t, 3 H), 1.10 (d, 6 H) | 502 | Intermediate 45 |
| **46** | 4-[(2,4-Difluorophenyl) amino]-7-ethoxy-6-(4-isopropylpiperazin-1-yl)quinoline-3-carboxamide | CD₃OD 8.76 (s, 1 H), 7.46 (m, 1 H), 7.36 (s, 1 H), 7.34 (s, 1 H), 7.20 (m, 1 H), 7.13 (m, 1 H), 4.30 (q, 2 H), 3.58 (m, 5 H), 3.26 (m, 2 H), 2.95 (m, 2 H), 1.55 (t, 3 H), 1.42 (d, 6 H) | 470 | Intermediate 46 |
| **47** | 4-[(3-Chloro-2-fluorophenyl)amino]-7-ethoxy-6-(4-isopropylpiperazin-1-yl)quinoline-3-carboxamide | CD₃OD 8.81 (s, 1 H), 7.26 (s, 1 H), 7.16 (m, 1 H), 7.04 (m, 1 H), 6.98 (s, 1 H), 6.83 (m, 1 H), 4.24 (q, 2 H), 2.91 (m, 4 H), 2.69 (m, 5 H), 1.51 (1, 3 H), 1.11 (d, 6 H) | 486 | Intermediate 47 |
| **48** | 7-Ethoxy-4-[(2-fluoro-5-methylphenyl)amino]-6-(4-isopropylpiperazin-1-yl)quinoline-3-carboxamide | CD₃OD 8.78 (s, 1H), 7.21 (s, 1 H), 7.03 (dd, 1 H), 7.00 (s, 1 H), 6.93 (m, 1 H), 6.78 (dd, 1 H), 4.23 (q, 2 H), 2.83 (m, 4 H), 2.68 (m, 1 H), 2.65 (m, 4 H), 2.19 (s, 3 H), 1.50 (t, 3H), 1.11 (d, 6H) | 466 | Intermediate 48 |
| **49** | 4-[(3-Chloro-4-fluorophenyl)amino]-7-ethoxy-6-(4-isopropylpiperazin-1-yl)quinoline-3-carboxamide | CD₃OD 8.77 (s, 1 H), 7.25 (s, 1 H), 7.16 (dd, 1 H), 7.07 (d, 1 H), 7.03 (s, 1 H), 6.95 (dd, 1 H), 4.24 (q, 2 H), 2.96 (m, 4 H), 2.77 (m, 1 H), 2.74 (m, 4 H), 1.51 (t, 3 H), 1.13 (d, 6 H) | 486 | Intermediate 49 |
| **50** | 4-[(2,4-Difluorophenyl) amino]-7-ethoxy-6-(4-methyl-1,4-diazepan-1-yl)quinoline-3-carboxamide | 10.70 (s, 1 H), 8.85 (s, 1 H), 8.30 (s, 1 H), 7.61 (s, 1 H), 7.35 (m, 1 H), 7.25 (s, 1 H), 6.89 (m, 2 H), 6.63 (s, 1 H), 4.20 (q, 2 H), 3.20 (m, 2 H), 3.00 (m, 2 H), 2.55 (m, 7 H), 1.80 (m, 2 H), 1.40 (t, 3 H) | 455 | Intermediate 50 |
| **51** | 4-[(2,3-Dichlorophenyl) amino]-6-(4-isopropylpiperazin-1-yl)-7-methoxyquinoline-3-carboxamide | 10.78 (s, 1 H), 8.92 (s, 1 H), 8.35 (s, 1 H), 7.75 (s, 1 H), 7.33 (s, 1 H), 7.26 (m, 1 H), 7.15 (m, 1 H), 6.65 (s, 1 H), 6.58 (d, 1 H), 3.95 (s, 3 H), 2.75 (m, 4 H), 2.60 (m, 1 H), 2.46 (m, 4 H), 0.95 (d, 6 H) | 488 | Intermediate 51 |
| **52** | 4-[(2,4-Difluorophenyl) amino]-6-(4-isopropylpiperazin-1-yl)-7-methoxyquinoline-3-carboxamide | 10.72 (s, 1 H), 9.86 (s, 1 H), 8.30 (s, 1 H), 7.67 (s, 1 H), 7.35 (m, 1 H), 7.26 (s, 1 H), 7.01 (m, 2 H), 6.80 (s, 1 H), 3.92 (s, 3 H), 2.70-2.45 (m, 9 H), 0.95 (d, 6 H) | 455 | Intermediate 52 |
| **53** | 4-[(2,3-Dichlorophenyl) amino]-7-methoxy-6-(4-methyl-1,4-diazepan-1-yl)quinoline-3-carboxamide | 8.90 (s, 1 H), 8.45 (s, 1 H), 7.75 (s, 1 H), 7.30 (m, 2 H), 7.17 (m, 1 H), 6.63 (s, 1 H), 6.58 (d, 1 H), 3.98 (s, 3 H), 3.20 (m, 2 H), 3.10 (m, 2 H), 2.50 (m, 4 H), 2.25 (s, 3 H), 1.75 (m, 2 H) | 474 | Intermediate 53 |
| **54** | 4-[(2,4-Difluorophenyl) amino]-7-methoxy-6-(4-methyl-1,4-diazepan-1-yl)quinoline-3-carboxamide | 10.65 (s, 1 H), 8.80 (s, 1 H), 8.30 (s, 1 H), 7.60 (s, 1 H), 7.32 (m, 1 H), 7.20 (m, 1 H), 6.95 (m, 2 H), 6.75 (m, 1 H), 3.90 (s, 3 H), 3.10 (m, 4 H), 3.00 (m, 4 H), 2.20 (s, 3 H), 1.69 (m, 2 H) | 441 | Intermediate 54 |
| **55** | 4-[(2,3-Dichlorophenyl) amino]-6-(4-ethylpiperazin-l-yl)-7-methoxyquinoline-3-carboxamide | 10.70 (s, 1 H), 8.85 (s, 1 H), 8.26 (s, 1 H), 7.67 (s, 1 H), 7.35 (m, 1 H), 7.28 (s, 1 H), 7.05 (m, 2 H), 6.80 (s, 1 H), 3.90 (s, 3 H), 2.75 (m, 4 H), 2.55 (m, 6 H), 0.95 (t, 3 H) | 474 | Intermediate 55 |
| **56** | 4-[(2,4-Difluorophenyl) amino]-6-(4-ethylpiperazin-1-yl)-7-methoxyquinoline-3-carboxamide | 10.80 (s, 1 H), 8.92 (s, 1 H), 8.40 (s, 1 H), 7.80 (s, 1 H), 7.35 (s, 1 H), 7.25 (m, 1 H), 7.15 (m, 1 H), 6.69 (s, 1 H), 6.55 (d, 1 H), 3.95 (s, 3 H), 2.75 (m 4 H), 2.38 (m, 4 H), 2.31 (q, 2 H), 1.00 (t, 3 H) | 441 | Intermediate 56 |
| **57** | 4-[(3,4-Dichlorophenyl) amino]-6-(4-ethylpiperazin-1-yl)-7-methoxyquinoline-3-carboxamide | CDCl₃ 10.45 (s, 1 H), 8.74 (s, 1 H), 7.30 (m, 2 H), 7.03 (d, 1 H), 6.91 (s, 1 H), 6.80 (d, 1 H), 4.00 (s, 3 H), 2.89 (s, 4 H), 2.54 (s, 4 H), 2.44 (q, 2 H), 1.09 (t, 3 H) | 474 | Intermediate 57 |
| **58** | 4-[(3,4-Dichlorophenyl) amino]-6-(4-isopropylpiperazin-1-yl)-7-methoxyquinoline-3-carboxamide | 10.11 (s, 1 H), 8.80 (s, 1 H), 8.13 (s, 1H), 7.60 (s, 1 H), 7.44 (d, 1 H), 7.31 (s, 1 H), 7.10 (d, 1 H), 6.97 (s, 1 H), 6.84 (d, 1 H), 3.94 (s, 3 H), 2.84 (s, 4 H), 2.63 (s, 1 H), 2.48 (s, 4 H), 0.96 (d, 6 H) | 488 | Intermediate 58 |
| **59** | 4-[(3,4-Dichlorophenyl) amino]-7-methoxy-6-(4-methyl-1,4-diazepan-1-yl)quinoline-3-carboxamide | CDCl₃ 10.40 (s, 1 H), 8.69 (s, 1 H), 7.28 (m, 2 H), 7.02 (d, 1 H), 6.80 (m, 2 H), 3.98 (s, 3 H), 3.25 (m, 2 H), 3.08 (m, 2 H), 2.64 (m, 4 H), 2.40 (br s, 3 H), 1.87 (m, 2 H) | 474 | Intermediate 59 |
| **60** | 4-[(3,4-Dichlorophenyl) amino]-7-ethoxy-6-(4-methyl-1,4-diazepan-1-yl)quinoline-3-carboxamide | CD₃OD 8.72 (s, 1 H), 7.38 (d, 1 H), 7.22 (s, 1 H), 7.04 (s, 1 H), 6.86 (m, 2 H), 4.23 (q, 2 H), 3.29 (m, 2 H), 3.11 (m, 2 H), 2.76 (m, 2 H), 2.65 (m, 2 H), 2.36 (s, 3 H), 1.92 (m, 2 H), 1.53 (t, 3 H) | 488 | Intermediate 60 |
| **61** | 7-Ethoxy-4-[(2-fluoro-5-methylphenyl)amino]-6-(4-methyl-1,4-diazepan-1-yl)quinoline-3-carboxamide | CD₃OD 8.73 (s, 1 H), 7.19 (s, 1 H), 7.04 (dd, 1 H), 6.89 (m, 2 H), 6.75 (d, 1 H), 4.21 (q, 2 H), 3.17 (m, 2 H), 2.96 (m, 2 H), 2.73 (m, 2 H), 2.64 (m, 2 H), 2.35 (s, 3 H), 2.19 (s, 3 H), 1.89 (m, 2 H), 1.52 (t,3H) | 452 | Intermediate 61 |
| **62** | 4-[(3-Chloro-4-fluorophenyl)amino]-7-ethoxy-6-(4-methyl-1,4-diazepan-1-yl)quinoline-3-carboxamide | CD₃OD 8.71 (s,1H), 7.21 (s, 1 H), 7.15 (m, 1 H), 7.04 (m, 1 H), 6.94 (m, 1 H), 6.87 (s, 1 H), 4.23 (q, 2 H), 3.24 (m, 2 H), 3.07 (m, 2 H), 2.78 (m, 2 H), 2.68 (m, 2 H), 2.37 (s, 3 H), 1.94 (m, 2 H), 1.52 (t, 3 H) | 472 | Intermediate 62 |
| **63** | 4-[(2-Fluorophenyl) amino]-7-methoxy-6-(4-methylpiperazin-1-yl)quinoline-3-carboxamide | 10.85 (s, 1 H), 8.71 (s, 1 H), 7.53 (s, 1 H), 7.19 (s, 1 H), 6.68 - 7.06 (m, 6 H), 3.84 (s, 3 H), 2.65 (s, 4 H), 2.24 (s, 4 H), 2.08 (s, 3 H) | 410 | Intermediate 63 |
| **64** | tert-Butyl 4-{3-(aminocarbonyl)-4-[(3-chloro-4-fluorophenyl) amino]-7-ethoxy quinolin-6-yl}piperazine-1-carboxylate | CD₂Cl₂ 10.42 (s,1H), 8.78 (s, 1 H), 7.31 (s, 1 H), 7.08 (m, 1 H), 6.90 (m, 1 H), 6.88 (s, 1 H), 6.72 (m, 1 H), 4.22 (q, 2 H), 3.44 (m, 4 H), 2.76 (m, 4 H), 1.50 (t, 3 H), 1.44 (s, 9 H) | 544 | Intermediate 64 |
| **65** | tert-Butyl 4-{3-(aminocarbonyl)-7-ethoxy-4-[(2-fluoro-5-methylphenyl)amino] quinolin-6-yl}piperazine-1-carboxylate | CD₂Cl₂ 10.50 (s,1 H), 8.76 (s, 1 H), 7.29 (s, 1 H), 7.02 (m, 1 H), 6.94 (s, 1 H), 6.88 (m, 1 H), 6.77 (d, 1 H), 4.21 (q, 2 H), 3.42 (m, 4 H), 2.70 (m, 4 H), 2.18 (s, 3 H), 1.49 (t, 3 H), 1.44 (s, 9 H) | 523 | Intermediate 65 |
| **66** | tert-Butyl 4-{3-(aminocarbonyl)-4-[(3-chloro-2-fluorophenyl) amino]-7-ethoxy quinolin-6-yl}piperazine-1-carboxylate | CD₂Cl₂ 10.51 (s, 1H), 8.75 (s,1 H), 7.29 (s, 1 H), 7.07 (m, 2 H), 6.85 (m, 2 H), 4.22 (q, 2 H), 3.46 (m, 4 H), 2.76 (m, 4 H), 1.50 (t, 3 H), 1.44 (s, 9 H) | 544 | Intermediate 66 |
| **67** | 7-Methoxy-4-[(3-methoxy-2-methylphenyl)amino]-6-(4-methylpiperazin-1-yl)quinoline-3-carboxamide | 10.96 (s, 1 H), 8.64 (s, 1 H), 7.44 (s, 1H), 7.10 (s, 1 H), 6.80-6.85 (m, 1H), 6.72 (s, 1H), 6.70 (s, 1 H), 6.57 (d, 1H), 6.28 (d, 1H), 3.80 (s, 3 H), 3.69 (s, 3 H), 2.54 (s, 4 H), 2.22 (s, 4 H), 2.12 (s, 3 H), 2.08 (s, 3 H) | 436 | Intermediate 67 |
| **68** | 4-[(4-Chloro-2-methylphenyl)amino]-7-methoxy-6-(4-methylpiperazin-1-yl)quinoline-3-carboxamide | 10.73 (s, 1 H), 8.87 (s, 1 H), 8.30 (s, 1 H), 7.65 (s, 1 H), 7.40-7.41 (m, 1H), 7.25 (s, 1H), 7.10-7.13 (m, 1 H), 6.67 (m, 2 H), 3.89 (s, 3 H), 3.34 (s, 3 H), 2.68 (s, 4H), 2.34 (s, 4 H), 2.17 (s, 3 H) | 440 | Intermediate 68 |
| **69** | 4-[(3-Chloro-2-methylphenyl)amino]-7-ethoxy-6-(4-methylpiperazin-1-yl)quinoline-3-carboxamide | 10.85 (s, 1H), 8.90 (s, 1 Fly, 8.30 (s, 1H), 7.60 (s, 1H), 7.20 (m, 2 H), 7.09 (t, 1 H), 6.65 (m, 2 H), 4.15 (q, 2 H), 2.65 (m, 4 H), 2.50 (s, 3 H), 2.30 (m, 4 H), 1.40 (t, 3 H) | 453 | Intermediate 69 |
| **70** | 4-[(3-Chloro-2-fluorophenyl)amino]-7-ethoxy-6-(4-methylpiperazin-1-yl)quinoline-3-carboxamide | 10.70 (s, 1 H), 8.85 (s, 1 H), 8.30 (s, 1 H), 7.55 (s, 1 H), 7.20 (m, 2 H), 7.05 (m, 1 H), 6.80 (m, 2 H), 4.20 (q, 2 H), 2.75 (m, 4 H), 2.49 (s, 3 H), 2.30 (m, 4 H), 1.40 (t, 3 H) | 457 | Intermediate 70 |
| **71** | 7-Ethoxy-4-[(3-ethylphenyl)amino]-6-(4-methylpiperazin-1-yl)quinoline-3-carboxamide | 10.70 (s, 1 H), 8.85 (s, 1 H), 8.20 (s, 1 H), 7.60 (s, 1 H), 7.20 (m, 2 H), 6.90-6.70 (m, 4 H), 4.20 (q, 2 H), 2.70 (m, 4 H), 2.50 (m, 5 H), 2.30 (m, 4 H), 1.40 (t, 3 H), 1.10 (t, 3 H) | 433 | Intermediate 71 |
| **72** | 4-[(3-Chlorophenyl) amino]-7-ethoxy-6-(4-methylpiperazin-1-yl)quinoline-3-carboxamide | THF-d8 10.38 (s, 1 H), 8.86 (s, 1 H), 7.60 (s, 1 H), 7.09-7.15 (m, 3 H), 6.74-6.81 (m, 4 H), 4.07 (q, 2 H), 2.70 (s, 4 H), 2.26 (s, 4 H), 2.09 (s, 3 H), 1.86 (t, 3 H) | 440 | Intermediate 72 |
| **73** | 4-[(2-Chloro-4-fluorophenyl)amino]-7-ethoxy-6-(4-methylpiperazin-1-yl)quinoline-3-carboxamide | 10.85 (s, 1 H), 8.95 (s, 1 H), 8.38 (s, 1 H), 7.75 (s, 1 H), 7.65 (d, 1 H), 7.30 (s, 1 H), 7.15 (m, 1 H), 6.85 (m, 1 H), 6.69 (s, 1 H), 4.22 (q, 2 H), 2.80 (m, 4 H), 2.40 (m, 4 H), 2.21 (s, 3 H), 1.45 (t, 3 H) | 457 | Intermediate 73 |
| **74** | 4-[(4-Chloro-2-fluorophenyl)amino]-7-ethoxy-6-(4-methylpiperazin-1-yl)quinoline-3-carboxamide | 10.64 (s, 1 H), 8.85 (s, 1 H), 8.30 (s, 1 H), 7.69 (s, 1 H), 7.50 (d, 1 H), 7.27 (s, 1 H), 7.15 (d, 1 H), 6.89 (m, 1 H), 6.80 (s, 1 H), 4.20 (q, 2 H), 2.80 (m, 4 H), 2.35 (m, 4 H), 2.18 (s, 3 H), 1.40 (t, 3 H) | 457 | Intermediate 74 |
| **75** | 7-Ethoxy-4-[(3-methylphenyl)amino]-6-(4-methylpiperazin-1-yl)quinoline-3-carboxamide | 10.70 (s, 1 H), 8.90 (s, 1 H), 8.27 (s, 1 H), 7.65 (s, 1 H), 7.28 (s, 1 H), 7.20 (m, 1 H), 6.93 (m, 2 H), 6.80 (m, 2 H), 4.22 (q, 2 H), 2.75 (m, 4 H), 2.37 (m, 4 H), 2.26 (s, 3 H), 2.20 (s, 3 H), 1.45 (t, 3 H) | 419 | Intermediate 75 |
| **76** | 4-[(2-Chloro-3-methylphenyl)amino]-7-ethoxy-6-(4-methylpiperazin-1-yl)quinoline-3-carboxamide | 10.80 (s, 1 H), 8.89 (s, 1 H), 8.32 (s, 1 H), 7.65 (s, 1 H), 7.22 (s, 1 H), 7.00 (m, 2 H), 6.70 (s, 1 H), 6.53 (m, 1 H), 4.18 (q, 2 H), 2.69 (m, 4 H), 2.38 (s, 3 H), 2.30 (m, 4 H), 2.15 (s, 3 H), 1.39 (t, 3 H) | 453 | Intermediate 76 |
| **77** | 7-Ethoxy-4-[(3-fluoro-2-methylphenyl)amino]-6-(4-methylpiperazin-1-yl)quinoline-3-carboxamide | 10.75 (s, 1 H), 8.86 (s, 1 H), 8.30 (s, 1 H), 7.65 (s, 1 H), 7.20 (s, 1 H), 7.05 (m, 1 H), 6.90 (m, 1 H), 6.66 (s, 1 H), 6.49 (d, 1 H), 4.20 (q, 2 H), 2.70 (m, 4 H), 2.35 (m, 4 H), 2.22 (s, 3 H), 2.15 (s, 3 H), 1.39 (t, 3 H) | 437 | Intermediate 77 |
| **78** | 4-[(3,4-Difluorophenyl)amino]-7-methoxy-6-(4-methylpiperazin-1-yl)quinoline-3-carboxamide | THF-d8 10.99 (s, 1 H), 8.85 (s, 1 H), 7.70 (s, 1 H), 7.34 (s, 1 H), 7.16 (m, 1 H), 6.99 (s, 1 H), 6.89 (m, 2 H), 6.73 (m, 1 H), 3.99 (s, 3 H), 2.84 (s, 4 H), 2.41 (s, 4 H), 2.23 (s, 3 H) | 428 | Intermediate 78 |
| **79** | 7-Methoxy-6-(4-methylpiperazin-1-yl)-4-{[2-methyl-3-(trifluoromethyl)phenyl]a mino}quinoline-3-carboxamide | THF-d8 10.96 (s, 1 H), 8.72 (s, 1 H), 7.67 (s, 1 H), 7.25 (d, 1 H), 7.18 (s, 1 H), 7.02 (t, 1 H), 6.87 (s, 1 H), 6.78 (d, 1 H), 6.64 (s, 1 H), 3.83 (s, 3 H), 2.63 (s, 4 H), 2.45 (s, 3 H), 2.21 (s, 4 H), 2.06 (s, 3 H) | 474 | Intermediate 79 |
| **80** | 4-[(4-Chlorophenyl) amino]-7-methoxy-6-(4-methylpiperazin-1-yl)quinoline-3-carboxamide | THF-d8 10.86 (s, 1 H), 8.70 (s, 1 H), 7.62 (s, 1 H), 7.18 (s, 1 H), 7.10 (d, 2 H), 6.78 (m, 4 H), 3.84 (s, 3 H), 2.68 (s, 4 H), 2.25 (s, 4 H), 2.09 (s, 3 H) | 426 | Intermediate 80 |
| **81** | 4-[(2-Fluoro-4-methylphenyl)amino]-7-methoxy-6-(4-methyl piperazin-1-yl)quinoline-3-carboxamide | THF-d8 10.85 (s, 1 H), 8.67 (s, 1 H), 7.48 (s, 1 H), 7.16 (s, 1 H), 6.64-6.89 (m, 5 H), 3.82 (s, 3 H), 2.63 (s, 4 H), 2.24 (s, 4 H), 2.18 (s, 3 H), 2.08 (s, 3 H) | 424 | Intermediate 81 |
| **82** | 7-Methoxy-6-(4-methylpiperazin-1-yl)-4-{[3-(trifluoromethyl) phenyl]ammo}quinoline-3-carboxamide | THF-d8 10.96 (s, 1 H), 8.79 (s, 1 H), 7.60 (s, 1 H), 7.23 (m, 3 H), 6.89 (m, 4 H), 4.09 (s, 3 H), 2.81 (s, 4 H), 2.37 (s, 4 H), 2.20 (s, 3 H) | 460 | Intermediate 82 |
| **83** | 7-Ethoxy-4-[(2-fluoro-5-methylphenyl)amino]-6-(4-methylpiperazin-1-yl)quinoline-3-carboxamide | CD₂Cl₂ 10.47 (s, 1 H), 8.76 (s, 1 H), 7.26 (s, 1 H), 7.01 (m, 1 H), 6.95 (s, 1 H), 6.85 (m, 1 H), 6.76 (d, 1 H), 6.02 (br s, 2 H), 4.20 (q, 2 H), 2.81 (m, 4 H), 2.48 (m, 4 H), 2.29 (s, 3 H), 2.19 (s, 3 H), 1.49 (t, 3 H) | 438 | Intermediate 83 |
| **84** | 4-[(3-Chloro-2-methylphenyl)amino]-7-ethoxy-6-(4-ethyl piperazin-1-yl)quinoline-3-carboxamide | 10.80 (s, 1 H), 8.86 (s, 1 H), 8.30 (s, 1 H), 7.65 (s, 1 H), 7.20 (m, 2 H), 7.06 (m, 1 H), 6.65 (s, 2 H), 4.15 (q, 2 H), 2.66 (m, 4 H), 2.50 (s, 3 H), 2.35 (m, 6 H), 1.40 (t, 3 H), 0.98 (t, 3 H) | 467 | Intermediate 84 |
| **85** | 4-[(3-Chloro-2-methylphenyl)amino]-7-ethoxy-6-(4-methyl-1,4-diazepan-1-yl)quinoline-3-carboxamide | CD₂Cl₂ 10.46 (s, 1 H), 8.68 (s, 1 H), 7.19 (s, 1 H), 7.12 (d, 1 H), 6.96 (m, 1 H), 6.68 (d, 1 H), 6.63 (s, 1 H), 6.05 (br s, 2 H), 4.17 (q, 2 H), 3.08 (m, 2 H), 2.93 (m, 2 H), 2.52 (m, 4 H), 2.46 (s, 3 H), 2.29 (s, 3 H), 1.68 (m, 2 H), 1.49 (t, 3 H) | 467 | Intermediate 85 |
| **86** | 7-Ethoxy-6-(4-ethylpiperazin-1-yl)-4-[(3-fluoro-2-methylphenyl)amino] quinoline-3-carboxamide | 10.80 (s, 1 H), 8:90 (s, 1 H), 8.34 (s, 1 H), 7.70 (s, 1 H), 7.30 (s, 1 H), 7.12 (m, 1 H), 6.95 (m, 1 H), 6.75 (s, 1 H), 6.54 (m, 1 H), 4.21 (q, 2 H), 2.66 (m, 4 H), 2.55 (s, 3 H), 2.40 (m, 6 H), 1.45 (t, 3 H), 1.05 (t, 3 H) | 451 | Intermediate 86 |
| **87** | 7-Ethoxy-4-[(3-fluoro-2-methylphenyl)amino]-6-(4-methyl-1,4-diazepan-1-yl)quinoline-3-carboxamide | CD₂Cl₂ 10.30 (s, 1 H), 8.60 (s, 1 H), 7.10 (s, 1 H), 6.85 (m, 1 H), 6.65 (m, 1 H), 6.56 (s, 1 H), 6.42 (d, 1 H), 4.05 (q, 2 H), 3.00 (m, 2 H), 2.80 (m, 2 H), 2.49 (m, 2 H), 2.40 (m, 2 H), 2.20 (s, 6 H), 1.69 (m, 2 H), 1.36 (t, 3 H) | 451 | Intermediate 87 |
| **88** | 7-Ethoxy-4-[(2-fluoro-4-methylphenyl)amino]-6-(4-methylpiperazin-1-yl)quinoline-3-carboxamide | 10.79 (s, 1 H), 8.83 (s, 1 H), 8.26 (s, 1 H), 7.63 (s, 1 H), 7.20 (s, 1 H), 7.09 (d, 1 H), 6.91 (m, 2 H), 6.80 (s, 1 H), 4.17 (q, 2 H), 2.68 (s, 4 H), 2.32 (s, 4 H), 2.27 (s, 3 H), 2.15 (s, 3 H), 1.39 (t, 3 H) | 439 | Intermediate 88 |
| **89** | 7-Ethoxy-4-[(3-methoxy-2-methylphenyl)amino]-6-(4-methylpiperazin-1-yl)quinoline-3-carboxamide | 10.89 (s, 1 H), 8.83 (s, 1 H), 8.28 (s, 1 H), 7.58 (s, 1 H), 7.16 (s, 1 H), 7.05 (m, 1 H), 6.77 (d, 1 H), 6.70 (s, 1 H), 6.38 (d, 1 H), 4.14 (q, 2 H), 3.80 (s, 3 H), 2.59 (s, 4 H), 2.27 (s, 4 H), 2.16 (s, 3 H), 2.14 (s, 3 H), 1.39 (t, 3 H) | 450 | Intermediate 89 |
| **90** | 4-[(2,5-Difluorophenyl) amino]-7-ethoxy-6-(4-methylpiperazin-1-yl)quinoline-3-carboxamide | CD₃OD 8.82 (s, 1 H), 7.29 (s, 1 H), 7.19 (m, 1 H), 7.03 (s, 1 H), 6.79 (m, 1 H), 6.58 (m, 1 H), 4.25 (q, 2 H), 2.93 (m, 4 H), 2.55 (m, 4 H), 2.30 (s, 3 H), 1.52 (% 3 H) | 442 | Intermediate 90 |
| **91** | 4-[(2,5-Difluorophenyl) amino]-7-ethoxy-6-(4-ethylpiperazin-1-yl)quinoline-3-carboxamide | CD₃OD 8.82 (s, 1 H), 7.29 (s, 1 H), 7.20 (m, 1 H), 7.04 (s, 1 H), 6.79 (m, 1 H), 6.58 (m, 1 H), 4.25 (q, 2 H), 2.95 (m, 4 H), 2.59 (m, 4 H), 2.48 (q, 2 H), 1.52 (t, 3 H), 1.11 (t, 3 H) | 456 | Intermediate 91 |
| **92** | 4-[(2,5-Difluorophenyl) amino]-7-ethoxy-6-(4-methyl-1,4-diazepan-1-yl)quinoline-3-carboxamide (t,3H) | CD₃OD 8.76 (s, 1 H), 7.26 (s, 1 H), 7.17 (m, 1 H), 6.88 (s, 1 H), 6.76 (m, 1 H), 6.51 (m, 1 H), 4.25 (q, 2 H), 3.29 (m, 2 H), 3.08 (m, 2 H), 2.80 (m, 2 H), 2.68 (m, 2 H), 2.37 (s, 3 H), 1.93 (m, 2 H), 1.54 | 456 | Intermediate 92 |
| **93** | 7-Ethoxy-6-(4-ethylpiperazin-1-yl)-4-[(2-fluoro-4-methylphenyl)amino] quinoline-3-carboxamide | 10.79 (s, 1 H), 8.84 (s, 1 H), 8.27 (s, 1 H), 7.63 (s, 1 H), 7.20 (s, 1 H), 7.13 (d, 1 H), 6.90 (m, 2 H), 6.80 (s, 1 H), 4.16 (q, 2 H), 2.68 (s, 4 H), 2.36 (s, 4 H), 2.31 (s, 2 H), 2.27 (s, 3 H), 1.38 (t, 3 H), 0.97 (t, 3 H) | 453 | Intermediate 93 |
| **94** | 4-[(3,4-Dimethylphenyl) amino]-7-ethoxy-6-(4-methylpiperazin-1-yl)quinoline-3-carboxamide | 10.74 (s,1 H), 8.80 (s, 1 H), 8.18 (s, 1 H), 7.54 (s, 1 H), 7.16 (s, 1 H), 7.03 (d, 1 H), 6.85 (s, 1 H), 6.79 (s, 1 H), 6.72 (d, 1 H), 4.15 (q, 2 H), 2.65 (s, 4 H), 2.29 (s, 4 H), 2.16 (s, 3 H), 2.14 (s, 3 H), 2.13 (s, 3 H), 1.38 (t, 3 H) | 435 | Intermediate 94 |
| **95** | 4-[(2,4-Difluorophenyl) amino]-6-ethoxy-7-(4-methylpiperazin-1-yl)quinoline-3-carboxamide | 10.55 (s, 1 H), 8.83 (s, 1 H), 8.25 (s, 1 H), 7.63 (s, 1 H), 7.35 (m, 1 H), 7.19 (s, 1 H), 6.98 (d, 2 H), 6.80 (s, 1 H), 3.65 (q, 2 H), 3.15 (s, 4 H), 2.48 (s, 4 H), 2.22 (s, 3 H), 1.21 (t, 3 H) | 442 | Intermediate 95 |
| **96** | 4-[(2,3-Dichlorophenyl) amino]-6-ethoxy-7-(4-methylpiperazin-1-yl)quinoline-3-carboxamide | 10.65 (s, 1 H), 8.91 (s, 1 H), 8.35 (s, 1 H), 7.75 (s, 1 H), 7.25 (s, 1 H), 7.22 (d, 1 H), 7.14 (m, 1 H), 6.66 (s, 1 H), 6.54 (d, 1 H), 3.66 (q, 2 H), 3.17 (s, 4 H), 2.49 (s, 4 H), 2.23 (s, 3 H), 1.21 (t, 3 H) | 474 | Intermediate 96 |
| **97** | 4-[(2,3-Difluorophenyl) amino]-7-ethoxy-6-(4-methylpiperazin-1-yl)quinoline-3-carboxamide | 10.65 (s, 1 H), 8.89 (s, 1 H), 8.30 (s, 1 H), 7.70 (s, 1 H), 7.25 (s, 1 H), 7.05 (m, 2 H), 6.85 (s, 1 H), 6.65 (m, 1 H), 4.16 (q, 2 H), 2.75 (m, 4 H), 2.35 (m, 4 H), 2.15 (s, 3 H), 1.40 (t, 3 H) | 441 | Intermediate 97 |
| **98** | 4-[(2,3-Dimethylphenyl) amino]-7-ethoxy-6-(4-methylpiperazin-1-yl)quinoline-3-carboxamide | 10.98 (s, 1 H), 8.85 (s, 1 H), 8.25 (s, 1 H), 7.55 (s, 1 H), 7.16 (s, 1 H), 7.00 (m, 2 H), 6.65 (m, 2 H), 4.15 (q, 2 H), 2.60 (m, 4 H), 2.30 (m, 7 H), 2.20 (s, 3 H), 2.15 (s, 3 H), 1.40 (t, 3 H) | 433 | Intermediate 98 |
| **99** | 4-[(4-Chloro-3-fluorophenyl)amino]-7-ethoxy-6-(4-methylpiperazin-1-yl)quinoline-3-carboxamide | 10.15 (s, I H), 8.80 (s, 1 H), 8.12 (s, 1 H), 7.60 (s, 1 H), 7.39 (m, 1 H), 7.28 (s, 1 H), 6.98-6.90 (m, 2 H), 6.70 (d, 1 H), 4.20 (q, 2 H), 2.90 (m, 4 H), 2.40 (m, 4 H), 1.40 (t, 3 H) | 458 | Intermediate 99 |
| **100** | 4-[(2,3-Dichlorophenyl) amino]-6-ethoxy-7-(4-ethylpiperazin-1-yl)quinoline-3-carboxamide | 10.65 (s, 1 H), 8.92 (br s, 1 H), 8.35 (s, 1 H), 7.74 (s, 1 H), 7.23 (d, 2 H), 7.14 (m, 1 H), 6.66 (s, 1 H), 6.54 (d, 1 H), 3.66 (q, 2 H), 3.18 (s, 4 H), 2.51 (s, 4 H), 2.37 (q, 2 H), 1.21 (t, 3 H), 1.03 (t, 3 H) | 488 | Intermediate 100 |
| **101** | 4-[(2,4-Difluorophenyl) amino]-6-ethoxy-7-(4-ethylpiperazin-1-yl)quinoline-3-carboxamide | 10.57 (s, 1 H), 8.85 (s, 1 H), 8.27 (s, 1 H), 7.64 (s, 1 H), 7.37 (m, 1 H), 7.21 (s, 1 H), 6.99 (m, 2 H), 6.81 (s, 1 H), 3.66 (q, 2 H), 3.17 (s, 4 H), 2.51 (s, 4 H), 2.39 (q, 2 H), 1.21 (t, 3 H), 1.04 (t, 3 H) | 456 | Intermediate 101 |
| **102** | 4-[(3-Chloro-2,4-difluorophenyl)amino]-7-ethoxy-6-(4-ethyl piperazin-1-yl)quinoline-3-carboxamide | 10.60 (s, 1 H), 8.85 (s, 1 H), 8.27 (s, 1 H), 7.65 (s, 1 H), 7.25 (m, 2 H), 6.95 (m, 1 H), 6.85 (s, 1 H), 4.20 (q, 2 H), 2.80 (m, 4 H), 2.40 (m, 4 H), 2.31 (m, 2 H), 1.40 (t, 3 H), 1.00 t, 3 H) | 489 | Intermediate 102 |
| **103** | 4-[(3-Chloro-2,4-difluorophenyl)ammo]-7-ethoxy-6-(4-methylpiperazin-1-yl)quinoline-3-carboxamide | 10.60 (s, 1 H), 8.83 (s, 1 H), 8.28 (s, 1 H), 7.65 (s, 1 H), 7.25 (m, 2 H), 7.00 (m, 1H), 6.88 (s, 1 H), 4.20 (q, 2 H), 3.80 (m, 4 H), 2.39 (m, 4 H), 2.20 (s, 3 H), 1.40 (t, 3 H) | 475 | Intermediate 103 |
| **104** | 4-[(3-Chloro-4-fluorophenyl)amino]-6-ethoxy-7-(4-methylpiperazin-1-yl)quinoline-3-carboxamide | 10.12 (s, 1 H), 8.82 (s, 1 H), 8.15 (s, 1 H), 7.59 (s, 1 H), 7.27 (m, 2 H), 7.14 (d, 1 H), 6.97 (s, 1 H), 6.88 (d, 1 H), 3.81 (q, 2 H), 3.19 (s, 4 H), 2.48 (s, 4 H), 2.25 (s, 3 H), 1.27 (t, 3 H) | 458 | Intermediate 104 |
| **105** | 4-[(3-Chloro-4-fluorophenyl)amino]-6-ethoxy-7-(4-ethylpiperazin-1-yl)quinoline-3-carboxamide | 10.17 (s, 1 H), 8.86 (s, 1 H), 8.19 (s, 1 H), 7.63 (s, 1 H), 7.34 (m, 1 H), 7.29 (s, 1 H), 7.19 (d, 1 H), 7.01 (s, 1 H), 6.92 (d, 1 H), 3.85 (q, 2 H), 3.24 (s, 4 H), 2.61 (s, 4 H), 2.44 (q, 2 H), 1.32 (t, 3 H), 1.10 (t, 3 H) | 472 | Intermediate 105 |
| **106** | 4-{[4-Fluoro-2-(trifluoromethyl)phenyl]a mino}-7-methoxy-6-(4-methylpiperazin-1- yl)quinoline-3-carboxamide | THF-d8 11.14 (s, 1 H), 8.75 (s, 1 H), 7.58 (s, 1 H), 7.42 (m, 1 H), 7.20 (s, 1 H), 7.02 (m, 1 H), 6.88 (s, 1 H), 6.64 (m, 1 H), 6.58 (s, 1 H), 3.83 (s, 3 H), 2.72 (s, 4 H), 2.23 (s, 4 H), 2.08 (s, 3 H) | 478 | Intermediate 106 |
| **107** | 4-[(2-Chloro-4-fluorophenyl)amino]-7-methoxy-6-(4-methylpiperazin-1-yl)quinoline-3-carboxamide | 10.95 (s, 1 H), 8.83 (s, 1 H), 7.63 (s, 1 H), 7.32 (m, 1 H), 7.30 (s, 1 H), 6.92 (s, 1 H), 6.87 (m, 1 H), 6.74 (s, 1 H), 6.71 (m, 1 H), 3.95 (s, 3 H), 2.79 (s, 4 H), 2.37 (s, 4 H), 2.19 (s, 3 H) | 444 | Intermediate 107 |
| **108** | 7-Methoxy-6-(4-methylpiperazin-1-yl)-4-{[3-(trifluoromethoxy) phenyl]amino}quinoline-3-carboxamide | 10.37 (s, 1 H), 8.86 (s, 1 H), 8.23 (s, 1 H), 7.65 (s, 1 H), 7.31-7.38 (m, 2 H), 6.85-6.96 (m, 4 H), 3.94 (s, 3 H), 2.76 (s, 4 H), 2.35 (s, 4 H), 2.16 (s, 3 H) | 476 | Intermediate 108 |
| **109** | 4-[(2,6-Dimethylphenyl)amino]-7-methoxy-6-(4-methylpiperazin-1-yl)quinoline-3-carboxamide | 11.39 (s, 1 H), 8.82 (s, 1 H), 8.21 (s, 1 H), 7.51 (s, 1 H), 7.15-7.20 (m, 4 H), 6.64 (s, 1 H), 3.87 (s, 3 H), 2.44 (s, 4 H), 2.28 (s, 4 H), 2.15 (s, 3 H), 2.06 (s, 6 H) | 420 | Intermediate 109 |
| **110** | 4-[(2,4-Difluorophenyl) amino]-7-ethoxy-6-(4-ethyl-1,4-diazepan-1-yl)quinoline-3-carboxamide | 10.55 (s, 1 H), 8.81 (s, 1 H), 8.30 (s, 1 H), 7.80 (s, 1 H), 7.45 (m, 3 H), 7.20 (m, 2 H), 4.25 (m, 2 H), 3.15 (m, 6 H), 2.80 (m, 2 H), 2.30 (m, 2 H), 2.10 (m, 2 H), 1.50 (t, 3 H), 1.25 (t, 3 H) | 469 | Intermediate 110 |
| **111** | 4-[(2,3-Dichlorophenyl) amino]-6-(4-ethyl-1,4-diazepan-1-yl)-7-methoxyquinoline-3-carboxamide | 9.00 (s, 1 H), 8.51 (s, 1 H), 7.95 (s, 1 H), 7.60 (m, 1 H), 7.48 (s, 1 H), 7.40-7.32 (m, 2 H), 6.89 (s, 1 H), 4.00 (s, 3 H), 3.20 (m, 4 H), 2.95 (m, 2 H), 2.70 (m, 2 H), 2.20 (m, 2 H), 2.05 (m, 2 H), 1.29 (t, 3 H) | 487 | Intermediate 111 |
| **112** | 4-[(2,3-Dichlorophenyl) amino]-7-ethoxy-6-(4-ethyl-1,4-diazepan-1-yl)quinoline-3-carboxamide | 10.00 (s, 1 H), 8.90 (s, 1 H), 8.40 (s, 1 H), 7.90 (s, 1 H), 7.65 (d, 1 H), 7.30 (m, 3 H), 6.80 (s, 1 H), 4.15 (q, 2 H), 3.15 (m, 4 H), 2.85 (m, 2 H), 2.60 (m, 2 H), 2.15 (m, 2 H), 2.00 (m, 2 H), 1.40 (t, 3 H), 1.15 (t, 3 H) | 501 | Intermediate 112 |
| **113** | 4-[(2,4-Difluorophenyl) amino]-7-isopropoxy-6-(4-methylpiperazin-1-yl)quinoline-3-carboxamide | 10.74 (s, 1 H), 8.83 (s, 1 H), 8.28 (s, 1 H), 7.65 (s, 1 H), 7.36 (t, 1 H), 7.23 (s, 1 H), 7.02 (in, 2 H), 6.79 (s, 1 H), 4.81 (m, 1 H), 2.71 (s, 4 H), 2.35 (s, 4 H), 2.16 (s, 3 H), 1.34 (d, 6 H) | 457 | Intermediate 254 |
| **114** | 4-[(3,4-Dichlorophenyl) amino]-7-isopropoxy-6-(4-methylpiperazin-1-yl)quinoline-3-carboxamide | 10.13 (s, 1 H), 8.78 (s, 1 H), 8.12 (s, 1 H), 7.59 (s, 1 H), 7.46 (d, 1 H), 7.28 (s, 1 H), 7.11 (s, 1 H), 6.96 (s, 1 H), 6.87 (m, 1 H), 4.83 (m, 1 H), 2.86 (s, 4 H), 2.39 (s, 4 H), 2.18 (s, 3 H), 1.36 (d, 6 H) | 490 | Intermediate 255 |
| **115** | 4-[(3-Chloro-2-fluorophenyl)amino]-7-isopropoxy-6-(4-methylpiperazin-1-yl)quinoline-3-carboxamide | 10.66 (s, 1 H), 8.86 (s, 1 H), 8.29 (s, 1 H), 7.69 (s, 1 H), 7.29 (s, 1 H), 7.21 (t, 1 H), 7.07 (t, 1 H), 6.88 (s, 1 H), 6.76 (m, 1 H), 4.85 (m, 1 H), 2.83-2.50 (m, 8 H), 2.36 (s, 3 H), 1.37 (d, 6 H) | 474 | Intermediate 256 |
| **116** | 4-[(2,3-Dichlorophenyl) amino]-7-isopropoxy-6-(4-methylpiperazin-1-yl)quinoline-3-carboxamide | 10.83 (s, 1 H), 8.92 (s, 1 H), 8.38 (s, 1 H), 7.77 (s, 1 H), 7.30 (s, 1 H), 7.26 (s, 1 H), 7.16 (m, 1 H), 6.67 (s, 1 H), 6.62 (d, 1 H), 4.85 (m, 1 H), 2.74 (s, 4 H), 2.36 (s, 4 H), 2.17 (s, 3 H), 1.3 7 (d, 6 H) | 490 | Intermediate 257 |
| **117** | 4-[(3-Chloro-4-fluorophenyl)amino]-7-isopropoxy-6-(4-methylpiperazin-1-yl)quinoline-3-carboxamide | 10.37 (s, 1 H), 8.81 (s, 1 H), 8.18 (s, 1 H), 7.61 (s, 1 H), 7.31 (m, 1 H), 7.27 (s, 1 H), 7.12 (m, 1 H), 6.91 (s, 2 H), 4.86 (m, 1 H), 2.82 (s, 4 H), 2.41 (s, 4 H), 2.20 (s, 3 H), 1.36 (d, 6 H) | 474 | Intermediate 258 |
| **118** | 4-[(2,4-Difluorophenyl) amino]-6-morpholin-4-ylquinoline-3-carboxamide | 10.39 (s, 1 H), 8.80 (s, 1 H), 8.26 (s, 1 H), 7.82 (d, 1 H), 7.69 (s, 1 H), 7.59 (d, 1 H), 7.35 (t, 1 H), 7.01 (m, 2 H), 6.86 (s, 1 H), 3.68 (m, 4 H), 2.94 (m, 4 H) | 384 | Intermediate 259 |
| **119** | 4-[(3-Chloro-4-fluorophenyl)amino]-6- 1 morpholin-4-ylquinoline-3-carboxamide | 9.83 (s, 1 H), 8.72 (s, 1 H), 8.09 (s, H), 7.84 (d, 1 H), 7.64 (d, 1 H), 7.59 (m, 1 H), 7.28 (t, 1 H), 7.10 (m, 1 H), 7.01 (s, 1 H), 6.86 (m, 1 H), 3.71 (m, 4 H), 3.05 (m, 4 H) | 401 | Intermediate 260 |
| **120** | 4-[(2,3-Dichlorophenyl) amino]-6-morpholin-4-ylquinoline-3-carboxamide | 10.56 (s, 1 H), 8.91 (s, 1 H), 8.42 (s, 1 H), 7.89 (d, 2 H), 7.65 (m, 1 H), 7.25 (m, 1 H), 7.14 (t, 1 H), 6.66 (s, 1 H), 6.55 (d, 1 H), 3.66 (m, 4 H), 2.94 (m, 4 H) | 417 | Intermediate 261 |
| **121** | 4-[(2,4-Difluorophenyl) amino]-6-(4-methylpiperazin-1-yl)quinoline-3-carboxamide | 10.40 (s, 1 H), 8.77 (s, 1 H), 8.27 (s, 1 H), 7.77 (s, 1 H), 7.67 (s, 1 H), 7.55 (d, 1 H), 7.33 (t, 1 H), 6.99 (m, 2 H), 6.79 (s, 1 H), 2.94 (m, 4 H), 2.34 (m, 4 H), 2.16 (s, 3 H) | 398 | Intermediate 262 |
| **122** | 4-[(2,4-Dichlorophenyl) amino]-6-(4-methylpiperazin-1-yl)quinoline-3-carboxamide | 10.52 (s, 1 H), 8.86 (s, 1 H), 8.37 (s, 1 H), 7.82 (m, 2 H), 7.70 (d, 1 H), 7.60 (m, 1 H), 7.21 (m, 1 H), 6.58 (m, 2 H), 2.96 (m, 4 H), 2.34 (m, 4 H), 2.16 (s, 3 H) | 430 | Intermediate 263 |
| **123** | 4-[(3,4-Dichlorophenyl) amino]-6-(4-methylpiperazin-1-yl)quinoline-3-carboxamide | 9.67 (s, 1 H), 8.69 (s, 1 H), 8.05 (s, 1 H), 7.83 (d, 1 H), 7.61 (m, 2 H), 7.43 (d, 1 H), 7.05 (m, 2 H), 6.84 (m, 1 H), 3.16 (m, 4 H), 2.48 (m, 4 H), 2.25 (s, 3 H) | 430 | Intermediate 264 |
| **124** | 4-[(2,3-Dichlorophenyl) amino]-6-(4-methylpiperazin-1-yl)quinoline-3-carboxamide | CD₃OD 8.83 (s, 1 H), 7.89 (d, 1 H), 7.83 (d, 1 H), 7.55 (d, 1 H), 7.39 (m, 2 H), 7.08 (d, 1 H), 3.62 (m, 2 H), 3.54 (m, 2 H), 3.21 (m, 2 H), 3.06 (m, 2 H), 2.90 (s, 3 H) | 429 | Intermediate 265 |
| **125** | tert-Butyl 4-{3-(aminocarbonyl)-4-[(2,3-dichlorophenyl)amino]-7-ethoxyquinolin-6-yl}piperazine-1-carboxylate | CD₂Cl₂ 10.20 (s, 1 H), 8.65 (s, 1 H), 7.15 (s, 1 H), 6.95 (d, 1H), 6.80 (m, 1 H), 6.60 (s, 1 H), 6.40 (d, 1 H), 4.05 (q, 2 H), 3.25 (m, 4 H), 2.60 (m, 4 H), 1.32 (t, 3 H), 1.28 (s, 9 H) | 560 | Intermediate 113 |
| **126** | tert-Butyl 4-{3-(aminocarbonyl)-4-[(2,4-difluorophenyl)amino]-7-methoxyquinolin-6-yl}piperazine-1-carboxylate | 10.60 (s, 1 H), 8.78 (s, 1 H), 8.20 (s, 1 H), 7.60 (s, 1 H), 7.30 (m, 1 H), 7.22 (s, 1 H), 6.98 (m, 2 H), 6.80 (s, 1 H), 3.86 (s, 3 H), 3.25 (m, 4 H), 2.59 (m, 4 H), 1.31 (s, 9 H) | 513 | Intermediate 114 |
| **127** | tert-Butyl 4-{3-(aminocarbonyl)-4-[(2,4-difluorophenyl)amino]-7-methoxyquinolin-6-yl}-1,4-diazepane-1-carboxylate | | 527 | Intermediate 115 |
| **128** | tert-Butyl 4-{3-(aminocarbonyl)-4-[(2,3-dichlorophenyl)amino]-7-ethoxyquinolin-6-yl}-1,4-diazepane-1-carboxylate | CDCl₃ 10.63 (s, 1 H), 8.75 (s, 1 H), 8.20 (s, 1 H), 7.88 (s, 1 H), 7.56 (s, 1 H), 7.30 (m, 1 H), 7.18 (m, 1 H), 6.90 (m, 1 H), 6.70 (m, 1 H), 3.85 (s, 3 H), 3.20 (m, 4 H), 3.13 (m, 1 H), 3.01 (m, 1 H), 2.95 (m, 2 H), 1.58 (m, 2 H), 1.25-1.18 (d, 9H) | 573 | Intermediate 116 |
| **129** | tert-Butyl 4-{3-(aminocarbonyl)-4-[(2,4-difluorophenyl)amino]-7-ethoxyquinolin-6-yl}piperazine-1-carboxylate | CDCl₃ 10.60 (s, 1 H), 8.85 (s, 1 H), 7.31 (s, 1 H), 6.90 (m, 3 H), 6.75 (m, 1 H), 4.20 (q, 2 H), 3.50 (m, 4 H), 2.75 (m, 4 H), 1.40 (t, 3 H), 1.38 (s, 9 H) | 527 | Intermediate 117 |
| **130** | tert-Butyl 4-{3-(aminocarbonyl)-4-[(2,4-difluorophenyl)amino]-7-ethoxyquinolin-6-yl}-1,4-diazepane-1-carboxylate | | 541 | Intermediate 118 |
| **131** | tert-Butyl 4-{3-(aminocarbonyl)-4-[(2,3-dichlorophenyl)amino]-7-methoxyquinolin-6-yl}-1,4-diazepane-1-carboxylate | | 560 | Intermediate 119 |
| **132** | 4-[(2-Fluoro-5-methylphenyl)amino]-7-methoxy-6-(4-methylpiperazin-1-yl)quinoline-3-carboxamide | 10.82 (s, 1 H), 8.88 (s, 1 H), 8.31 (s, 1 H), 7.68 (s, 1 H), 7.27 (s, 1 H), 7.17 (m, 1 H), 6.86 (m, 2 H), 6.72 (m, 1 H), 3.94 (s, 3 H), 2.69 (s, 4 H), 2.34 (s, 4 H), 2.17 (s, 3 H), 2.15 (s, 3 H) | 424 | Intermediate 120 |
| **133** | 4-[(2,5-Difluorophenyl)amino]-7-methoxy-6-(4-methylpiperazin-1-yl)quinoline-3-carboxamide | 10.68 (s, 1 H), 8.90 (s, 1 H), 8.33 (s, 1 H), 7.73 (s, 1 H), 7.33 (m, 2 H), 6.88 (m, 2 H), 6.64 (m, 1 H), 3.96 (s, 3 H), 2.78 (s, 4 H), 2.38 (s, 4 H), 2.18 (s, 3 H) | 428 | Intermediate 121 |
| **134** | 4-[(3-Chloro-2-methylphenyl)amino]-7-methoxy-6-(4-methylpiperazin-1-yl)quinoline-3-carboxamide | 10.86 (s, 1 H), 8.88 (s, 1 H), 8.31 (s, 1 H), 7.66 (s, 1 H), 7.24 (s, 1 H), 7.19 (m, 1 H), 7.08 (m, 1 H), 6.68 (m, 2 H), 3.92 (s, 3 H), 2.64 (s, 4 H), 2.38 (s, 3 H), 2.33 (s, 4 H), 2.17 (s, 3 H) | 440 | Intermediate 122 |
| **135** | 4-[(2-Chloro-3-methylphenyl)amino]-7-methoxy-6-(4-methylpiperazin-1-yl)quinoline-3-carboxamide | 10.81 (s, 1 H), 8.90 (s, 1 H), 8.32 (s, 1 H), 7.69 (s, 1 H), 7.27 (s, 1 H), 7.05 (d, 1 H), 7.03 (s, 1 H), 6.69 (s, 1 H), 6.54 (m, 1 H), 3.92 (s, 3 H), 2.66 (s, 4 H), 2.40 (s, 3 H), 2.33 (s, 4 H), 2.15 (s, 3 H) | 440 | Intermediate 123 |
| **136** | 4-[(2,4-Difluorophenyl) amino]-6-[3-(dimethylamino)pyrrolidi n-1-yl]-7-methoxy quinoline-3-carboxamide | THF-d₈ 10.76 (s, 1 H), 8.62 (s, 1 H), 7.50 (s, 1 H), 7.14 (s, 1 H), 6.93 (m, 1 H), 6.71 (m, 3 H), 6.37 (s, 1 H), 3.82 (s, 3 H), 3.20 (m, 1 H), 3.00 (t, 1 H), 2.89 (t, 1 H), 2.43 (m, 2 H), 2.04 (s, 6 H), 1.85 (m, 1 H), 1.56 (m, 1 H) | 442 | Intermediate 124 |
| **137** | 4-[(3-Chloro-2-fluorophenyl)amino]-7-methoxy-6-(4-methylpiperazin-1-yl)quinoline-3-carboxamide | 10.69 (s, 1 H), 8.87 (s, 1 H), 8.30 (s, 1 H), 7.71 (s, 1 H), 7.30 (s, 1 H), 7.24 (m, 1 H), 7.07 (m, 1 H), 6.85 (s, 1 H), 6.79 (m, 1 H), 3.94 (s, 3 H), 2.74 (s, 4 H), 2.36 (s, 4 H), 2.17 (s, 3 H) | 444 | Intermediate 125 |
| **138** | 4-[(3-Chloro-5-fluorophenyl)amino]-7-ethoxy-6-(4-methyl piperazin-1-yl)quinoline-3-carboxamide | CDCl₃ 10.37 (s, 1 H), 8.75 (s, 1 H), 7.30 (s, 1 H), 6.92 (s, 1 H), 6.71 (m, 2 H), 6.51 (d, 1 H), 4.23 (q, 2 H), 2.93 (s, 4 H), 2.53 (s, 4 H), 2.31 (s, 3 H), 1.54 (t, 3 H) | 459 | Intermediate 126 |
| **139** | 7-Ethoxy-6-(4-methylpiperazin-1-yl)-4-[(2,3,4-trifluorophenyl) amino]quinoline-3-carboxamide | CDCl₃ 10.41 (s, 1 H), 8.74 (s, 1 H), 7.28 (s, 1 H), 6.87 (s, 1 H), 6.79 (m, 1 H), 6.61 (m, 1 H), 4.21 (q, 2 H), 2.87 (s, 4 H), 2.52 (s, 4 H), 2.31 (s, 3 H), 1.52 (t, 3 H) | 460 | Intermediate 127 |
| **140** | 4-[(5-Chloro-2-methylphenyl)amino]-7-ethoxy-6-(4-methylpiperazin-1-yl)quinoline-3-carboxamide | CDCl₃ 10.46 (s, 1 H), 8.71 (s, 1 H), 7.26 (s, 1 H), 7.16 (d, 1 H), 6.97 (dd, 1 H), 6.79 (m, 2 H), 4.21 (q, 2 H), 2.80 (s, 4 H), 2.49 (s, 4 H), 2.35 (s, 3 H), 2.30 (s, 3 H), 1.52 (t, 3 H) | 455 | Intermediate 128 |
| **141** | 7-Ethoxy-4-[(4-methoxy- CDCl₃ 2-methylphenyl)amino]- H), 7.18 6-(4-methylpiperazin-1- 6.78 (d, yl)quinoline-3- (br s, 2 carboxamide H), 2.71 2.29 (s, 3 H) | 10.68 (s, 1 H), 8.65 (s, 1 (s, 1 H, 6.89 (m, 2 H), 1 H), 6.62 (dd, 1 H), 5.92 H), 4.15 (q, 2 H), 3.75 (s, 3 (s, 4 H), 2.44 (s, 4 H), 3 H), 2.25 (s, 3 H), 1.48 (t, | 451 | Intermediate 129 |
| **142** | 4-{[2-Chloro-5- CDCl₃ (trifluoromethyl)phenyl] H), 7.55 amino}-7-ethoxy-6-(4- 7.15 methylpiperazin-1- (s, 1 H), yl)quinoline-3- H), 2.86 carboxamide 2.32 (s, | 10.43 (s, 1 H), 8.81 (s, 1 (d, 1 H), 7.35 (s, 1 H), (dd, 1 H), 6.88 (s, 1 H), 6.77 6.00 (br s, 2 H), 4.23 (q, 2 (s, 4 H), 2.50 (s, 4 H), 3 H), 1.53 (t, 3 H) | 509 | Intermediate 130 |
| **143** | 4-[(2,4- 10.26 Difluorophenyl)amino]- (s, 1 H), 7-ethoxy-6-(4-methyl-3- H), 6.95 oxopiperazin-1- 6.53 (s, yl)quinoline-3- 3.04 (m, carboxamide (s, 2 H), | (s, 1 H), 8.50 (s, 1 H), 7.91 7.30 (s, 1 H), 7.01 (t, 1 (s, 1 H), 6.64 (m, 2 H), 1 H), 3.87 (q, 2 H), 2.91 - 5 H), 2.82 (s, 2 H), 2.16 1.08 (t, 3 H) | 474 | Intermediate 131 |
| **144** | 4-[(2,3- 10.71 Dichlorophenyl)amino]- (s, 1 H), 7-(4-ethylpiperazin-1-yl)- H), 7.23 6-methoxyquinoline-3- 6.67 (s, carboxamide (m, 4 H), 1.02 | (s, 1 H), 8.92 (s, 1 H), 8.36 7.75 (s, 1 H), 7.25 (s, 1 (d, 1 H), 7.15 (m, 1 H), 1 H), 6.57 (d, 1 H), 3.16 H), 2.52 (m, 4 H), 2.37 (q, 2 (t, 3 H) | 474 | Intermediate 132 |
| **145** | 4-[(2-Fluoro-5- CD₂Cl₂ methylphenyl)amino]-6- H), 7.30 methoxy-7-(4- 6.87 (m, methylpiperazin-1- (br, 2 yl)quinoline-3- H), 2.55 carboxamide 2.19 (s, | 10.37 (s, 1 H), 8.76 (s, 1 (s, 1 H), 7.01 (m, 1 H), 2 H), 6.76 (d, 1 H), 6.09 H), 3.43 (s, 3 H), 3.22 (s, 4 (s, 4 H), 2.30 (s, 3 H), 3 H) | 424 | Intermediate 133 |
| **146** | 7-(4-Ethylpiperazin-1-yl)4-[(2-fluoro-5-methylphenyl)amino]-6-methoxyquinoline-3-carboxamide | CD₂Cl₂ 10.36 (s, 1 H), 8.76 (s, 1 H), 7.31 (s, 1 H), 7.01 (m, 1 H), 6.87 (m, 2 H), 6.77 (d, 1 H), 6.06 (br, 2 H), 3.43 (s, 3 H), 3.23 (s 4 H), 2.60 (s, 4 H), 2.44 (q, 2 H), 2.19 (s, 3 H), 1.09 (t, 3 H) | 438 | Intermediate 134 |
| **147** | 4-[(3-Chloro-2-fluorophenyl)amino]-6-methoxy-7-(4-methylpiperazin-1-yl)quinoline-3-carboxamide | CD₂Cl₂ 10.33 (s, 1 H), 8.78 (s, 1 H), 7.34 (s, 1 H), 7.06 (m, 1 H), 6.91 (m, 1 H), 6.81 (s, 1 H), 6.71 (m, 1 H), 6.04 (br, 2 H), 3.51 (s, 3 H), 3.24 (s, 4 H), 2.55 (s, 4 H), 2.31 (s, 3 H) | 444 | Intermediate 135 |
| **148** | 4-[(3-Chloro-2-fluorophenyl)amino]-7-(4-ethylpiperazin-1-yl)-6-methoxyquinoline-3-carboxamide | CD₂Cl₂ 10.33 (s, 1 H), 8.78 (s, 1 H), 7.34 (s, 1 H), 7.06 (m, 1 H), 6.91 (m, 1 H), 6.82 (s, 1 H), 6.71 (m, 1 H), 6.02 (br, 2 H), 3.51 (s, 3 H), 3.25 (s, 4 H), 2.60 (s, 4 H), 2.45 (q, 2 H), 1.09 (1, 3 H) | 458 | Intermediate 136 |
| **149** | 4-[(2-Fluoro-5-methylphenyl)amino]-6-methoxy-7-(4-methyl-1,4-diazepan-1-yl)quinoline-3-carboxamide | CD₂Cl₂ 10.35 (s, 1 H), 8.73 (s, 1 H), 7.15 (s, 1 H), 7.01 (m, 1 H), 6.82 (m, 2 H), 6.77 (d, 1 H), 6.16 (br, 2 H), 3.48 (m, 4 H), 3.40 (s, 3 H), 2.77 (m, 2 H), 2.64 (m, 2 H), 2.35 (s, 3 H), 2.19 (s, 3 H), 2.03 (m, 2 H) | 437 | Intermediate 137 |
| **150** | 4-[(2,4-Difluorophenyl)amino]-6-methoxy-7-(4-methyl-1,4-diazepan-1-yl)quinoline-3-carboxamide | CD₂Cl₂ 10.36 (s, 1 H), 8.70 (s, 1 H), 7.16 (s, 1 H), 6.94 (m, 2 H), 6.79 (m, 1 H), 6.73 (s, 1 H), 5.91 (br, 2 H), 3.49 (m, 4 H), 3.44 (s, 3 H), 2.76 (m, 2 H), 2.63 (m, 2 H), 2.35 (s, 3 H), 2.02 (m, 2 H) | 441 | Intermediate 138 |
| **151** | 4-[(2-Chloro-3-fluorophenyl)amino]-7-ethoxy-6-(4-methylpiperazin-1-yl)quinoline-3-carboxamide | CD₂Cl₂ 10,39 (s, 1 H), 8.75 (s, 1 H), 7.28 (s, 1 H), 7.24 (m, 1 H), 6.87 (s, 1 H), 6.71 (m, 2 H), 5.95 (br, 2 H), 4.22 (q, 2 H), 2.86 (s, 4 H), 2.44 (s, 4 H), 2.26 (s, 3 H), 1.50 (t, 3 H) | 458 | Intermediate 139 |
| **152** | 4-[(2-Chloro-3-fluorophenyl)amino]-7-ethoxy-6-(4-ethylpiperazin-1-yl)quinoline-3-carboxamide | CD₂Cl₂ 10.38 (s, 1 H), 8.75 (s, 1 H) 7.27 (s, 1 H), 7.24 (m, 1 H), 6.87 (s, 1 H), 6.71 (m, 2 H), 6.10 (br, 2 H), 4.21 (q, 2 H), 2.87 (s, 4 H), 2.48 (s, 4 H), 2.39 (q, 2 H), 1.50 (t, 3 H), 1.05 (t, 3 H) | 472 | Intermediate 140 |
| **153** | 4-[(2-Chloro-3-fluorophenyl)amino]-7-ethoxy-6-(4-isopropylpiperazin-1-yl)quinoline-3-carboxamide | CD₂Cl₂ 10.36 (s, 1 H), 8.75 (s, 1 H), 7.28 (s, 1 H), 7.24 (m, 1 H), 6.87 (s, 1 H), 6.71 (m, 2 H), 5.98 (br, 2 H), 4.22 (q, 2 H), 2.85 (s, 4 H), 2.64 (m, 1 H), 2.57 (s, 4 H), 1.51 (t, 3 H), 1.03 (d, 6 H) | 486 | Intermediate 141 |
| **154** | *tert*-Butyl 4-{3-(aminocarbonyl)-7-ethoxy-4-[(2-fluoro-4-methylphenyl)amino]qui nolin-6-yl}piperazine-1-carboxylate | CD₂Cl₂ 10:61 (s, 1 H), 8.85 (s, 1 H), 7.29 (s, 1 H), 6.99 (d, 1 H), 6.90 (s, 1 H), 6.79 (m, 2 H), 4.20 (q, 2 H), 3.46 (m, 4 H), 2.71 (m, 4 H), 2.35 (s, 3 H), 1.51 (t, 3 H), 1.49 (s, 9 H) | 524 | Intermediate 142 |
| **155** | 4-[(2,3-Dichlorophenyl)amino]-7-ethoxy-6-(3-oxopiperazin-1-yl)quinoline-3-carboxamide | 10.80 (s, 1 H), 8.93 (s, 1 H), 8.38 (s, 1 H), 7.87 (s, 1 H), 7.76 (s, 1 H), 7.34 (s, 1 H), 7.24 (d, 1 H), 7.14 (m, 1 H), 6.69 (s, 1 H), 6.57 (d, 1 H), 4.22 (q, 2 H), 3.27 (s, 2 H), 3.18 (s, 2 H), 3.11 (s, 2 H), 1.42 (t, 3 H) | 474 | Intermediate 143 |
| **156** | 7-Ethoxy-4-[(2-fluoro-5-methylphenyl)amino]-6-(3-oxopiperazin-1-yl)quinoline-3-carboxamide | 10.72 (s, 1 H), 8.86 (s, 1H), 8.29 (s, 1H), 7.86 (s, 1 H), 7.64 (s, 1 H), 7.27 (s, 1H), 7.14 (m, 1H), 6.88 (s, 2 H), 6.73 (d, 1H), 4.20 (q, 2 H), 3.18 (m, 4 H), 3.03 (m, 2 H), 1.41 (t, 3 H) | 438 | Intermediate 144 |
| **157** | 4-[(2,4-Difluorophenyl) amino]-7-ethoxy-6-(3-oxopiperazin-1-yl)quinoline-3-carboxamide | MeOD 8.78 (s, 1H), 7.27 (s, 1H), 7.07 (m, 2 H), 7.03 (s, 1H), 6.92 (m, 1H), 4.25 (q, 2 H), 3.45 (s, 2 H), 3.34 (m, 2 H), 3.15 (m, 2 H), 1.51 (t, 3 H) | 442 | Intermediate 145 |
| **158** | 4-[(2-Chloro-4-methylphenyl)amino]-7-ethoxy-6-(4-methylpiperazin-1-yl)quinoline-3-carboxamide | 10.85 (s, 1H), 8.90 (s, 1H), 8.30 (s, 1 H), 7.69 (s, 1 H), 7.38 (s, 1 H), 7.20 (s, 1 H), 7.00 (d, 1 H), 6.65 (m, 2 H), 4.15 (q, 2 H), 2.69 (s, 4 H), 2.30 (s, 4 H), 2.20 (s, 3 H), 2.15 (s, 3 H), 1.40 (t, 3 H) | 454 | Intermediate 146 |
| **159** | 7-Ethoxy-4-{[2-fluoro-3-(trifluoromethyl)phenyl] amino}-6-(4-methyl piperazin-1-yl)quinoline-3-carboxamide | 10.65 (s, 1H), 8.85 (s, 1H), 8.30 (s, 1H), 7.70 (s, 1 H), 7.40-7.15 (m, 4 H), 6.85 (s, 1 H), 4.20 (q, 2 H), 2.80 (s, 4 H), 2.35 (s, 4 H), 2.15 (s, 3 H), 1.40 (t, 3 H) | 492 | Intermediate 147 |
| **160** | 4-[(2,4-Dimethoxy phenyl)amino]-7-ethoxy-6-(4-methylpiperazin-1-yl)quinoline-3-carboxamide | 10.75 (s, 1H), 8.80 (s, 1H), 8.20 (s, 1 H), 7.50 (s, 1H), 7.16 (s, 1 H), 6.85-6.70 (m, 3 H), 6.50 (d, 1 H), 4.15 (q, 2 H), 3.75 (s, 6H), 2.65 (s, 4 H), 2.30 (s, 4 H), 2.17 (s, 3H), 1.40 (t, 3H) | 466 | Intermediate 148 |
| **161** | 4-[(2-Chloro-4-fluoro-5-methylphenyl)amino]-7-ethoxy-6-(4-methyl piperazin-1-yl)quinoline-3-carboxamide | 10.95 (s, 1H), 8.96 (s, 1H), 8.40 (s, 1H), 7.75 (s, 1H), 7.55 (d, 1 H), 7.30 (s, 1 H), 6.80 (d, 1 H), 6.70 (s, 1H), 4.21 (q, 2 H), 2.80 (s, 4 H), 2.35 (s, 4 H), 2.20 (s, 3 H), 2.10 (s, 3 H), 1.45 (t, 3 H) | 472 | Intermediate 149 |
| **162** | 4-[(5-Chloro-2-fluorophenyl)amino]-7-ethoxy-6-(4-methyl piperazin-1-yl)quinoline-3-carboxamide | 10.70 (s, 1 H), 8.90 (s, 1 H), 8.35 (s, 1H), 7.75 (s, 1H), 7.30 (m, 2 H), 7.10 (m, 1 H), 6.85 (s, 1H), 6.80 (d, 1 H), 4.20 (q, 2 H), 3.80 (s, 4 H), 2.35 (s, 4 H), 2.20 (s, 3 H), 1.40 (t, 3 H) | 458 | Intermediate 150 |
| **163** | 7-Ethoxy-4-{[2-methoxy-5-(trifluoromethyl)phenyl] amino}-6-(4-methyl piperazin-1-yl)quinoline-3-carboxamide | 10.55 (s, 1H), 8.90 (s, 1 H), 8.35 (s, 1H), 7.65 (s, 1 H), 7.30 (m, 3 H), 6.70 (m, 2 H), 4.21 (q, 2 H), 3.95 (s, 3 H), 2.75 (s, 4 H), 2.30 (s, 4 H), 2.15 (s, 3 H), 1.40 (t, 3 H) | 504 | Intermediate 151 |
| **164** | 4-[(2,3-Dichlorophenyl) amino]-7-(2-methoxyethoxy)-6-(4-methylpiperazin-1-yl) quinoline-3-carboxamide | 11.33 (s, 1H), 9.47 (s, 1H), 8.34 (s, 1H), 7.78 (s, 1H), 7.67 (m, 2 H), 7.50 (s, 1H), 7.23 (s, 1 H), 7.10 (m, 1 H), 4.79 (m, 2 H), 4.32 (m, 2 H), 3.89 (s, 3 H), 3.30 (m, 4 H), 2.86 (m, 4 H), 2.66 (s, 3 H) | 506 | Intermediate 159 |
| **165** | 4-[(2,4-Difluorophenyl) amino]-7-(2-methoxyethoxy)-6-(4-methylpiperazin-1-yl) quinoline-3-carboxamide | 10.73 (s, 1H), 8.86 (s, 1 H), 8.28 (s, 1H), 7.66 (s, 1 H), 7.37 (m, 1 H), 7.26 (s, 1H), 7.03 (m, 2 H), 6.81 (s, 1H), 4.25 (m, 2 H), 3.73 (m, 2 H), 3.35 (s, 3 H), 2.75 (s, 4 H), 2.35 (s, 4 H), 2.17 (s, 3 H) | 472 | Intermediate 160 |
| **166** | 4-[(2-Fluoro-4-methylphenyl)amino]-7-(2-methoxyethoxy)-6-(4-methylpiperazin-1-yl)quinoline-3-carboxamide | 10.80 (s, 1H), 8.85 (s, 1H), 8.28 (s, 1 H), 7.64 (s, 1H), 7.23 (s, 1 H), 7.15 (d, 1H), 6.92 (m, 2 H), 6.82 (s, 1 H), 4.23 (m, 2 H), 3.74 (m, 2 H), 3.31 (s, 3 H), 2.70 (s, 4 H), 2.32 (s, 4 H), 2.29 (s, 3 H), 2.16 (s, 3H) | 468 | Intermediate 161 |
| **167** | 4-[(2-Chloro-3-fluorophenyl)amino]-7-(2-methoxyethoxy)-6-(4-methylpiperazin-1-yl)quinoline-3-carboxamide | 11.14 (s, 1 H), 9.43 (s, 1 H), 8.27 (s, 1 H), 7.84 (m, 1H), 7.76 (s, 1 H), 7.42 (s, 1H), 7.36 (s, 1 H), 7.34 (d, 1 H), 7.24 (d, 1H), 4.78 (m, 2 H), 4.29 (m, 2 H), 3.88 (s, 3 H), 3.34 (s, 4 H), 2.88 (s, 4 H), 2.67 (s, 3 H) | 488 | Intermediate 164 |
| **168** | 4-[(2-Fluor-5-methylphenyl)amino]-7-(2-methoxyethoxy)-6-(4-methylpiperazin-1-yl)quinoline-3-carboxamide | 11.36 (s, 1H), 9.41 (s, 1 H), 8.27 (s, 1 H), 7.72 (s, 1 H), 7.56 (m, 1 H), 7.39 (m, 3 H), 7.20 (m, 1 H), 4.75 (m, 2 H), 4.28 (m, 2 H), 3.88 (s, 3 H), 3.27 (s, 4 H), 2.84 (s, 4 H), 2.65 (s, 3 H), 2.44 (s, 3 H) | 468 | Intermediate 156 |
| **169** | 4-[(2,5-Difluorophenyl)amino]-7-(2-methoxyethoxy)-6-(4-methylpiperazin-1-yl)quinoline-3-carboxamide | 10.66 (s, 1H), 8.88 (s, 1 H), 8.32 (s, 1 H), 7.73 (s, 1H), 7.23 (s, 1 H), 7.32 (m, 2 H), 6.86 (m, 2 H), 6.62 (m, 1H), 4.27 (m, 2 H), 3.75 (m, 2 H), 3.35 (s, 3H), 2.81 (s, 4 H), 2.37 (s, 4 H), 2.17 (s, 3 H) | 472 | Intermediate 157 |
| **170** | 4-[(3-Chloro-2-fluorophenyl)amino]-7-(2-methoxyethoxy)-6-(4-methylpiperazin-1-yl)quinoline-3-carboxamide | 10.70 (s, 1H), 8.84 (s, 1H), 8.40 (s, 1H), 7.60 (s, 1H), 7.24 (s, 1 H), 7.13 (m, 1 H), 7.03 (m, 1H), 6.85 (s, 1 H), 6.75 (m, 1H), 4.23 (s, 2 H), 3.74 (s, 2 H), 3.33 (s, 3 H), 2.74 (s, 4 H), 2.34 (s, 4 H), 2.15 (s, 3H) | 488 | Intermediate 158 |
| **171** | 4-[(2-Fluoro-4-methylphenyl)amino]-7-(2-methoxyethoxy)-6-morpholin-4-ylquinoline-3-carboxamide | 10.82 (s, 1H), 8.86 (s, 1H), 8.29 (s, 1 H), 7.65 (s, 1H), 7.26 (s, 1 H), 7.14 (d, 1H), 6.92 (m, 3 H), 4.24 (m, 2 H), 3.73 (m, 2 H), 3.62 (m, 4 H), 3.36 (s, 3 H), 2.69 (s, 4 H), 2.27 (s, 3 H) | 455 | Intermediate 152 |
| **172** | 4-[(2-Fluoro-4-methylphenyl)amino]-6-(4-isopropylpiperazin-1-yl)-7-(2-methoxyethoxy) quinoline-3-carboxamide | 10.78 (s, 1H), 8.83 (s, 1 H), 8.27 (s, 1 H), 7.63 (s, 1H), 7.22 (s, 1 H), 7.14 (d, 1H), 6.89 (m, 2 H), 6.79 (s, 1H), 4.23 (m, 2 H), 3.72 (m, 2 H), 3.33 (s, 3 H), 2.68 (s, 4 H), 2.60 (m, 1H), 2.43 (s, 4 H), 2.27 (s, 3 H), 0.96 (d, 6 H) | 496 | Intermediate 153 |
| **173** | 4-[(2-Fluoro-5-methylphenyl)amino]-6-(4-isopropylpiperazin-1-yl)-7-(2-methoxyethoxy) quinoline-3-carboxamide | 10.75 (s, 1H), 8.95 (s, 1H), 8.27 (s, 1 H), 7.64 (s, 1H), 7.25 (s, 1 H), 7.15 (m, 1 H), 6.89 (m, 1H), 6.83 (s, 1 H), 6.71 (d, 1H), 4.25 (m, 2 H), 3.75 (m, 2 H), 3.34 (s, 3 H), 2.71 (s, 4 H), 2.60 (m, 1H), 2.45 (s, 4 H), 2.13 (s, 3 H), 0.96 (d, 6 H) | 496 | Intermediate 154 |

### Example 174

### 4-[(2,3-Dichlorophenyl)amino]-7-methoxy-6-piperazine-1-ylquinoline-3-carboxamide dihydrochloride

To a solution of trifluoracetic acid/dichloromethane (10 mL, 1:1) was added *tert*-butyl 4-{3-(aminocarbonyl)-4-[(2,3-dichlorophenyl)amino]-7-methoxyquinolin-6-yl}piperazine-1-carboxylate (Example 15, 250 mg, 0.46 mmol). After 2 hours, the solvent was removed under reduced pressure, the residue was taken up in MeOH (3 mL) and acidified with ethereal HCl. The resulting crystals were collected, washed with Et₂O and dried to give a solid (160 mg). ¹H NMR: 11.95 (s, 1 H), 9.28 (s, 2H), 9.00 (s, 1H), 8.45 (s, 1H), 7.86 (s, 1H), 7.59 (m, 2 H), 7.37 (m, 2 H), 7.21 (s, 1 H), 4.01 (s, 3H), 3.17 (m, 4 H), 3.07 (m, 4 H); *m*/*z:* 445

### Examples 175-185

The following compounds were prepared by a similar method to Example 174 using the appropriate starting materials. Some examples were isolated as the hydrochloride salts.

| **Ex.** | **Compound** | **NMR** | **M/z** | **SM** |
|---|---|---|---|---|
| **175** | 4-[(2,3-Dichlorophenyl)amino]-7-ethoxy-6-piperazin-1-ylquinoline-3-carboxamide | 10.78 (s, 1H), 8.92 (s, 1 H), 8.36 (s, 1 H), 7.76 (s, 1H), 7.31 (s, 1 H), 7.24 (d, 1H), 7.13 (m, 1 H), 6.66 (s, 1H), 6.57 (d, 1 H), 4.20 (q, 2 H), 2.86 (m, 4 H), 2.75 (m, 4 H), 1.41 (t, 3 H) | 460 | Example 125 |
| **176** | 4-[(2,4-Difluorophenyl)amino]-7-methoxy-6-piperazin-1-ylquinoline-3-carboxamide | 10.80 (s, 1 H), 8.91 (s, 1H), 8.35 (s, 1 H), 7.70 (s, 1 H), 7.40 (m, 1 H), 7.32 (s, 1 H), 7.06 (m, 2 H), 6.85 (s, 1 H), 4.15 (m, 1 H), 3.99 (s, 3 H), 3.45 (m, 2 H), 3.20 (m, 2 H), 2.80 (m, 2 H), 2.70 (m, 2 H) | 413 | Example 126 |
| **177** | 6-(1,4-Diazepan-1-yl)-4-[(2,4-difluorophenyl)amino]-7-methoxyquinoline-3-carboxamide | 10.60 (s, 1 H), 8.76 (s, 1H), 8.20 (s, 1 H), 7.56 (s, 1H), 7.29 (m, 1H), 7.15 (s, 1H), 6.90 (m, 2 H), 6.65 (m, 1 H), 3.86 (s, 3 H), 3.70 (m, 1 H), 3.00 (m, 4 H), 2.65 (m, 4 H), 1.55 (m, 2 H) | 427 | Example 127 |
| **178** | 4-[(3-Chloro-4-fluorophenyl)amino]-7-ethoxy-6-piperazin-1- 2 ylquinoline-3-carboxamide 4 | CD₂Cl₂ 10.41 (s, 1H), 8.77 (s, 1H), 7.28 (s, 1H), 7.07 (m, 1H), 6.87 (m, H), 6.72 (m, 1 H), 6.14 (br s, 2 H), 4.21 (q, 2 H), 2.89 (m, 4 H), 2.76 (m, H), 1.50 (t, 3 H) | 444 | Example 64 |
| **179** | 7-Ethoxy-4-[(2-fluoro-5-methylphenyl)amino]-6-piperazin-1-ylquinoline-3- H), carboxamide (q, | CD₂Cl₂ 10.45 (s, 1 H), 8.75 (s, 1H), 7.26 (s, 1 H), 7.01 (m, 1H), 6.95 (s, 1 6.85 (m, 1 H), 6.76 (m, 1 H), 4.20 2 H), 2.90 (m, 4 H), 2.73 (m, 4 H), 2.18 (s, 3H), 1.49 (t, 3H) | 424 | Example 65 |
| **180** | 4-[(3-Chloro-2-fluorophenyl)amino]-7-ethoxy-6-piperazin-1- 1 ylquinoline-3-carboxamide 4 | CD₂Cl₂ 10.51 (s, 1H), 8.75 (s, 1 H), 7.27 (s, 1 H), 7.06 (m, 2 H), 6.90 (m, H), 6.85 (m, 1H), 5.96 (br s, 2 H), 4.20 (q, 2 H), 2.92 (m, 4 H), 2.76 (m, H), 1.50 (t, 3 H) | 444 | Example 66 |
| **181** | 6-(1,4-Diazepan-1-yl)-4-[(2,3-dichlorophenyl)amino]-7-ethoxyquinoline-3-carboxamide 4 | 10.70 (s, 1H), 8.85 (s, 1 H), 8.35 (s, 1 H), 7.75 (s, 1H), 7.26 (m, 2 H), 7.15 (m, 1H), 6.55 (m, 2 H), 4.20 (q, 2 H), 4.10 (m, 1H), 3.05 (m, 4 H), 2.70 (m, H), 1.65 (m, 2 H), 1.44 (t, 3 H) | 473 | Example 128 |
| **182** | 4-[(2,4-Difluorophenyl)amino]-7-ethoxy-6-piperazin-1- (s, ylquinoline-3-carboxamide 4 | 10.71 (s, 1H), 8.84 (s, 1 H), 8.27 (s, 1 H), 7.64 (s, 1 H), 7.36 (m, 1 H), 7.22 1 H), 7.00 (m, 2 H), 6.77 (s, 1H), 4.16 (q, 2 H), 2.73 (m, 4 H), 2.63 (m, H), 1.39 (t, 3 H) | 427 | Example 129 |
| **183** | 6-(1,4-Diazepan-1-yl)-4-[(2,4-difluorophenyl)amino]-7- (s, ethoxyquinoline-3-carboxamide 4 | 10.65 (s, 1 H), 8.80 (s, 1H), 8.25 (s, 1 H), 7.63 (s, 1H), 7.35 (m, 1H), 7.20 1H), 6.95 (m, 2 H), 6.70 (s, 1 H), 4.16 (q, 2 H), 3.05 (m, 4 H), 2.75 (m, H), 1.61 (m, 2 H), 1.40 (t, 3 H) | 441 | Example 130 |
| **184** | 6-(1,4-Diazepan-1-yl)-4- [(2,3-dichlorophenyl)amino]-7-methoxyquinoline-3-carboxamide | 10.79 (s, 1 H), 8.95 (s, 1H), 8.42 (s, 1 H), 7.80 (s, 1 H), 7.32 (m, 2 H), 7.20 (m, 1 H), 6.65 (m, 2 H), 4.00 (s, 3 H), 3.85 (m, 1 H), 3.16 (m, 4 H), 2.78 (m, 4 H), 1.65 (m, 2H) | 460 | Example 131 |
| **185** | 7-Ethoxy-4-[(2-fluoro-4-methylphenyl)amino]-6-piperazin-1-ylqumoline-3-carboxamide | CD₂Cl₂ 10.37 (s, 1 H), 8.63 (s, 1H), 7.15 (s, 1H), 6.85 (m, 1H), 6.80 (m, 3 H), 4.10 (q, 2 H), 2.78 (m, 4 H), 2.60 (m, 4H), 2.22 (s, 3 H), 1.40 (t, 3 H) | 424 | Example 154 |

### Example 186

### 2-(4-{3-(Aminocarbonyl)-4-[(2,3-dichlorophenyl)amino]-7-methoxyquinolin-6-yl}piperazin-1-yl)-2-oxoethyl acetate

To a solution of 4-[(2,3-dichlorophenyl)amino]-7-methoxy-6-piperazin-1-ylquinoline-3-carboxamide dihydrochloride (Example 174, 120 mg, 0.23 mmol) and diisopropylethylamine (160 µL, 0.92 mmol) in THF (10 mL) at -10° C under N₂, was added dropwise over 10 minutes a solution of 2-chloro-2-oxoethyl acetate (30 µL, 0.28 mmol) in THF (10 mL). After stirring for 1 hour at -10° C, the solvent was removed under reduced pressure and the residue partitioned between EtOAc and saturated aqueous Na₂CO₃ solution. The organic layer was dried (Na₂SO₄), filtered and concentrated, and the residue was crystallized from Et₂O / EtOAc to give 100 mg solid. NMR: 8.98 (s, 1 H), 8.46 (s, 1H), 7.78 (s, 1 H), 7.37 (s, 1H), 7.25 (d, 1H), 7.14 (t, 1H), 6.72 (s, 1H), 6.58 (d, 1 H), 4.77 (s, 2 H), 3.98 (s, 3 H), 3.44 (m, 4 H), 2.78 (m, 4 H), 2.07 (s, 3 H); *m*/*z*: 545.

### Examples 187-190

The following compounds were prepared by a similar method to Example 186 using the appropriate starting materials.

| **Ex.** | **Compound** | **NMR** | **M/z** | **SM** |
|---|---|---|---|---|
| **187** | 6-(4-Acetylpiperazin-1-yl)-4-[(2,3-dichlorophenyl)amino]-7-methoxyquinoline-3-carboxamide | CD₃OD 8.77 (s, 1 H), 7.23 (s, 1H), 7.13 (dd, 1H), 7.00 (t, 1 H), 6.75 (s, 1 H), 6.57 (dd, 1H), 3.93 (s, 3 H), 3.50 (m, 4 H), 2.75 (m, 2 H), 2.67 (m, 2 H), 1.99 (s, 3 H) | 487 | Example 174 |
| **188** | 6-(4-Acetylpiperazin-1-yl)-4-[(2,3-dichlorophenyl)amino]-7-ethoxyquinoline-3-carboxamide | CD₂Cl₂ 10.42 (s, 1H), 8.82 (s, 1H), 7.34 (s, 1 H), 7.13 (d, 1H), 6.97 (m, 1 H), 6.79 (s, 1H), 6.60 (d, 1 H), 4.24 (q, 2 H), 3.62 (m, 2 H), 3.50 (m, 2 H), 2.79 (m, 4 H), 2.04 (s, 3 H), 1.51 (t, 3 H) | 502 | Example 175 |
| **189** | 6-(4-Acetyl-1,4-diazepan-1-yl)-4-[(2,3-dichlorophenyl)amino]-7-ethoxyquinoline-3-carboxamide | 10.75 (d, 1H), 8.95 (s, 1H), 8.42 (s, 1 H), 7.80 (s, 1H), 7.34 (m, 2 H), 7.20 (m, 1 H), 6.70 (s, 1H), 6.65 (d, 1H), 4.28 (q, 2 H), 3.55-3.12 (m, 11 H), 1.80 (m, 1 H), 1.70 (m, 1 H), 1.50 (t, 3 H) | 516 | Example 181 |
| **190** | 6-(4-Acetylpiperazin-1-yl)-7-ethoxy-4-[(2-fluoro-4-methylphenyl)amino] quinoline-3-carboxamide | CD₂Cl₂ 10.49 (s, 1 H), 8.72 (s, 1 H), 7.26 (s, 1H), 6.95 (d, 1 H), 6.91 (s, 1 H), 6.87 (m, 2 H), 5.97 (br, 2 H), 4.20 (q, 2 H), 3.58 (m, 2 H), 3.46 (m, 2H), 2.78 (m, 2 H), 2.66 (m, 2 H), 2.32 (s, 3 H), 2.04 (s, 3 H), 1.49 (t, 3 H) | 466 | Example 185 |

### Example 191

### 4-[(2,3-Dichlorophenyl)amino]-6-(4-glycoloylpiperazin-1-yl)-7-methoxyquinoline-3-carboxamide

A mixture of 2-(4-{3-(aminocarbonyl)-4-[(2,3-dichlorophenyl)amino]-7-methoxyquinolin-6-yl}piperazin-1-yl)-2-oxoethyl acetate (Example 186, 70 mg, 0.13 mmol), K₂CO₃ (0.5 g, 3.6 mmol), MeOH (5 mL), and water (1 mL) was stirred vigorously for 1 hour. Most of the solvent was removed under reduced pressure, and the residue partitioned between water (10 mL) and EtOAc (10 mL). The desired product precipitated in the separatory funnel, and was collected by filtration. The aqueous layer was further extracted with EtOAc, and the combined organic extracts were dried (Na₂SO₄), filtered, and concentrated under reduced pressure to give a solid identical by NMR and LC-MS to the earlier material. The solids were combined to give 25 mg. NMR: 10.85 (s, 1 H), 9.01 (s, 1H), 8.43 (s, 1 H), 7.83 (s, 1H), 7.43 (s, 1H), 7.33 (dd, 1H), 7.21 (t, 1 H), 6.78 (s, 1 H), 6.65 (d, 1 H), 4.63 (t, 1H), 4.14 (d, 2 H), 4.04 (s, 3 H), 3.57 (m, 2 H), 3.45 (m, 2 H), 2.82 (m, 4 H); *m*/*z*: 503.

### Example 192

### 4-[(2,3-Dichlorophenyl)amino]-7-ethoxy-6-[4-(2-hydroxyethyl)piperazin-1-yl]quinoline-3-carboxamide

A mixture of 4-[(2,3-dichlorophenyl)amino]-7-ethoxy-6-piperazin-1-ylquinoline-3-carboxamide (Example 175, 67 mg, 0.146 mmol) and glycolaldehyde (26 mg, 0.43 mmol) in toluene:MeOH (6 mL, 5:1) was heated to reflux for 1 hour. The reaction mixture was concentrated and dissolved in THF (15 mL). Sodium triacetoxyborohydride (155 mg, 0.73 mmol) and acetic acid (0.1 mL) were added and the reaction mixture was heated to reflux for 1 hour. Water was added, and the mixture extracted with EtOAc (4 x 50 mL). The combined organic extracts were dried (MgSO₄), filtered, concentrated, and the residue purified with reverse phase HPLC to give 36 mg of a yellow solid. NMR: 10.50 (s, 1H), 8.95 (s, 1 H), 8.40 (s, 1H), 7.85 (s, 1 H), 7.60 (d, 1H), 7.52 (s, 1H), 7.39 (m, 2 H), 7.20 (s, 1 H), 4.25 (q, 2 H), 3.80 (m, 2 H), 3.50 (m, 4 H), 3.20 (m, 4 H), 2.95 (m, 2 H), 1.43 (t, 3 H); *m*/*z*: 503.

### Examples 193-200

The following compounds were prepared by the procedure of Example 192 using the appropriate starting materials.

| **Ex.** | **Compound** | **NMR** | **M/z** | **SM** |
|---|---|---|---|---|
| **193** | 4-[(2,3-Dichlorophenyl)amino]-7-ethoxy-6-(4-isopropyl-1,4-diazepan-1-yl)quinoline-3-carboxamide | 10.69 (s, 1H), 8.85 (s, 1H), 8.33 (s, 1 H), 7.70 (s, 1H), 7.23 (m, 2 H), 7.14 (m, 1 H), 6.65 (m, 2 H), 4.20 (q, 2 H), 3.35 (m, 1 H), 3.10 (m, 4 H), 2.90-2.55 (m, 4 H), 1.65 (m, 2 H), 1.45 (t, 3 H), 0.92 (m, 6 H) | 515 | Example 181 |
| **194** | 4-[(2,4-Difluorophenyl) amino]-7-ethoxy-6-[4-(2-hydroxyethyl) piperazin-1-yl]quinoline-3-carboxamide | 11.00 (s, 1 H), 8.80 (s, 1 H), 8.20 (s, 1 H), 7.70 (s, 1 H), 7.40-7.10 (m, 5 H), 4.22 (q, 2 H), 3.78 (m, 2 H), 3.55-2.95 (m, 10 H), 1.45 (t, 3 H) | 471 | Example 182 |
| **195** | 6-[4-(Cyclopropylmethyl) piperazin-1-yl]-4-[(2,4-difluorophenyl)amino]-7-ethoxyquinoline-3-carboxamide | 10.10 (s, 1 H), 8.80 (s, 1 H), 8.20 (s, 1 H), 7.70 (s, 1H), 7.45 (m, 4 H), 7.15 (m, 1 H), 4.29 (q, 2 H), 3.50 (m, 4 H), 3.15 (m, 4 H), 2.90 (m, 2 H), 1.45 (t, 3 H), 1.09 (m, 1 H), 0.65 (m, 2 H), 0.35 (m, 2 H) | 481 | Example 182 |
| **196** | 4-[(2,4-Difluorophenyl) amino]-7-ethoxy-6-(4-isopropyl-1,4-diazepan-1-yl)quinoline-3-carboxamide | 10.70 (s, 1H), 8.87 (s, 1H), 8.32 (s, 1 H), 7.70 (s, 1H), 7.40 (m, 1H), 7.26 (s, 1 H), 7.05 (m, 2 H), 6.75 (s, 1H), 4.22 (q, 2 H), 3.10 (m, 4 H), 2.90 (m, 1H), 2.65 (m, 4 H), 1.75 (m, 2 H), 1.49 (t; 3 H), 1.00 (m, 6 H) | 484 | Example 183 |
| **197** | 4-[(2,4-Difluorophenyl) amino]-6-(4-isopropyl-1,4-diazepan-1-yl)-7-methoxyquinoline-3-carboxamide | 10.55 (s, 1 H), 8.70 (s, 1H), 8.20 (s, 1 H), 7.55 (s, 1 H), 7.25 (m, 1H), 7.10 (s, 1 H), 6.85 (m, 2 H), 6.62 (s, 1H), 3.80 (s, 3 H), 3.30 (m, 4 H), 2.95 (m, 4 H), 2.69 (m, 1 H), 1.47 (m, 2 H), 0.80 (d, 6 H) | 469 | Example 177 |
| **198** | 4-[(2,4-Difluorophenyl) amino]-6-(4-ethyl-1,4-diazepan-1-yl)-7-methoxyquinoline-3-carboxamide | 10.35 (s, 1H), 8.82 (s, 1H), 8.33 (s, 1 H), 7.77 (s, 1 H), 7.42 (m, 3 H), 7.20 (m, 2 H), 4.00 (s, 3 H), 3.50-3.10 (m, 6 H), 2.90 (m, 2 H), 2.28 (m, 2 H), 2.10 (m, 2 H), 1.28 (t, 3 H) | 455 | Example 177 |
| **199** | 4-[(2,3-Dichlorophenyl) amino]-6-(4-isopropyl-1,4-diazepan-1-yl)-7-methoxyquinoline-3-carboxamide | 10.77 (s, 1H), 8.92 (s, 1 H), 8.40 (s, 1 H), 7.79 (s, 1H), 7.30 (m, 2 H), 7.20 (m, 1 H), 6.70 (s, 1H), 6.60 (d, 1H), 4.00 (s, 3 H), 3.40 (m, 4 H), 3.18 (m, 4 H), 2.80 (m, 1H), 1.65 (m, 2 H), 0.95 (m, 6 H) | 502 | Example 184 |
| **200** | 7-Ethoxy-4-[(2-ffuoro-4-methylphenyl)amino]-6-[4-(2-hydroxyethyl) piperazin-1-yl]quinoline-3-carboxamide | CD₂Cl₂ 10.44 (s, 1H), 8.70 (s, 1H), 7.23 (s, 1 H), 6.94 (d, 1H), 6.91 (s, 1 H), 6.86 (m, 2 H), 5.97 (br, 2 H), 4.19 (q, 2 H), 3.56 (t, 2 H), 2.78 (s, 4 H), 2.53 (m, 6H), 2.31 (s, 3 H), 1.48 (t, 3 H) | 468 | Example 185 |

### Example 201

### 4-[(2,3-Dichlorophenyl)amino]-7-ethoxy-6-(4-glycoloylpiperazin-1-yl)quinoline-3-carboxamide

A mixture of 4-[(2,3-dichlorophenyl)amino]-7-ethoxy-6-piperazin-1-ylquinoline-3-carboxamide hydrochloride (Example 175, 80 mg, 0.14 mmol), glycolic acid (38 mg, 0.5 mmol), HATU (106 mg, 0.28 mmol) and DIEA (72 mg, 0.56 mmol) in anhydrous DMF (3 ml) was stirred at room temperature for 5 hours. The crude mixture was purified with reverse phase HPLC to give 30 mg of a yellow solid. NMR: 12.10 (s, 1 H), 8.98 (s, 1H), 8.46 (s, 1 H), 7.95 (s, 1 H), 7.65 (d, 1H), 7.42 (m, 2 H), 7.35 (d, 1H), 6.96 (s, 1 H), 4.25 (q, 2 H), 4.09 (s, 2 H), 3.60 (m, 4 H), 2.80 (m, 2 H), 2.70 (m, 2 H), 1.25 (t, 3H); *m*/*z*: 518.

### Examples 202-206

The following compounds were prepared by a similar method to Example 201 using the appropriate starting materials.

| **Ex.** | **Compound** | **NMR** | **M/z** | **SM** |
|---|---|---|---|---|
| **202** | 4-[(2,3-Dichlorophenyl) amino]-7-ethoxy-6-{4-[(2S)- 1 2-hydroxypropanoyl] piperazin-1-yl}quinoline-3-carboxamide 4 | 12.15 (s, 1H), 8.96 (s, 1H), 8.45 (s, H), 7.95 (s, 1H), 7.65 (d, 1 H), 7.40 (m, 3 H), 6.99 (s, 1H), 4.40 (m, 1 H), 4.26 (q, 2 H), 3.55 (m, 4 H), 2.80 (m, H), 1.48 (t, 3 H), 1.15 (d, 3 H) | 532 | Example 175 |
| **203** | 4-[(2,3-Dichlorophenyl) amino]-7-ethoxy-6-{4-[(2R)- 1 2-hydroxypropanoyl] (s, piperazin-1-yl}quinoline-3-carboxamide 4 | 12.29 (s, 1 H), 9.05 (s, 1 H), 8.57 (s, H), 7.96 (s, 1H), 7.65 (d, 1 H), 7.50 1 H), 7.40 (m, 2 H), 6.98 (s, 1 H), 4.42 (m, 1H), 4.25 (q, 2 H), 3.60 (m, H), 2.80 (m, 4 H), 1.45 (t, 3 H), 1.20 (d, 3 H) | 532 | Example 175 |
| **204** | 7-Ethoxy-4-[(2-fluoro-4-methylphenyl)amino]-6-(4-glycoloylpiperazin-1-yl)quinoline-3-carboxamide | CD₂Cl₂ 10.48 (s, 1H), 8.73 (s, 1H), 7.26 (s, 1H), 6.95 (d, 1H), 6.92 (s, 1 H), 6.86 (m, 2 H), 5.97 (br, 2 H), 4.20 (q, 2 H), 4.11 (s, 2 H), 3.66 (m, 2 H), 3.27 (m, 2 H), 2.78 (m, 2 H), 2.71 (m, 2 H), 2.32 (s, 3 H), 1.49 (% 3 H) | 481 | Example 185 |
| **205** | 7-Ethoxy-4-[(2-fluoro-4-methylphenyl)amino]-6-{4-[(2S)-2-hydroxypropanoyl] piperazin-1-yl}quinoline-3-carboxamide 2 | CD₂Cl₂ 10.47 (s, 1H), 8.72 (s, 1H), 7.26 (s, 1H), 6.95 (d, 1 H), 6.92 (s, 1 H), 6.87 (m, 2 H), 5.96 (br, 2H), 4.41 (q, 1H), 4.20 (q, 2 H), 3.66 (m, 2 H), 3.42 (m, 2 H), 2.79 (m, 2 H), 2.72 (m, H), 2.32 (s, 3 H), 1.49 (t, 3 H), 1.27 (d, 3 H) | 496 | Example 185 |
| **206** | 7-Ethoxy-4-[(2-fluoro-4-methylphenyl)amino]-6- {4-[(2R)-2-hydroxypropanoyl] piperazin-1-yl}quinoline-3-carboxamide 2 (d, | CD₂Cl₂ 10.47 (s, 1H), 8.72 (s, 1H), 7.26 (s, 1H), 6.95 (d, 1H), 6.92 (s, 1 H), 6.87 (m, 2 H), 5.96 (br, 2H), 4.41 (q, 1 H), 4.21 (q, 2 H), 3.66 (m, 2 H), 3.42 (m, 2 H), 2.79 (m, 2 H), 2.73 (m, H), 2.32 (s, 3 H), 1.49 (t, 3 H), 1.27 3 H) | 496 | Example 185 |

### Example 207

### 6-(4-Cyclopropyl-1,4-diazepan-1-yl)-4-[(2,4-difluorophenyl)amino]-7-ethoxyquinoline-3-carboxamide

To a solution of 6-(1,4-diazepan-1-yl)-4-[(2,4-difluorophenyl)amino]-7-ethoxyquinoline-3-carboxamide (Example 183, 100 mg, 0.227 mmol) in MeOH (10 mL) was added [(1-ethoxycyclopropyl)oxy]trimethyl silane (237 mg, 1.36 mmol), acetic acid (136 mg, 2.27 mmol), 4Å molecular sieves (2 g) and sodium cyanoborohydride (291 mg, 4.54 mmol). The resulting reaction mixture was stirred at reflux overnight. Water was added, the mixture was extracted with EtOAc (3 x 30 mL), and the combined organic extracts were dried (Na₂SO₄), concentrated and the residue purified with an ISCO chromatography system to give 100 mg of a light yellow solid. NMR: 10.66 (s, 1 H), 8.89 (s, 1 H), 8.25 (s, 1 H), 7.65 (s, 1 H), 7.35 (m, 1 H), 7.20 (s, 1H), 6.95 (m, 2H), 6.68 (s, 1H), 4.17 (q, 2 H), 3.03 (m, 4 H), 2.73 (m, 4 H), 1.86 (m, 1 H), 1.70 (m, 2 H), 1.42 (t, 3 H), 0.41 (m, 2 H), 0.27 (m, 2 H); *m*/*z:* 482.

### Examples 208-215

The following compounds were prepared by a similar method to Example 207 using the appropriate starting materials.

| **Ex.** | **Compound** | **NMR** | **M/z** | **SM** |
|---|---|---|---|---|
| **208** | 6-(4-Cyclopropyl piperazin-1-yl)-4-[(2,4-difluorophenyl)amino]-7-ethoxyquinoline-3-carboxamide | 10.79 (s, 1H), 8.90 (s, 1H), 8.31 (s, 1 H), 7.70 (s, 1H), 7.40 (m, 1H), 7.30 (s, 1 H), 7.09 (m, 2 H), 6.86 (s, 1 H), 4.25 (q, 2 H), 2.79 (m, 8 H), 2.65 (m, 1 H), 1.46 (t, 3 H), 0.71 (m, 2 H), 0.63 (m, 2 H) | 467 | Example 182 |
| **209** | 6-(4-Cyclopropyl piperazin-1-yl)-4-[(2,4-difluorophenyl)amino]-7-methoxyquinoline-3-carboxamide | 10.65 (s, 1H), 8.91 (s, 1H), 8.30 (s, 1 H), 7.68 (s, 1 H), 7.38 (m, 1H), 7.30 (s, 1 H), 7.06 (m, 2 H), 6.86 (s, 1 H), 3.95 (s, 3 H), 2.70 (m, 4 H), 2.56 (m, 4 H), 1.65 (m, 1H), 0.41 (m, 2 H), 0.30 (m, 2 H) | 453 | Example 176 |
| **210** | 6-(4-Cyclopropyl piperazin-1-yl)-4-[(2,3-dichlorophenyl)amino]-7-methoxyquinoline-3-carboxamide | 10.80 (s, 1H), 8.95 (s, 1H), 8.40 (s, 1 H), 7.79 (s, 1H), 7.35 (s, 1H), 7.28 (d, 1 H), 7.15 (m, 1H), 6.69 (s, 1H), 6.60 (d, 1H), 3.98 (s, 3 H), 2.71 (m, 4 H), 2.56 (m, 4 H), 1.65 (m, 1 H), 0.40 (m, 2 H), 0.20 (m, 2 H) | 486 | Example 174 |
| **211** | 6-(4-Cyclopropyl-1,4-diazepan-1-yl)-4-[(2,4-difluorophenyl)amino]-7-methoxyquinoline-3-carboxamide | 10.69 (s, 1H), 8.80 (s, 1 H), 8.28 (s, 1 H), 7.65 (s, 1 H), 7.36 (m, 1 H), 7.24 (s, 1 H), 6.97 (m, 2 H), 6.70 (s, 1H), 3.91 (s, 3H), 3.05 (m, 4 H), 2.70 (m, 4 H), 1.82 (m, 1H), 1.67 (m, 2 H), 0.40 (m, 2 H), 0.27 (m, 2 H) | 467 | Example 177 |
| **212** | 6-(4-Cyclopropyl-1,4-diazepan-1-yl)-4-[(2,3-dichlorophenyl)amino]-7-methoxyquinoline-3-carboxamide | 10.70 (s, 1H), 8.89 (s, 1H), 8.35 (s, 1 H), 7.63 (s, 1 H), 7.25 (m, 2 H), 7.12 (m, 1H), 6.55 (m, 2 H), 3.95 (s, 3 H), 3.12 (m, 2 H), 3.07 (m, 2 H), 2.65 (m, 4 H), 1.85 (m, 1 H), 1.65 (m, 2 H), 0.42 (m, 2 H), 0.26 (m, 2 H) | 500 | Example 184 |
| **213** | 4-[(3-Chloro-4-fluorophenyl)amino]-6-(4-cyclopropyl piperazin-1-yl)-7-ethoxyquinoline-3-carboxamide | CD₃OD 8.81 (s, 1H), 7.26 (s, 1H), 7.20 (m, 1H), 7.03 (m, 1H), 7.00 (s, 1 H), 6.90 (m, 1 H), 4.25 (q, 2 H), 2.88 (m, 4 H), 2.79 (m, 4 H), 1.84 (m, 1 H), 1.52 (t, 3 H), 0.55 (m, 2 H), 0.48 (m, 2 H) | 484 | Example 178 |
| **214** | 6-(4-Cyclopropyl piperazin-1-yl)-7-ethoxy-4-[(2-fluoro-5-methylphenyl)amino] quinoline-3-carboxamide | CD₃OD 8.78 (s, 1H), 7.23 (s, 1H), 7.06 (dd, 1H), 7.01 (s, 1 H), 6.94 (m, 1 H), 6.79 (d, 1 H), 4.23 (q, 2 H), 2.80 (m 4 H), 2.69 (m, 4 H), 2.20 (s, 3H), 1.69 (m, 1H), 1.51 (t, 3H), 0.50 (m, 2 H), 0.42 (m, 2 H) | 464 | Example 179 |
| **215** | 4-[(3-Chloro-2-fluorophenyl)amino]-6-(4-cyclopropyl piperazin-1-yl)-7-ethoxyquinoline-3-carboxamide | CD₃OD 8.76 (s, 1H), 7.25 (s, 1H), 7.17 (dd, 1H), 7.06 (d, 1H), 7.02 (s, 1 H), 6.95 (m, 1H), 4.24 (q, 2 H), 2.88 (m, 4 H), 2.72 (m, 4 H), 1.70 (m, 1 H), 1.52 (t, 3 H), 0.50 (m, 2 H), 0.43 (m, 2 H) | 484 | Example 180 |

### Example 216

### 4-[(2,4-Difluorophenyl)amino]-7-(2-hydroxyethoxy)-6-(4-methylpiperazin-1-yl)quinoline-3-carboxamide

To a solution of ethyl 7-(2-{[*tert*-butyl(dimethyl)silyl]oxy}ethoxy)-4-[(2,4-difluorophenyl)amino]-6-(4-methylpiperazin-1-yl)quinoline-3-carboxylate (Intermediate 162, 0.200 g, 0.33 mmol) and formamide (0.132 mL, 3.33 mmol) in DMF (5 mL), heated at 100°C for 30 minutes, was added a solution of sodium methoxide in MeOH (0.5 M, 0.85 mL, 0.43 mmol). After 16 hours, the reaction was cooled, water (50 mL) was added, and the reaction mixture extracted with EtOAc (3 x 50 mL). The combined organic extracts were concentrated and tetrabutylammonium fluoride solution (1.0 M in THF, 1 mL) added. The mixture was allowed to stand for 1 hour. Water (10 mL) was added and the mixture was extracted with EtOAc (3 x 10 mL). The combined organic extracts were concentrated and the residue purified with reverse phase HPLC. The product was recrystallized (hexanes/acetone) to give 85 mg (56%) of a yellow solid. ¹H NMR: 10.73 (s, 1H), 8.85 (s, 1 H), 8.29 (s, 1H), 7.66 (s, 1 H), 7.37 (t, 1 H), 7.26 (s, 1 H), 7.03 (m, 2 H), 6.80 (s, 1H), 4.87 (t, 1H), 4.16 (m, 2 H), 3.79 (m, 2 H), 2.76 (s, 4 H), 2.37 (s, 4 H), 2.18 (s, 3 H); *m*/*z:* 458.

### Example 217

The following compound was prepared by a similar method to Example 216 using the appropriate starting material.

| **Ex.** | **Compound** | **NMR** | **M/z** | **SM** |
|---|---|---|---|---|
| **217** | 4-[(3-Chloro-2-fluorophenyl)amino]-7-(2-hydroxyethoxy)-6-(4-methylpiperazin-1-yl)quinoline-3-carboxamide | 10.69 (s, 1 H), 8.88 (s, 1H), 8.31 (s, 1 H), 7.70 (s, 1 H), 7.30 (s, 1 H), 7.23 (t, 1 H), 7.08 (s, 1H), 6.84 (m, 2 H), 4.87 (t, 1H), 4.18 (m, 2 H), 3.80 (m, 2 H), 2.81 (s, 4 H), 2.37 (s, 4 H), 2.18 (s, 3 H) | 474 | Intermediate 163 |

### Preparation of Starting Materials

### Intermediate 1

### Ethyl 4-[(2,3-dichlorophenyl)amino]-7-methoxy-6-(4-methylpiperazin-1-yl)quinoline-3-carboxylate

A mixture of ethyl 6-bromo-4-[(2,3-dichlorophenyl)amino]-7-methoxyquinoline-3-carboxylate (Intermediate 155; 400 mg, 0.82 mmol), tris(dibenzylideneacetone) dipalladium(0) (60 mg, 0.066 mmol), (R,S)-2,2'-bis(diphenylphosphino)-1,1'-binaphthyl (124 mg, 0.20 mmol), Cs₂CO₃ (390 mg, 1.2 mmol), and N-methylpiperazine (227 µL, 2.05 mmol) in anhydrous toluene under N₂ was heated at 100°C for 18 hours. The reaction mixture was filtered, concentrated, and the crude oil purified by reverse phase HPLC to give 117 mg of a solid; *m*/*z:* 489.

### Intermediates 2-154

The following compounds were prepared by a method similar to Intermediate 1 using the appropriate starting materials.

| **Int** | **Compound** | **M/z/NMR** | **SM** |
|---|---|---|---|
| **2** | Ethyl4-[(2,4-dichlorophenyl)amino]-7-methoxy-6-(4-methylpiperazin-1-yl)quinoline-3-carboxylate | | Intermediate 165 |
| **3** | Ethyl 4-[(3,4-dichlorophenyl)amino]-7-methoxy-6-(4-methylpiperazin-1-yl)quinoline-3-carboxylate | | Intermediate 166 |
| **4** | Ethyl 4-[(2,4-difluorophenyl)amino]-7-methoxy-6-(4-methylpiperazin-1-yl)quinoline-3-carboxylate | 458 | Intermediate 167 |
| **5** | Ethyl 4-[(2,3-dichlorophenyl)amino]-7-methoxy-6-morpholin-4-ylquinoline-3-carboxylate | | Intermediate 155 |
| **6** | Ethyl 4-[(2,4-difluorophenyl)amino]-7-methoxy-6-morpholin-4-ylquinoline-3-carboxylate | | Intermediate 167 |
| **7** | Ethyl 4-[(3,4-dichlorophenyl)amino]-7-methoxy-6-morpholin-4-ylquinoline-3-carboxylate | | Intermediate 166 |
| **8** | Ethyl 4-[(3,4-dichlorophenyl)amino]-7-methoxy-6-piperidin-1-ylquinoline-3-carboxylate | | Intermediate 166 |
| **9** | Ethyl 4-[(2,3-dichlorophenyl)amino]-6-methoxy-7-(4-methylpiperazin-1-yl)quinoline-3-carboxylate | | Intermediate 168 |
| **10** | Ethyl 4-[(3,4-dichlorophenyl)amino]-6-methoxy-7-(4-methylpiperazin-1-yl)quinoline-3-carboxylate | | Intermediate 169 |
| **11** | Ethyl 4-[(2,4-difluorophenyl)amino]-7-ethoxy-6-(4-methylpiperazin-1-yl)quinoline-3-carboxylate | 471 | Intermediate 170 |
| **12** | Ethyl 4-[(2,3-dichlorophenyl)amino]-7-ethoxy-6-(4-methylpiperazin-1-yl)quinoline-3-carboxylate | 504 | Intermediate 171 |
| **13** | Ethyl 4-[(2,4-difluorophenyl)amino]-7-ethoxy-6-morpholin-4-ylquinoline-3-carboxylate | | Intermediate 170 |
| **14** | Ethyl 4-[(3,4-dichlorophenyl)amino]-7-ethoxy-6-morpholin-4-ylquinoline-3-carboxylate | | Intermediate 172 |
| **15** | Ethyl 6-[4-(tert-butoxycarbonyl)piperazin-1-yl]-4-[(2,3-dichlorophenyl)amino]-7-methoxyquinoline-3-carboxylate | 575 | Intermediate 155 |
| **16** | Ethyl 4-[(2,4-difluorophenyl)amino]-7-fluoro-6-(4-methylpiperazin-1-yl)quinoline-3-carboxylate | 445 | Intermediate 173 |
| **17** | Ethyl 4-[(2,3-dichlorophenyl)amino]-7-fluoro-6-(4-methylpiperazin-1-yl)quinoline-3-carboxylate | | Intermediate 174 |
| **18** | Ethyl 4-[(2,4-difluorophenyl)amino]-7-fluoro-6-morpholin-4-ylquinoline-3-carboxylate | | Intermediate 173 |
| **19** | Ethyl 4-[(2,3-dichlorophenyl)amino]-5-fluoro-6-(4-methylpiperazin-1-yl)quinoline-3-carboxylate | | Intermediate 175 |
| **20** | Ethyl 7-ethoxy-4-[(4-ethylphenyl)amino]-6-(4-methylpiperazin-1-yl)quinoline-3-carboxylate | 463 | Intermediate 176 |
| **21** | Ethyl 4-[(3-chloro-4-fluorophenyl)amino]-7-ethoxy-6-(4-methylpiperazin-1-yl)quinoline-3-carboxylate | 487 | Intermediate 177 |
| **22** | Ethyl4-[(3,4-dichlorophenyl)amino]-7-ethoxy-6-(4-methylpiperazin-1-yl)quinoline-3-carboxylate | 503 | Intermediate 172 |
| **23** | Ethyl 4-[(2,3-dichlorophenyl)amino]-7-ethoxy-6-(4-ethylpiperazin-1-yl)quinoline-3-carboxylate | 517 | Intermediate 171 |
| **24** | Ethyl 4-[(3-chloro-2-fluorophenyl)amino]-7-ethoxy-6-morpholin-4-ylquinoline-3-carboxylate | 474 | Intermediate 178 |
| **25** | Ethyl 7-ethoxy-4-[(2-fluoro-5-methylphenyl)amino]-6-morpholin-4-ylquinoline-3-carboxylate | 454 | Intermediate 179 |
| **26** | Ethyl 4-[(3-chloro-4-fluorophenyl)amino]-7-ethoxy-6-morpholin-4-ylquinoline-3-carboxylate | 454 | Intermediate 177 |
| **27** | Ethyl 7-ethoxy-4-[(4-ethylphenyl)amino]-6-morpholin-4-ylquinoline-3-carboxylate | 450 | Intermediate 176 |
| **28** | Ethyl 4-[(2,3-dichlorophenyl)amino]-7-ethoxy-6-morpholin-4-ylquinoline-3-carboxylate | 490 | Intermediate 171 |
| **29** | Ethyl 4-[(2,3-dichlorophenyl)amino]-7-ethoxy-6-(4-isopropylpiperazin-1-yl)quinoline-3-carboxylate | 530 | Intermediate 171 |
| **30** | Ethyl 4-[(2,3-dichlorophenyl)amino]-7-ethoxy-6-(4-methyl-1,4-diazepan-1-yl)quinoline-3-carboxylate | 516 | Intermediate 171 |
| **31** | Ethyl 4-[(2,3-dichlorophenyl)amino]-7-ethoxy-6-(3-hydroxypyrrolidin-1-yl)quinoline-3-carboxylate | 489 | Intermediate 171 |
| **32** | Ethyl 4-[(2,3-dichlorophenyl)amino]-6-{4-[2-(dimethylamino)ethyl]piperazin-1-yl}-7-ethoxyquinoline-3-carboxylate | 559 | Intermediate 171 |
| **33** | Ethyl 4-[(2,3-dichlorophenyl)amino]-7-ethoxy-6-[4-(2-methoxyethyl)piperazin-1-yl]quinoline-3-carboxylate | 546 | Intermediate 171 |
| **34** | Ethyl 4-[(3,4-dichlorophenyl)amino]-7-ethoxy-6-(4-ethylpiperazin-1-yl)quinoline-3-carboxylate | 517 | Intermediate 172 |
| **35** | Ethyl 4-[(2,4-difluorophenyl)amino]-7-ethoxy-6-(4-ethylpiperazin-1-yl)quinoline-3-carboxylate | 485 | Intermediate 170 |
| **36** | Ethyl 4-[(3-chloro-2-fluorophenyl)amino]-7-ethoxy-6-(4-ethylpiperazin-1-yl)quinoline-3-carboxylate | 501 | Intermediate 178 |
| **37** | Ethyl 7-ethoxy-6-(4-ethylpiperazin-1-yl)-4-[(2-fluoro-5-methylphenyl)amino]quinoline-3-carboxylate | 481 | Intermediate 179 |
| **38** | Ethyl 4-[(3-chloro-4-fluorophenyl)amino]-7-ethoxy-6-(4-ethylpiperazin-1-yl)quinoline-3-carboxylate | 501 | Intermediate 177 |
| **39** | Ethyl 7-ethoxy-4-[(4-ethylphenyl)amino]-6-(4-ethylpiperazin-1-yl)quinoline-3-carboxylate | 477 | Intermediate 176 |
| **40** | Ethyl 6-[4-(2-cyanoethyl)piperazin-1-yl]-4-[(2,3-dichlorophenyl)amino]-7-ethoxyquinoline-3-carboxylate | 541 | Intermediate 171 |
| **41** | Ethyl 4-[(2,3-dichlorophenyl)amino]-7-ethoxy-6-(4-hydroxypiperidin-1-yl)quinoline-3-carboxylate | 503 | Intermediate 171 |
| **42** | Ethyl 7-ethoxy-4-[(4-ethylphenyl)amino]-6-(4-methyl-1,4-diazepan-1-yl)quinoline-3-carboxylate | 477 | Intermediate 176 |
| **43** | Ethyl 4-[(2,4-difluorophenyl)amino]-7-ethoxy-6-(3-hydroxypyrrolidin-1-yl)quinoline-3-carboxylate | 457 | Intermediate 170 |
| **44** | Ethyl4-[(2,4-difluorophenyl)amino]-7-ethoxy-6-(4-hydroxypiperidin-1-yl)quinoline-3-carboxylate | 471 | Intermediate 170 |
| **45** | Ethyl 4-[(3,4-dichlorophenyl)amino]-7-ethoxy-6-(4-isopropylpiperazin-1-yl)quinoline-3-carboxylate | 531 | Intermediate 172 |
| **46** | Ethyl4-[(2,4-difluorophenyl)amino]-7-ethoxy-6-(4-isopropylpiperazin-1-yl)quinoline-3-carboxylate | 499 | Intermediate 170 |
| **47** | Ethyl 4-[(3-chloro-2-fluorophenyl)amino]-7-ethoxy-6-(4-isopropylpiperazin-1-yl)quinoline-3-carboxylate | 515 | Intermediate 178 |
| **48** | Ethyl7-ethoxy-4-[(2-fluoro-5-methylphenyl)amino]-6-(4-isopropylpiperazin-1-yl)quinoline-3-carboxylate | 515 | Intermediate 179 |
| **49** | Ethyl 4-[(3-chloro-4-fluorophenyl)amino]-7-ethoxy-6-(4-isopropylpiperazin-1-yl)quinoline-3-carboxylate | 491 | Intermediate 177 |
| **50** | Ethyl 4-[(2,4-difluorophenyl)amino]-7-ethoxy-6-(4-methyl-1,4-diazepan-1-yl)quinoline-3-carboxylate | 484 | Intermediate 170 |
| **51** | Ethyl4-[(2,3-dichlorophenyl)amino]-6-(4-isopropylpiperazin-1-yl)-7-methoxyquinoline-3-carboxylate | 517 | Intermediate 155 |
| **52** | Ethyl 4-[(2,4-difluorophenyl)amino]-6-(4-isopropylpiperazin-1-yl)-7-methoxyquinoline-3-carboxylate | 484 | Intermediate 167 |
| **53** | Ethyl 4-[(2,3-dichlorophenyl)amino]-7-methoxy-6-(4-methyl-1,4-diazepan-1-yl)quinoline-3-carboxylate | 503 | Intermediate 155 |
| **54** | Ethyl 4-[(2,4-difluorophenyl)amino]-7-methoxy-6-(4-methyl-1,4-diazepan-1-yl)quinoline-3-carboxylate | 470 | Intermediate 167 |
| **55** | Ethyl 4-[(2,3-dichlorophenyl)amino]-6-(4-ethylpiperazin-1-yl)-7-methoxyquinoline-3-carboxylate | 503 | Intermediate 155 |
| **56** | Ethyl 4-[(2,4-difluorophenyl)amino]-6-(4-ethylpiperazin-1-yl)-7-methoxyquinoline-3-carboxylate | 470 | Intermediate 167 |
| **57** | Ethyl 4-[(3,4-dichlorophenyl)amino]-6-(4-ethylpiperazin-1-yl)-7-methoxyquinoline-3-carboxylate | 503 | Intermediate 166 |
| **58** | Ethyl 4-[(3,4-dichlorophenyl)amino]-6-(4-isopropylpiperazin-1-yl)-7-methoxyquinoline-3-carboxylate | 517 | Intermediate 166 |
| **59** | Ethyl4-[(3,4-dichlorophenyl)amino]-7-methoxy-6-(4-methyl-1,4-diazepan-1-yl)quinoline-3-carboxylate | 503 | Intermediate 166 |
| **60** | Ethyl 4-[(3,4-dichlorophenyl)amino]-7-ethoxy-6-(4-methyl-1,4-diazepan-1-yl)quinoline-3-carboxylate | 517 | Intermediate 172 |
| **61** | Ethyl 7-ethoxy-4-[(2-fluoro-5-methylphenyl)amino]-6-(4-methyl-1,4-diazepan-1-yl)quinoline-3-carboxylate | 481 | Intermediate 179 |
| **62** | Ethyl 4-[(3-chloro-4-fluorophenyl)amino]-7-ethoxy-6-(4-methyl-1,4-diazepan-1-yl)quinoline-3-carboxylate | 487 | Intermediate 177 |
| **63** | Ethyl4-[(2-fluorophenyl)amino]-7-methoxy-6-(4-methylpiperazin-1-yl)quinoline-3-carboxylate | | Intermediate 180 |
| **64** | Ethyl6-[4-(tert-butoxycarbonyl)piperazin-1-yl]-4-[(3-chloro-4-fluorophenyl)amino]-7-ethoxyquinoline-3-carboxylate | 573 | Intermediate 177 |
| **65** | Ethyl 6-[4-(tert-butoxycarbonyl)piperazin-1-yl]-7-ethoxy-4-[(2-fluoro-5-methylphenyl)amino]quinoline-3-carboxylate | 553 | Intermediate 179 |
| **66** | Ethyl6-[4-(tert-butoxycarbonyl)piperazin-1-yl]-4-[(3-chloro-2-fluorophenyl)amino]-7-ethoxyquinoline-3-carboxylate | 573 | Intermediate 178 |
| **67** | Ethyl 7-methoxy-4-[(3-methoxy-2-methylphenyl)amino]-6-(4-methylpiperazin-1-yl)quinoline-3-carboxylate | 465 | Intermediate 181 |
| **68** | Ethyl 4-[(4-chloro-2-methylphenyl)amino]-7-methoxy-6-(4-methylpiperazin-1-yl)quinoline-3-carboxylate | 469 | Intermediate 182 |
| **69** | Ethyl 4-[(3-chloro-2-methylphenyl)amino]-7-ethoxy-6-(4-methylpiperazin-1-yl)quinoline-3-carboxylate | 463 | Intermediate 183 |
| **70** | Ethyl 4-[(3-chloro-2-fluorophenyl)amino]-7-ethoxy-6-(4-methylpiperazin-1-yl)quinoline-3-carboxylate | 486 | Intermediate 178 |
| **71** | Ethyl 7-ethoxy-4-[(3-ethylphenyl)amino]-6-(4-methylpiperazin-1-yl)quinoline-3-carboxylate | 462 | Intermediate 184 |
| **72** | Ethyl4-[(3-chlorophenyl)amino]-7-ethoxy-6-(4-methylpiperazin-1-yl)quinoline-3-carboxylate | 469 | Intermediate 185 |
| **73** | Ethyl 4-[(2-chloro-4-fluorophenyl)amino]-7-ethoxy-6-(4-methylpiperazin-1-yl)quinoline-3-carboxylate | 486 | Intermediate 186 |
| **74** | Ethyl 4-[(4-chloro-2-fluorophenyl)amino]-7-ethoxy-6-(4-methylpiperazin-1-yl)quinoline-3-carboxylate | 486 | Intermediate 187 |
| **75** | Ethyl 7-ethoxy-4-[(3-methylphenyl)amino]-6-(4-methylpiperazin-1-yl)quinoline-3-carboxylate | 448 | Intermediate 188 |
| **76** | Ethyl 4-[(2-chloro-3-methylphenyl)amino]-7-ethoxy-6-(4-methylpipemzin-1-yl)quinoline-3-carboxylate | 482 | Intermediate 189 |
| **77** | Ethyl 7-ethoxy-4-[(3-fluoro-2-methylphenyl)amino]-6-(4-methylpiperazin-1-yl)quinoline-3-carboxylate | 466 | Intermediate 190 |
| **78** | Ethyl 4-[(3,4-difluorophenyl)amino]-7-methoxy-6-(4-methylpiperazin-1-yl)quinoline-3-carboxylate | 457 | Intermediate 191 |
| **79** | Ethyl 7-methoxy-6-(4-methylpiperazin-1-yl)-4-{[2-methyl-3-(trifluoromethyl)phenyl]amino}quinoline-3-carboxylate | | Intermediate 192 |
| **80** | Ethyl 4-[(4-chlorophenyl)amino]-7-methoxy-6-(4-methylpiperazin-1-yl)quinoline-3-carboxylate | 455 | Intermediate 193 |
| **81** | Ethyl4-[(2-fluoro-4-methylphenyl)amino]-7-methoxy-6-(4-methylpiperazin-1-yl)quinoline-3-carboxylate | 453 | Intermediate 194 |
| **82** | Ethyl 7-methoxy-6-(4-methylpiperazin-1-yl)-4-{[3-(trifluoromethyl)phenyl]amino}quinoline-3-carboxylate | 489 | Intermediate 195 |
| **83** | Ethyl 7-ethoxy-4-[(2-fluoro-5-methylphenyl)amino]-6-(4-methylpiperazin-1-yl)quinoline-3-carboxylate | 467 | Intermediate 179 |
| **84** | Ethyl 4-[(3-chloro-2-methylphenyl)amino]-7-ethoxy-6-(4-ethylpiperazin-1-yl)quinoline-3-carboxylate | 496 | Intermediate 183 |
| **85** | Ethyl 4-[(3-chloro-2-methylphenyl)amino]-7-ethoxy-6-(4-methyl-1,4-diazepan-1-yl)quinoline-3-carboxylate | 496 | Intermediate 183 |
| **86** | Ethyl 7-ethoxy-6-(4-ethylpiperazin-1-yl)-4-[(3-fluoro-2-methylphenyl)amino]quinoline-3-carboxylate | 480 | Intermediate 190 |
| **87** | Ethyl 7-ethoxy-4-[(3-fluoro-2-methylphenyl)amino]-6-(4-methyl-1,4-diazepan-1-yl)quinoline-3-carboxylate | 480 | Intermediate 190 |
| **88** | Ethyl 7-ethoxy-4-[(2-fluoro-4-methylphenyl)amino]-6-(4-methylpiperazin-1-yl)quinoline-3-carboxylate | 467 | Intermediate 196 |
| **89** | Ethyl 7-ethoxy-4-[(3-methoxy-2-methylphenyl)amino]-6-(4-methylpiperazin-1-yl)quinoline-3-carboxylate | 479 | Intermediate 197 |
| **90** | Ethyl 4-[(2,5-difluorophenyl)amino]-7-ethoxy-6-(4-methylpiperazin-1-yl)quinoline-3-carboxylate | 471 | Intermediate 198 |
| **91** | Ethyl 4-[(2,5-difluorophenyl)amino]-7-ethoxy-6-(4-ethylpiperazin-1-yl)quinoline-3-carboxylate | 485 | Intermediate 198 |
| **92** | Ethyl 4-[(2,5-difluorophenyl)amino]-7-ethoxy-6-(4-methyl-1,4-diazepan-1-yl)quinoline-3-carboxylate | 485 | Intermediate 198 |
| **93** | Ethyl 7-ethoxy-6-(4-ethylpiperazin-1-yl)-4-[(2fluoro-4-methylphenyl)amino]quinoline-3-carboxylate | 481 | Intermediate 196 |
| **94** | Ethyl4-[(3,4-dimethylphenyl)amino]-7-ethoxy-6-(4-methylpiperazin-1-yl)quinoline-3-carboxylate | 463 | Intermediate 199 |
| **95** | Ethyl 4-[(2,4-difluorophenyl)amino]-6-ethoxy-7-(4-methylpiperazin-1-yl)quinoline-3- carboxylate | 471 | Intermediate 200 |
| **96** | Ethyl 4-[(2,3-dichlorophenyl)amino]-6-ethoxy-7-(4-methylpiperazin-1-yl)quinoline-3-carboxylate | 503 | Intermediate 201 |
| **97** | Ethyl 4-[(2,3-difluorophenyl)amino]-7-ethoxy-6-(4-methylpiperazin-1-yl)quinoline-3-carboxylate | 471 | Intermediate 202 |
| **98** | Ethyl4-[(2,3-dimethylphenyl)amino]-7-ethoxy-6-(4-methylpiperazin-1-yl)quinoline-3-carboxylate | 463 | Intermediate 203 |
| **99** | Ethyl 4-[(4-chloro-3-fluorophenyl)amino]-7-ethoxy-6-(4-methylpiperazin-1-yl)quinoline-3-carboxylate | 488 | Intermediate 204 |
| **100** | Ethyl 4-[(2,3-dichlorophenyl)amino]-6-ethoxy-7-(4-ethylpiperazin-1-yl)quinoline-3-carboxylate | 517 | Intermediate 201 |
| **101** | Ethyl 4-[(2,4-difluorophenyl)amino]-6-ethoxy-7-(4-ethylpiperazin-1-yl)quinoline-3-carboxylate | 485 | Intermediate 200 |
| **102** | Ethyl 4-[(3-chloro-2,4-difluorophenyl)amino]-7-ethoxy-6-(4-ethylpiperazin-1-yl)quinoline-3-carboxylate | 518 | Intermediate 205 |
| **103** | Ethyl 4-[(3-chloro-2,4-difluorophenyl)amino]-7-ethoxy-6-(4-methylpiperazin-1-yl)quinoline-3-carboxylate | 504 | Intermediate 205 |
| **104** | Ethyl4-[(3-chloro-4-fluorophenyl)amino]-6-ethoxy-7-(4-methylpiperazin-1-yl)quinoline-3-carboxylate | 487 | Intermediate 206 |
| **105** | Ethyl 4-[(3-chloro-4-fluorophenyl)amino]-6-ethoxy-7-(4-ethylpiperazin-1-yl)quinoline-3-carboxylate | 501 | Intermediate 206 |
| **106** | Ethyl 4-{[4-fluoro-2-(trifluoromethyl)phenyl]amino}-7-methoxy-6-(4-methylpiperazin-1-yl)quinoline-3-carboxylate | | Intermediate 207 |
| **107** | Ethyl 4-[(2-chloro-4-fluorophenyl)amino]-7- methoxy-6-(4-methylpiperazin-1-yl)quinoline-3-carboxylate | 473 | Intermediate 208 |
| **108** | Ethyl 7-methoxy-6-(4-methylpiperazin-1-yl)-4-{[3-(trifluoromethoxy)phenyl]amino}quinoline-3-carboxylate | 505 | Intermediate 209 |
| **109** | Ethyl 4-[(2,6-dimethylphenyl)amino]-7-methoxy-6-(4-methylpiperazin-1-yl)quinoline-3-carboxylate | 449 | Intermediate 210 |
| **110** | Ethyl 4-[(2,4-difluorophenyl)amino]-7-ethoxy-6-(4-ethyl-1,4-diazepan-1-yl)quinoline-3-carboxylate | 498 | Intermediate 170 and Intermediate 252 |
| **111** | Ethyl 4-[(2,3-dichlorophenyl)amino]-6-(4-ethyl-1,4-diazepan-1-yl)-7-methoxyquinoline-3-carboxylate | 516 | Intermediate 155 and Intermediate 252 |
| **112** | Ethyl 4-[(2,3-dichlorophenyl)amino]-7-ethoxy-6-(4-ethyl-1,4-diazepan-1-yl)quinoline-3-carboxylate | 530 | Intermediate 171 and Intermediate 252 |
| **113** | Ethyl 6-[4-(tert-butoxycarbonyl)piperazin-1-yl]-4-[(2,3-dichlorophenyl)amino]-7-ethoxyquinoline-3-carboxylate | 589 | Intermediate 171 |
| **114** | Ethyl6-[4-(tert-butoxycarbonyl)piperazin-1-yl]-4-[(2,4-difluorophenyl)amino]-7-methoxyquinoline-3-carboxylate | 542 | Intermediate 167 |
| **115** | Ethyl 6-[4-(tert-butoxycarbonyl)-1,4-diazepan-1-yl]-4-[(2,4-difluorophenyl)amino]-7-methoxyquinoline-3-carboxylate | 556 | Intermediate 167 |
| **116** | Ethyl 6-[4-(tert-butoxycarbonyl)-1,4-diazepan-1-yl]-4-[(2,3-dichlorophenyl)amino]-7-ethoxyquinoline-3-carboxylate | 602 | Intermediate 171 |
| **117** | Ethyl 6-[4-(tert-butoxycarbonyl)piperazin-1-yl]-4-[(2,4-difluorophenyl)amino]-7-ethoxyquinoline-3-carboxylate | 556 | Intermediate 170 |
| **118** | Ethyl 6-[4-(tert-butoxycarbonyl)-1,4-diazepan-1-yl]-4-[(2,4-difluorophenyl)amino]-7-ethoxyquinoline-3-carboxylate | 570 | Intermediate 170 |
| **119** | Ethyl 6-[4-(tert-butoxycarbonyl)-1,4-diazepan-1-yl]-4-[(2,3-dichlorophenyl)amino]-7-methoxyquinoline-3-carboxylate | 589 | Intermediate 155 |
| **120** | Ethyl 4-[(2-fluoro-5-methylphenyl)amino]-7-methoxy-6-(4-methylpiperazin-1-yl)quinoline-3-carboxylate | 453 | Intermediate 211 |
| **121** | Ethyl 4-[(2,5-difluorophenyl)amino]-7-methoxy-6-(4-methylpiperazin-1-yl)quinoline-3-carboxylate | 457 | Intermediate 212 |
| **122** | Ethyl 4-[(3-chloro-2-methylphenyl)amino]-7-methoxy-6-(4-methylpiperazin-1-yl)quinoline-3-carboxylate | 470 | Intermediate 213 |
| **123** | Ethyl 4-[(2-chloro-3-methylphenyl)amino]-7-methoxy-6-(4-methylpiperazin-1-yl)quinoline-3-carboxylate | 469 | Intermediate 214 |
| **124** | Ethyl 4-[(2,4-difluorophenyl)amino]-6-[3-(dimethylamino)pyrrolidin-1-yl]-7-methoxyquinoline-3-carboxylate | 471 | Intermediate 167 |
| **125** | Ethyl 4-[(3-chloro-2-fluorophenyl)amino]-7-methoxy-6-(4-methylpiperazin-1-yl)quinoline-3-carboxylate | 473 | Intermediate 215 |
| **126** | Ethyl 4-[(3-chloro-5-fluorophenyl)amino]-7-ethoxy-6-(4-methylpiperazin-1-yl)quinoline-3-carboxylate | 488 | Intermediate 216 |
| **127** | Ethyl 7-ethoxy-6-(4-methylpiperazin-1-yl)-4-[(2,3,4-trifluorophenyl)amino]quinoline-3-carboxylate | 489 | Intermediate 217 |
| **128** | Ethyl 4-[(5-chloro-2-methylphenyl)amino]-7-ethoxy-6-(4-methylpiperazin-1-yl)quinoline-3-carboxylate | 484 | Intermediate 218 |
| **129** | Ethyl 7-ethoxy-4-[(4-methoxy-2-methylphenyl)amino]-6-(4-methylpiperazin-1-yl)quinoline-3-carboxylate | 479 | Intermediate 219 |
| **130** | Ethyl 4-{[2-chloro-5-(trifluoromethyl)phenyl]amino}-7-ethoxy-6-(4-methylpiperazin-1-yl)quinoline-3-carboxylate | 538 | Intermediate 220 |
| **131** | Ethyl 4-[(2,4-difluorophenyl)amino]-7-ethoxy-6-(4-methyl-3-oxopiperazin-1-yl)quinoline-3-carboxylate | 485 | Intermediate 170 |
| **132** | Ethyl 4-[(2,3-dichlorophenyl)amino]-7-(4-ethylpiperazin-1-yl)-6-methoxyquinoline-3-carboxylate | 474 | Intermediate 168 |
| **133** | Ethyl 4-[(2-fluoro-5-methylphenyl)amino]-6-methoxy-7-(4-methylpiperazin-1-yl)quinoline-3-carboxylate | 454 | Intermediate 221 |
| **134** | Ethyl 7-(4-ethylpiperazin-1-yl)-4-[(2-fluoro-5-methylphenyl)amino]-6-methoxyquinoline-3-carboxylate | 467 | Intermediate 221 |
| **135** | Ethyl 4-[(3-chloro-2-fluorophenyl)amino]-6-methoxy-7-(4-methylpiperazin-1-yl)quinoline-3-carboxylate | 473 | Intermediate 222 |
| **136** | Ethyl 4-[(3-chloro-2-fluorophenyl)amino]-7-(4-ethylpiperazin-1-yl)-6-methoxyquinoline-3-carboxylate | 487 | Intermediate 222 |
| **137** | Ethyl 4-[(2-fluoro-5-methylphenyl)amino]-6-methoxy-7-(4-methyl-1,4-diazepan-1-yl)quinoline-3-carboxylate | 467 | Intermediate 221 |
| **138** | Ethyl 4-[(2,4-difluorophenyl)amino]-6-methoxy-7-(4-methyl-1,4-diazepan-1-yl)quinoline-3-carboxylate | 471 | Intermediate 223 |
| **139** | Ethyl 4-[(2-chloro-3-fluorophenyl)amino]-7-ethoxy-6-(4-methylpiperazin-1-yl)quinoline-3-carboxylate | 487 | Intermediate 224 |
| **140** | Ethyl 4-[(2-chloro-3-fluorophenyl)amino]-7-ethoxy-6-(4-ethylpiperazin-1-yl)quinoline-3-carboxylate | 501 | Intermediate 224 |
| **141** | Ethyl 4-[(2-chloro-3-fluorophenyl)amino]-7-ethoxy-6-(4-isopropylpiperazin-1-yl)quinoline-3-carboxylate | | Intermediate 224 |
| **142** | Ethyl 6-[4-(tert-butoxycarbonyl)piperazin-1-yl]-7-ethoxy-4-[(2-fluoro-4-methylphenyl)amino]quinoline-3-carboxylate | 553 | Intermediate 196 |
| **143** | Ethyl 4-[(2,3-dichlorophenyl)amino]-7-ethoxy-6-(3-oxopiperazin-1-yl)quinoline-3-carboxylate | 504 | Intermediate 171 |
| **144** | Ethyl 7-ethoxy-4-[(2-fluoro-5-methylphenyl)amino]-6-(3-oxopiperazin-1-yl)quinoline-3-carboxylate | 467 | Intermediate 179 |
| **145** | Ethyl 4-[(2,4-difluorophenyl)amino]-7-ethoxy-6-(3-oxopiperazin-1-yl)quinoline-3-carboxylate | 471 | Intermediate 170 |
| **146** | Ethyl 4-[(2-chloro-4-methylphenyl)amino]-7-ethoxy-6-(4-methylpiperazin-1-yl)quinoline-3-carboxylate | 9.98 (s, 1H), 8.99 (s, 1 H), 7.50 (s, 1 H), 7.30 (s, 1H), 7.10 (d, 1H), 6.90 (d, 1H), 6.77 (s, 1 H), 4.35 (q, 2 H), 4.20 (q, 2 H), 2.70 (s, 4 H), 2.40 (s, 4 H), 2.30 (s, 3 H), 2.20 (s, 3 H), 1.40 (m, 6 H) | Intermediate 225 |
| **147** | Ethyl 7-ethoxy-4-{[2-fluoro-3-(trifluoromethyl)phenyl]amino}-6-(4-methylpiperazin-1-yl)quinoline-3-carboxylate | 9.55 (s, 1 H), 8.87 (s, 1H), 7.45-7.15 (m, 5 H), 4.28 (q, 2 H), 4.12 (q, 2 H), 3.90 (s, 4 H), 2.42 (s, 4 H), 2.22 (s, 3 H), 1.45 (t, 3 H), 1.21 (t, 3 H) | Intermediate 226 |
| **148** | Ethyl 4-[(2,4-dimethoxyphenyl)amino]-7-ethoxy-6-(4-methylpiperazin-1-yl)quinoline-3-carboxylate | 9.98 (s, 1H), 8.90 (s, 1 H), 7.20 (s, 1 H), 7.00 (m, 1 H), 6.91 (s, 1H), 6.70 (s, 1H), 6.52 (t, 1 H), 4.35 (q, 2 H), 4.19 (q, 2 H), 3.80 (s, 3 H), 3.75 (s, 3 H), 2.68 (s, 4 H), 2.35 (s, 4 H), 2.20 (s, 3H), 2.35 (m, 6 H) | Intermediate 227 |
| **149** | Ethyl 4-[(2-chloro-4-fluoro-5-methylphenyl)amino]-7-ethoxy-6-(4-methylpiperazin-1-yl)quinoline-3-carboxylate | 9.89 (s, 1 H), 8.91 (s, 1 H), 7.57 (d, 1 H), 7.29 (s, 1H), 7.00 (d, 1 H), 6.75 (s, 1H), 4.32 (q, 2 H), 4.20 (q, 2 H), 2.75 (s, 4 H), 2.40 (s, 4 H), 2.21 (s, 3 H), 2.10 (s, 3 H), 1.45 (t, 3 H), 1.37 (t, 3 H) | Intermediate 228 |
| **150** | Ethyl 4-[(5-chloro-2-fluorophenyl)amino]-7-ethoxy-6-(4-methylpiperazin-1-yl)quinoline-3-carboxylate | 9.60 (s, 1H), 8.89 (s, 1H), 7.35 (m, 2 H), 7.15-7.05 (m, 3 H), 4.20 (q, 4 H), 2.90 (s, 4 H), 2.42 (s, 4 H), 2.20 (s, 3 H), 1.40 (t, 3 H), 1.25 (t, 3 H) | Intermediate 229 |
| **151** | Ethyl 7-ethoxy-4-{[2-methoxy-5-(trifluommethyl)phenyl]amino}-6-(4-methylpiperazin-1-yl)quinoline-3-carboxylate | 9.85 (s, 1H), 8.90 (s, 1H), 7.40 (m, 1 H), 7.30 (m, 2 H), 6.95 (s, 1 H), 6.85 (s, 1H), 4.30 (q, 2 H), 4.20 (q, 2 H), 3.90 (s, 3 H), 2.80 (s, 4 H), 2.40 (s, 4 H), 2.20 (s, 3 H), 1.40 (t, 3 H), 1.30 (t, 3H) | Intermediate 230 |
| **152** | Ethyl4-[(2-fluoro-4-methylphenyl)amino]-7-(2-methoxyethoxy)-6-morpholin-4-ylquinoline-3-carboxylate | 484 | Intermediate 231 |
| **153** | Ethyl 4-[(2-fluoro-4-methylphenyl)amino]-6-(4-isopropylpiperazin-1-yl)-7-(2-methoxyethoxy)quinoline-3-carboxylate | 525 | Intermediate 231 |
| **154** | Ethyl 4-[(2-fluoro-5-methylphenyl)amino]-6-(4-isopropylpiperazin-1-yl)-7-(2-methoxyethoxy)quinoline-3-carboxylate | 525 | Intermediate 232 |

### Intermediate 155

### Ethyl 6-bromo-4-[(2,3-dichlorophenyl)amino]-7-methoxyquinoline-3-carboxylate

A mixture of ethyl 6-bromo-4-chloro-7-methoxyquinoline-3-carboxylate (Intermediate 233; 1.0 g, 2.9 mmol), 2,3-dichloroaniline (535 mg, 3.2 mmol), acetic acid (900 µL) and DMF (8 mL) was heated to 100°C for 2 hours. The reaction mixture was poured into ice water, the pH adjusted to 9 with 0.1 N NaOH and the resulting precipitate filtered to give 900 mg of a solid; *m*/*z*: 471.

### Intermediates 156-232

The following compounds were prepared by a method similar to Intermediate 155 using the appropriate starting materials.

| **Int** | **Compound** | **M/z / NMR** | **SM** |
|---|---|---|---|
| **156** | Ethyl 4-[(2-fluoro-5-methylphenyl)amino]-7-(2-methoxyethoxy)-6-(4-methylpiperazin-1-yl)quinoline-3-carboxylate | 497 | Intermediate 277 |
| **157** | Ethyl4-[(2,5-difluorophenyl)amino]-7-(2-methoxyethoxy)-6-(4-methylpiperazin-1-yl)quinoline-3-carboxylate | 501 | Intermediate 277 |
| **158** | Ethyl 4-[(3-chloro-2-fluorophenyl)amino]-7-(2-methoxyethoxy)-6-(4-methylpiperazin-1-yl)quinoline-3-carboxylate | 517 | Intermediate 277 |
| **159** | Ethyl 4-[(2,3-dichlorophenyl)amino]-7-(2-methoxyethoxy)-6-(4-methylpiperazin-1-yl)quinoline-3-carboxylate | 534 | Intermediate 277 |
| **160** | Ethyl 4-[(2,4-difluorophenyl)amino]-7-(2-methoxyethoxy)-6-(4-methylpiperazin-1-yl)quinoline-3-carboxylate | 501 | Intermediate 277 |
| **161** | Ethyl 4-[(2-fluoro-4-methylphenyl)amino]-7-(2-methoxyethoxy)-6-(4-methylpiperazin-1-yl)quinoline-3-carboxylate | 497 | Intermediate 277 |
| **162** | Ethyl 7-(2-{[tert-butyl(dimethyl)silyl]oxy} ethoxy)-4-[(2,4-difluorophenyl)amino]-6-(4-methylpiperazin-1-yl)quinoline-3-carboxylate | 601 | Intermediate 284 |
| **163** | Ethyl 7-(2-{[tert-butyl(dimethyl)silyl]oxy}ethoxy)-4-[(3-chloro-2-fluorophenyl)amino]-6-(4-methylpiperazin-1-yl)quinoline-3-carboxylate | 617 | Intermediate 284 |
| **164** | Ethyl 4-[(2-chloro-3-fluorophenyl)amino]-7-(2-methoxyethoxy)-6-(4-methylpiperazin-1-yl)quinoline-3-carboxylate | 517 | Intermediate 277 |
| **165** | Ethyl 6-bromo-4-[(2,4-dichlorophenyl)amino]-7-methoxyquinoline-3-carboxylate | | Intermediate 233 |
| **166** | Ethyl 6-bromo-4-[(3,4-dichlorophenyl)amino]-7-methoxyquinoline-3-carboxylate | | Intermediate 233 |
| **167** | Ethyl 6-bromo-4-[(2,4-difluorophenyl)amino]-7-methoxyquinoline-3-carboxylate | 438 | Intermediate 233 |
| **168** | Ethyl 7-bromo-4-[(2,3-dichlorophenyl)amino]-6-methoxyquinoline-3-carboxylate | | Intermediate 236 |
| **169** | Ethyl 7-bromo-4-[(3,4-dichlorophenyl)amino]-6-methoxyquinoline-3-carboxylate | | Intermediate 236 |
| **170** | Ethyl 6-bromo-4-[(2,4-difluorophenyl)amino]-7-ethoxyquinoline-3-carboxylate | 452 | Intermediate 240 |
| **171** | Ethyl 6-bromo-4-[(2,3-dichlorophenyl)amino]-7-ethoxyquinoline-3-carboxylate | 485 | Intermediate 240 |
| **172** | Ethyl 6-bromo-4-[(3,4-dichlorophenyl)amino]-7-ethoxyquinoline-3-carboxylate | | Intermediate 240 |
| **173** | Ethyl 6-bromo-4-[(2,4-difluorophenyl)amino]-7-fluoroquinoline-3-carboxylate | | Intermediate 243 |
| **174** | Ethyl 6-bromo-4-[(2,3-dichlorophenyl)amino]-7-fluoroquinoline-3-carboxylate | | Intermediate 243 |
| **175** | Ethyl 6-bromo-4-[(2,3-dichlorophenyl)amino]-5-fluoroquinoline-3-carboxylate | | Intermediate 243 |
| **176** | Ethyl 6-bromo-7-ethoxy-4-[(4-ethylphenyl)amino]quinoline-3-carboxylate | 445 | Intermediate 240 |
| **177** | Ethyl 6-bromo-4-[(3-chloro-4-fluorophenyl)amino]-7-ethoxyquinoline-3-carboxylate | | Intermediate 240 |
| **178** | Ethyl 6-bromo-4-[(3-chloro-2-fluorophenyl)amino]-7-ethoxyquinoline-3-carboxylate | 469 | Intermediate 240 |
| **179** | Ethyl 6-bromo-7-ethoxy-4-[(2-fluoro-5-methylphenyl)amino]quinoline-3-carboxylate | 449 | Intermediate 240 |
| **180** | Ethyl 6-bromo-4-[(2-fluorophenyl)amino]-7-methoxyquinoline-3-carboxylate | 420 | Intermediate 233 |
| **181** | Ethyl 6-bromo-7-methoxy-4-[(3-methoxy-2-methylphenyl)amino]quinoline-3-carboxylate | 445 | Intermediate 233 |
| **182** | Ethyl 6-bromo-4-[(4-chloro-2-methylphenyl)amino]-7-methoxyquinoline-3-carboxylate | 449 | Intermediate 233 |
| **183** | Ethyl 6-bromo-4-[(3-chloro-2-methylphenyl)amino]-7-ethoxyquinoline-3-carboxylate | 463 | Intermediate 240 |
| **184** | Ethyl 6-bromo-7-ethoxy-4-[(3-ethylphenyl)amino]quinoline-3-carboxylate | 443 | Intermediate 240 |
| **185** | Ethyl 6-bromo-4-[(3-chlorophenyl)amino]-7-ethoxyquinoline-3-carboxylate | 449 | Intermediate 240 |
| **186** | Ethyl 6-bromo-4-[(2-chloro-4-fluorophenyl)amino]-7-ethoxyquinoline-3-carboxylate | 467 | Intermediate 240 |
| **187** | Ethyl 6-bromo-4-[(4-chloro-2-fluorophenyl)amino]-7-ethoxyquinoline-3-carboxylate | 467 | Intermediate 240 |
| **188** | Ethyl 6-bromo-7-ethoxy-4-[(3-methylphenyl)amino]quinoline-3-carboxylate | 429 | Intermediate 240 |
| **189** | Ethyl 6-bromo-4-[(2-chloro-3-methylphenyl)amino]-7-ethoxyquinoline-3-carboxylate | 463 | Intermediate 240 |
| **190** | Ethyl 6-bromo-7-ethoxy-4-[(3-fluoro-2-methylphenyl)amino]quinoline-3-carboxylate | 447 | Intermediate 240 |
| **191** | Ethyl 6-bromo-4-[(3,4-difluorophenyl)amino]-7-methoxyquinoline-3-carboxylate | 437 | Intermediate 233 |
| **192** | Ethyl 6-bromo-7-methoxy-4-{[2-methyl-3-(trifluoromethyl)phenyl]amino} quinoline-3-carboxylate | 484 | Intermediate 233 |
| **193** | Ethyl 6-bromo-4-[(4-chlorophenyl)amino]-7-methoxyquinoline-3-carboxylate | 435 | Intermediate 233 |
| **194** | Ethyl 6-bromo-4-[(2-fluoro-4-methylphenyl)amino]-7-methoxyquinoline-3-carboxylate | 434 | Intermediate 233 |
| **195** | Ethyl 6-bromo-7-methoxy-4-{[3-(trifluoromethyl)phenyl]amino}quinoline-3-carboxylate | 469 | Intermediate 233 |
| **196** | Ethyl 6-bromo-7-ethoxy-4-[(2-fluoro-4-methylphenyl)amino]quinoline-3-carboxylate | 447 | Intermediate 240 |
| **197** | Ethyl 6-bromo-7-ethoxy-4-[(3-methoxy-2-methylphenyl)amino]quinoline-3-carboxylate | 459 | Intermediate 240 |
| **198** | Ethyl 6-bromo-4-[(2,5-difluorophenyl)amino]-7-ethoxyquinoline-3-carboxylate | 454 | Intermediate 240 |
| **199** | Ethyl 6-bromo-4-[(3,4-dimethylphenyl)amino]-7-ethoxyquinoline-3-carboxylate | 444 | Intermediate 240 |
| **200** | Ethyl 7-bromo-4-[(2,4-difluorophenyl)amino]-6-ethoxyquinoline-3-carboxylate | 453 | Intermediate 246 |
| **201** | Ethyl 7-bromo-4-[(2,3-dichlorophenyl)amino]-6-ethoxyquinoline-3-carboxylate | 485 | Intermediate 246 |
| **202** | Ethyl 6-bromo-4-[(2,3-difluorophenyl)amino]-7-ethoxyquinoline-3-carboxylate | 452 | Intermediate 240 |
| **203** | Ethyl 6-bromo-4-[(2,3-dimethylphenyl)amino]-7-ethoxyquinoline-3-carboxylate | 444 | Intermediate 240 |
| **204** | Ethyl 6-bromo-4-[(4-chloro-3-fluorophenyl)amino]-7-ethoxyquinoline-3-carboxylate | 468 | Intermediate 240 |
| **205** | Ethyl 6-bromo-4-[(3-chloro-2,4-difluorophenyl)amino]-7-ethoxyquinoline-3-carboxylate | 485 | Intermediate 240 |
| **206** | Ethyl 7-bromo-4-[(3-chloro-4-fluorophenyl)amino]-6-ethoxyquinoline-3-carboxylate | 467 | Intermediate 246 |
| **207** | Ethyl 6-bromo-4-{[4-fluoro-2-(trifluoromethyl)phenyl]amino}-7-methoxyquinoline-3-carboxylate | 487 | Intermediate 233 |
| **208** | Ethyl 6-bromo-4-[(2-chloro-4-fluorophenyl)amino]-7-methoxyquinoline-3-carboxylate | 453 | Intermediate 233 |
| **209** | Ethyl 6-bromo-7-methoxy-4-{[3-(trifluoromethoxy)phenyl]amino}quinoline-3-carboxylate | 485 | Intermediate 233 |
| **210** | Ethyl 6-bromo-4-[(2,6-dimethylphenyl)amino]-7-methoxyquinoline-3-carboxylate | 429 | Intermediate 233 |
| **211** | Ethyl 6-bromo-4-[(2-fluoro-5-methylphenyl)amino]-7-methoxyquinoline-3-carboxylate | 433 | Intermediate 233 |
| **212** | Ethyl 6-bromo-4-[(2,5-difluorophenyl)amino]- 7-methoxyquinoline-3-carboxylate | 437 | Intermediate 233 |
| **213** | Ethyl 6-bromo-4-[(3-chloro-2-methylphenyl)amino]-7-methoxyquinoline-3-carboxylate | 449 | Intermediate 233 |
| **214** | Ethyl 6-bromo-4-[(2-chloro-3-methylphenyl)amino]-7-methoxyquinoline-3-carboxylate | 450 | Intermediate 233 |
| **215** | Ethyl 6-bromo-4-[(3-chloro-2-fluorophenyl)amino]-7-methoxyquinoline-3-carboxylate | 453 | Intermediate 233 |
| **216** | Ethyl 6-bromo-4-[(3-chloro-5-fluorophenyl)amino]-7-ethoxyquinoline-3-carboxylate | 469 | Intermediate 240 |
| **217** | Ethyl 6-bromo-7-ethoxy-4-[(2,3,4-trifluorophenyl)amino]quinoline-3-carboxylate | 470 | Intermediate 240 |
| **218** | Ethyl 6-bromo-4-[(5-chloro-2-methylphenyl)amino]-7-ethoxyquinoline-3-carboxylate | 465 | Intermediate 240 |
| **219** | Ethyl 6-bromo-7-ethoxy-4-[(4-methoxy-2-methylphenyl)amino]quinoline-3-carboxylate | 460 | Intermediate 240 |
| **220** | Ethyl 6-bromo-4-{[2-chloro-5-(trifluoromethyl)phenyl]amino}-7-ethoxyquinoline-3-carboxylate | 519 | Intermediate 240 |
| **221** | Ethyl 7-bromo-4-[(2-fluoro-5-methylphenyl)amino]-6-methoxyquinoline-3-carboxylate | 435 | Intermediate 236 |
| **222** | Ethyl 7-bromo-4-[(3-chloro-2-fluorophenyl)amino]-6-methoxyquinoline-3-carboxylate | 455 | Intermediate 236 |
| **223** | Ethyl 7-bromo-4-[(2,4-difluorophenyl)amino]-6-methoxyquinoline-3-carboxylate | 439 | Intermediate 236 |
| **224** | Ethyl 6-bromo-4-[(2-chloro-3-fluorophenyl)amino]-7-ethoxyquinoline-3-carboxylate | 469 | Intermediate 240 |
| **225** | Ethyl 6-bromo-4-[(2-chloro-4-methylphenyl)amino]-7-ethoxyquinoline-3-carboxylate | 9.90 (s, 1H), 9.00 (s, 1 H), 7.92 (s, 1 H), 7.40 (m, 2 H), 7.10 (m, 1H), 7.00 (m, 1 H), 4.29 (q, 2 H), 4.20 (q, 2 H), 2.31 (s, 3 H), 1.40 (t, 3 H), 1.30 (t, 3 H) | Intermediate 240 |
| **226** | Ethyl 6-bromo-7-ethoxy-4-{[2-fluoro-3-(trifluoromethyl)phenyl]amino}quinoline-3-carboxylate | 9.56 (s, 1H), 8.88 (s, 1H), 8.51 (s, 1 H), 7.50-7.30 (m, 4 H), 4.31 (q, 2 H), 3.91 (q, 2 H), 1.32 (t, 3 H), 1.10 (t, 3 H) | Intermediate 240 |
| **227** | Ethyl 6-bromo-4-[(2,4-dimethoxyphenyl)amino]-7-ethoxyquinoline-3-carboxylate | 10.05 (s, 1H), 8.92 (s, 1 H), 7.89 (s, 1 H), 7.32 (s, 1 H), 7.05 (d, 1 H), 6.71 (s, 1H), 6.52 (d, 1 H), 4.21 (q, 4 H), 3.79 (s, 3 H), 3.70 (s, 3 H), 1.40 (t, 3 H), 1.30 (t, 3 H) | Intermediate 240 |
| **228** | Ethyl 6-bromo-4-[(2-chloro-4-fluoro-5-methylphenyl)amino]-7-ethoxyquinoline-3-carboxylate | 9.80 (s, 1H), 8.95 (s, 1H), 8.05 (s, 1 H), 7.55 (m, 1 H), 7.45 (s, 1 H), 7.15 (m, 1H), 4.30 (q, 2 H), 4.18 (q, 2 H), 2.15 (s, 3 H), 1.45 (t, 3 H), 1.28 (t, 3 H) | Intermediate 240 |
| **229** | Ethyl 6-bromo-4-[(5-chloro-2-fluorophenyl)amino]-7-ethoxyquinoline-3- carboxylate | 9.55 (s, 1 H), 8.87 (s, 1H), 8.45 (s, 1 H), 7.50 (s, 1H), 7.35 (m, 1 H), 7.17 (m, 2H), 4.32 (q, 2 H), 4.00 (q, 2 H), 1.46 (t, 3 H), 1.19 (t, 3 H) | Intermediate 240 |
| **230** | Ethyl 6-bromo-7-ethoxy-4-{[2-methoxy-5-(trifluoromethyl)phenyl]amino}quinoline-3-carboxylate | 9.80 (s, 1H), 8.96 (s, 1H), 8.15 (s, 1 H), 7.45 (m, 2 H), 7.30 (m, 1H), 7.18 (s, 1 H), 4.30 (q, 2 H), 4.10 (q, 2 H), 3.85 (s, 3 H), 1.40 (t, 3 H), 1.20 (t, 3 H) | Intermediate 240 |
| **231** | Ethyl 6-chloro-4-[(2-fluoro-4-methylphenyl)amino]-7-(2-methoxyethoxy)quinoline-3-carboxylate | 433 | Intermediate 281 |
| **232** | Ethyl 6-chloro-4-[(2-fluoro-5-methylphenyl)amino]-7-(2-methoxyethoxy)quinoline-3-carboxylate | 433 | Intermediate 281 |

### Intermediate 233

### Ethyl 6-bromo-4-chloro-7-methoxyquinoline-3-carboxylate

This compound was described in WO 2002092571, and prepared in accordance with the procedures described in Burke T.R. et al., J. Med. Chem., 36 (1993) 425-432.

A solution of ethyl 6-bromo-7-methoxy-4-oxo-1,4-dihydroquinoline-3-carboxylate (Intermediate 234; 8.0 g, 0.025) in phosphorous oxychloride (100 mL) was heated under reflux overnight. After cooling, the solution was carefully poured into ~400 mL of ice water with stirring. The resulting mixture was made just basic with 2N NaOH and extracted with EtOAc. The organic layer was washed with water, dried (Na₂SO₄), and concentrated under reduced pressure to give 8.0 g (93%) of a white solid. ¹H NMR: 9.14 (s, 1 H), 8.55 (s, 1 H), 7.66 (s, 1 H), 4.42 (d, 2 H), 4.09 (s, 3 H), 1.38 (t, 3 H); *m*/*z:* 344.

### Intermediate 234

### Ethyl 6-bromo-7-methoxy-4-oxo-1,4-dihydroquinoline-3-carboxylate

A solution of diethyl {[(4-bromo-3-methoxyphenyl)amino]methylene}malonate (Intermediate 235; 11 g, 0.029 mol) in warm diphenyl ether (20 mL) was added dropwise over 15 minutes to refluxing diphenyl ether (180 mL). After 3 hours, the solution was cooled, diluted with hexane (200 mL), and the resulting precipitate collected to give 8.9 g (93%) of a white solid.

### Intermediate 235

### Diethyl {[(4-bromo-3-methoxyphenyl)amino]methylene}malonate

To a solution of 4-bromo-3-methoxyaniline (25 g, 0.12 mol) in CH₃CN (150 mL) was added diethylethoxymethylene malonate (27 mL, 0.13 mol). After 20 hours, the solvent was removed under reduced pressure and the residue dissolved in EtOAc. Hexane was added, and the resulting precipitate collected to give 37 g (80%) off-white solid. ¹H NMR: 10.68 (d, 1H), 8.38 (d, 1 H), 7.52 (d, 1 H), 7.20 (d, 1 H), 6.91 (dd, 1 H), 4.20 (q, 2 H), 4.11 (q, 2 H), 3.86 (s, 3 H), 1.23 (m, 6 H); *m*/*z*: 372.

### Intermediate 236

### Ethyl 7-bromo-4-chloro-6-methoxyquinoline-3-carboxylate

A mixture of ethyl 7-bromo-6-methoxy-4-oxo-1,4-dihydroquinoline-3-carboxylate (Intermediate 237; 4.0 g, 11.6 mmol) and phosphorous oxychloride (80 mL) was heated at reflux for 2.5 hours. The solution was cooled, and poured carefully onto ice (800 g) with stirring. The mixture was carefully neutralized with 2N NaOH, and the resulting precipitate was filtered, washed with water and dried to give 3.8 g white solid. ¹H NMR (CDCl₃): 9.00 (s, 1 H), 8.32 (s, 1H), 7.54 (s, 1 H), 4.43 (q, 2 H), 4.02 (s, 3 H), 1.39 (t, 3H).

### Intermediate 237

### Ethyl 7-bromo-6-methoxy-4-oxo-1,4-dihydroquinoline-3-carboxylate

A solution of diethyl {[(3-bromo-4-methoxyphenyl)amino]methylene}malonate (Intermediate 238; 10 g, 0.027 mol) in warm diphenyl ether (100 mL) was added dropwise over 15 minutes to refluxing diphenyl ether (100 mL). After 3 hours, the reaction mixture was cooled, and petroleum ether (120 mL) was added to the solid material, which was filtered and washed with hexane to give 8 g white solid. ¹H NMR: 8.54 (s, 1H), 7.92 (s, 1H), 7.65 (s, 1 H), 4.22 (q, 2 H), 3.95 (s, 3 H), 1.28 (t, 3 H).

### Intermediate 238

### Diethyl {[(3-bromo-4-methoxyphenyl)amino]methylene}malonate

A solution of 3-bromo-4-methoxyaniline (Intermediate 239; 8.3 g, 40.9 mmol) and diethyl ethoxymethylenemalonate (8.85 mL, 44.2 mmol) in CH₃CN (60 mL) was stirred for 2 hours. The solvent was removed under reduced pressure. Recrystallization of the residue from hexane gave 11 g white solid. ¹H NMR (CDCl₃): 10.98 (d, 1H), 8.40 (d, 1H), 7.40 (d, 1H), 7.08 (dd, 1 H), 6.91 (d, 1 H), 4.29 (m, 4 H), 3.91 (s, 3 H), 1.37 (m, 6 H).

### Intermediate 239

### 3-Bromo-4-methoxyaniline

The title compound was prepared according to the procedure in Liu Y.-Y. and Munich, M., J. Label Compd Radiophann., 18 (1981), 791-797.

### Intermediate 240

### Ethyl 6-bromo-4-chloro-7-ethoxyquinoline-3-carboxylate

### Preparation a)

A mixture of ethyl 6-bromo-7-ethoxy-4-oxo-1,4-dihydroquinoline-3-carboxylate (Intermediate 241; 16.8 g, 49 mmol) and phosphorous oxychloride (40 mL) was heated at reflux for 16 hours. The solution was cooled, and poured carefully into ice water (~500 mL) with stirring. The resulting solid was filtered, washed with water and dried to give 17.1 g of a light brown solid. ¹H NMR: 9.13 (s, 1H), 8.54 (s, 1H), 7.63 (s, 1H), 4.39 (m, 4 H), 1.46 (t, 3 H), 1.38 (t, 3 H).

### Alternative preparation b)

To a solution of diethyl {[(4-bromo-3-ethoxyphenyl)amino]methyl}malonate (Intermediate 242; 52.9 g, 0.137 mol) in toluene (125 ml) was added POCl₃ (209.9 g, 125 mL, 1.37 mol). The reaction mixture was stirred at 110°C for 48 hours, cooled and concentrated under reduced pressure. The residue was carefully treated with sat. NaHCO₃ solution until no more gas was evolved, and the resulting solid was filtered, washed with sat. NaHCO₃ and water, and then slurried in hot MeOH (~200 mL), cooled and filtered to give 42 g of an orange solid.

### Alternative preparation c)

Triethylamine (12.8 kg, 126 mol) was added to a suspension of 4-bromo-3-ethoxyaniline hydrochloride (29.94 kg, 118 mol) in toluene (119 L) and water (60 L) at ambient temperature. The suspension was stirred until a solution was obtained. The biphasic mixture was filtered through diatomaceous earth (4 kg) washing the cake with toluene (10 L) and the aqueous layer separated and discarded. Toluene was distilled out (13 L) to dry the mixture. Diethylethoxymethylene malonate (25.60 kg, 118 mol) was added slowly to the toluene solution at 70-80 °C, at a rate that maintained gentle reflux. Toluene and ethanol (70 L) were distilled out under reduced pressure (400 mbar, 85 °C). The reaction temperature was reduced to 60 °C and the reaction analysed by HPLC. Phosphorous oxychloride (45.6 kg, 297 mol) added over 45 minutes. The reaction was heated to 110 °C over 2 hours and held for at least 5 hours. The reaction was cooled to 70 °C and analysed by HPLC. Phosphorous oxychloride and toluene (50 L) were removed by distillation at reduced pressure (100-150 mbar, 50-67 °C). Toluene (40 L) was added and the mixture redistilled (40 L of distillate collected). Tetrahydrofuran (THF) (36 L) was added and the resulting mixture cooled to 20-25 °C. The resulting red solution was added slowly (over -50 minutes to control gas evolution) to a mixture of potassium bicarbonate (99 kg, 989 mol) in water (296 L) at ambient temperature. The reaction mixture was washed with further THF (3.6 L). The resulting suspension was stirred for 1 hour before isolating on a centrifuge. The wet product was slurried in ethanol (119 L) and heated to 70 °C. The slurry was cooled to ambient temperature and the product collected by centrifuge, washed with ethanol (40 L) and dried under reduced pressure (30 °C at 2 mbar) to give the title compound (30.8 kg, 86 mol, 73%).

### Intermediate 241

### Ethyl 6-bromo-7-ethoxy-4-oxo-1,4-dihydroquinoline-3-carboxylate

A solution of diethyl {[(4-bromo-3-ethoxyphenyl)amino]methyl}malonate (Intermediate 242; 25 g, 64 mmol) in warm diphenyl ether (150 mL) was added dropwise over 15 minutes to refluxing diphenyl ether (~250 mL). After 3 hours the reaction mixture was cooled, and hexane (~250 mL) added to the solid, which was filtered to give 16.8 g of a white crystalline solid, used without further purification.

### Intermediate 242

### Diethyl {[(4-bromo-3-ethoxyphenyl)amino]methyl}malonate

A solution of 4-bromo-3-ethoxyaniline (21 g, 0.1 mol) and diethyl ethoxymethylenemalonate (19 mL, 0.1 mol) in CH₃CN (150 mL) was stirred for 2 hours and then heated to 75°C for 16 hours. The solvent was removed under reduced pressure and the residue recrystallized from hexane to give 25 g of white solid, which was used without further purification.

### Intermediate 243

### Ethyl 6-bromo-4-chloro-7-fluoroquinoline-3-carboxylate and ethyl 6-bromo-4-chloro-5-fluoroquinoline-3-carboxylate

A mixture of ethyl 6-bromo-7-fluoro-4-hydroxyquinoline-3-carboxylate and ethyl 6-bromo-5-fluoro-4-hydroxyquinoline-3-carboxylate (Intermediate 244; 3.2 g, 10.19 mmol) was stirred in refluxing phosphorous oxychloride (4.6 ml, 50.96 mmol) for 3 hours. After cooling, the mixture was concentrated under reduced pressure, and CH₃CN (10 mL) was added. The mixture was poured slowly into NaHCO₃ solution and the resulting slurry extracted with EtOAc. The organic layer was dried (MgSO₄), filtered, concentrated under reduced pressure, and the residue purified by column chromatography (EtOAc/hexanes gradient) to give 0.15 g (4.7%) of ethyl 6-bromo-4-chloro-5-fluoroquinoline-3-carboxylate. ¹H NMR: 9.17 (s, 1 H), 8.64 (d, 1 H), 8.12 (d, 1H), 4.48 (q, 2 H), 1.40 (t, 3 H); *m*/*z:* 334, and 1.4 g (44%) of ethyl 6-bromo-7-fluoro-4-hydroxyquinoline-3-carboxylate ¹H NMR: 9.11 (s, 1 H), 8.22 (t, 1 H), 7.96 (d, 1 H), 4.48 (q, 2 H), 1.40 (t, 3 H); *m*/*z*: 334.

### Intermediate 244

### Ethyl 6-bromo-7-fluoro-4-hydroxyquinoline-3-carboxylate and ethyl 6-bromo-5-fluoro-4-hydroxyquinoline-3-carboxylate

A solution of diethyl {[(4-bromo-3-fluorophenyl)amino]methylene}malonate (Intermediate 245; 6.00 g, 16.66 mmol) and diphenyl ether (10 mL) was heated to 250°C for 40 minutes. After cooling, hexane (10 mL) was added, and the resulting precipitate filtered and washed with acetone (20 mL), to give 3.2 g (61 %) of a light brown solid, an insoluble mixture of isomers.

### Intermediate 245

### Diethyl {[(4-bromo-3-fluorophenyl)amino]methylene}malonate

A solution of 4-bromo-3-fluoroaniline (5.00 g, 28.3 mmol) and diethyl ethoxymethylenemalonate (5.7 mL, 28.4 mmol) in CH₃CN (15 mL) was stirred for 6 days. The reaction mixture was filtered to give 6.2 grams (65 %) of a white solid. ¹H NMR: 10.65 (d, 1 H), 8.36 (d, 1 H), 7.67 (t, 1 H), 7.59 (dd, 1 H), 7.24 (d, 1 H), 4.18 (m, 4 H), 1.29 (m, 6 H).

### Intermediate 246

### Ethyl 7-bromo-4-chloro-6-ethoxyquinoline-3-carboxylate

A suspension of ethyl 7-bromo-6-ethoxy-4-oxo-1,4-dihydroquinoline-3-carboxylate (Intermediate 247; 21 g, 0.06 mol) in phosphorous oxychloride was heated under reflux for 2.5 hours. After cooling, the reaction mixture was poured carefully into 2 L of ice water and neutralized with concentrated aqueous ammonia. The precipitate was collected, washed with water, dried, and recrystallized from EtOAc to give 17 g of an off-white solid. ¹H NMR: 9.01 (s, 1 H), 8.45 (s, 1 H), 7.63 (s, 1 H), 4.43 (q, 2 H), 4.34 (q, 2 H), 1.48 (t, 3 H), 1.38 (t, 3 H); *m*/*z*: 359.

### Intermediate 247

### Ethyl 7-bromo-6-ethoxy-4-oxo-1,4-dihydroquinoline-3-carboxylate

A solution of diethyl {[(3-bromo-4-ethoxyphenyl)amino]methylene}malonate (Intermediate 248; 33 g, 0.085 mol) in warm diphenyl ether (200 mL) was added dropwise over 30 minutes to refluxing diphenyl ether (400 mL). After 3 hours at reflux, the reaction was cooled. To the resulting gelatinous solid was added hexane, cyclohexane, and petroleum ether (~150 mL of each) and the mixture filtered. The crude material was triturated with hot hexane for 20 minutes to give 21g of a solid. ¹H NMR: 12.26 (s, 1H), 8.52 (s, 1 H), 7.91 (s, 1 H), 7.62 (s, 1 H), 4.21 (m, 4 H), 1.41 (t, 3 H), 1.28 (t, 3 H); *m*/*z*: 342.

### Intermediate 248

### Diethyl {[(3-bromo-4-ethoxyphenyl)amino]methylene} malonate

A solution of (3-bromo-4-ethoxyphenyl)amine (Intermediate 249; 22 g, 0.1 mol) and diethylethoxymethylene malonate (23 mL, 0.11 mol) in acetonitrile (150 mL) was stirred for 2 hours. The precipitate was collected and recrystallized/triturated with hexane to give 33 g of a solid, used without further purification. ¹H NMR (MeOD): 8.42 (s, 1 H), 7.51 (d, 1 H), 7.20 (m, 1 H), 7.05 (d, 1 H), 4.29 (q, 2 H), 4.22 (q, 2 H), 4.11 (q, 2 H), 1.44 (t, 3 H), 1.34 (m, 6 H); *m*/*z:* 384.

### Intermediate 249

### (3-Bromo-4-ethoxyphenyl)amine

To a stirred suspension of 2-bromo-1-ethoxy-4-nitrobenzene (Intermediate 250; 27 g, 0.11 mol), iron powder (50 g, 0.89 mol), and 50% aqueous ethanol (200 mL), was added dropwise over 30 minutes a solution of concentrated HCl (2 mL) in 50% aqueous ethanol (20 mL). Heating was applied after half the acidic solution was added to initiate the reaction. Thereafter, the reaction mixture was kept at reflux by adjusting the rate of addition of the acid. Reflux was maintained for 1.5 hours subsequent to the acid addition via external heating mantle. The reaction mixture was filtered hot through a bed of diatomaceous earth. After cooling, the aqueous filtrate was extracted with chloroform (2 x 300 mL) and EtOAc (2 x 300 mL). The organic extracts were combined, dried (Na₂SO₄), and concentrated under reduced pressure to give 22 g of a solid. ¹H NMR: 6.82 (m, 1 H), 6.53 (d, 1 H), 6.51 (s, 1 H), 3.91 (q, 2 H), 1.28 (t, 3 H); *m*/*z*: 216.

### Intermediate 250

### 2-Bromo-1-ethoxy-4-nitrobenzene

A mixture of iodoethane, (22.2 mL, 0.28 mol), 2-bromo-4-nitrophenol (Intermediate 251; 24 g, 0.11 mol) and potassium carbonate (30.6 g, 0.22 mol) in DMF (200 mL) was stirred for 18 hours. The reaction mixture was poured into ice water (2 L) and extracted with EtOAc (3 x 300 mL). The combined organic extracts were dried (Na₂SO₄), filtered, and concentrated under reduced pressure to give 27.1 g of a solid, used without further purification. ¹H NMR: 8.42 (d, 1 H), 8.25 (d, 1 H), 7.31 (d, 1 H), 4.28 (q, 2 H), 1.41 (t, 3 H).

### Intermediate 251

### 2-Bromo-4-nitrophenol

A mixture of 4-nitrophenol (50 g, 0.36 mol), acetic acid (400 mL), and bromine (27 mL, 0.52 mol) was stirred for 18 hours. Excess solvent was removed under reduced pressure and the residue was crystallized from dichloromethane/hexane to give 60 g of a solid, used without further purification. ¹H NMR (MeOD): 8.40 (d, 1 H), 8.12 (dd, 1 H), 7.02 (d, 1 H); *m*/*z*: 216.

### Intermediate 252

### 1-Ethyl-1,4-diazepane

A mixture of tert-butyl 4-ethyl-1,4-diazepane-1-carboxylate (Intermediate 253; 700 mg, 3.07 mmol) in 2M HCl in ether (10 mL) was stirred at room temperature for 2 hours. The white solid was filtered, washed with diethyl ether. The solid was dissolved in 20 ml of MeOH, about 2 g of MP-carbonate (3.1 mmol/g) was added to the solution. The resulting mixture was stirred at room temperature for 30 minutes, filtered, washed with MeOH, and the filtrate was concentrated to give 390 mg light yellow oil. ¹H NMR (CD₂Cl₂): 3.00 (m, 6H), 2.75 (m, 2 H), 2.62 (m, 2 H), 1.90 (m, 2 H), 1.10 (t, 3 H); *m*/*z:* 128.

### Intermediate 253

### tert-Butyl 4-ethyl-1,4-diazepane-1-carboxylate

A mixture of *tert*-butyl 1,4-diazepane-1-carboxylate (3 g, 15 mmol), iodoethane (3.51 g, 22.5 mmol) and potassium carbonate (4.14 g, 30 mmol) in acetonitrile (15 ml) was heated to reflux for 20 hours. The reaction mixture was cooled and filtered, washing with dichloromethane. The filtrate was concentrated and the residue purified with an ISCO chromatography system to give 700 mg oil. ¹H NMR (CD₂Cl₂): 3.60 (m, 4 H), 2.70 (m, 6 H), 1.95 (m, 2 H), 1.61 (s, 9 H), 1.20 (t, 3 H); *m*/*z*: 228.

### Intermediate 254

### Ethyl 4-[(2,4-difluorophenyl)amino]-7-isopropoxy-6-(4-methylpiperazin-1-yl)quinoline-3-carboxylate

A solution of ethyl 4-chloro-7-isopropoxy-6-(4-methylpiperazin-1-yl)quinoline-3-carboxylate (Intermediate 266; 0.25 g, 0.64 mmol), 2,4-difluoroaniline (0.22 mL, 1.91 mmol), and glacial acetic acid (0.1 mL) in EtOAc (5 mL) was heated at 77°C for 16 hours. The reaction mixture was cooled, NaHCO₃ solution (3 mL) was added, and the mixture was extracted with EtOAc (3 x 3 mL). The combined organic extracts were concentrated, and the residue purified by column chromatography (hexanes/EtOAc) to give 80 mg (53%) of a yellow solid. ¹H NMR: 9.69 (s, 1 H), 8.84 (s, 1 H), 7.39 (m, 1 H), 7.26 (s, 1 H), 7.19 (m, 1 H), 7.07 (m, 1 H), 6.96 (s, 1 H), 4.83 (m, 1 H), 4.39 (q, 2 H), 2.75 (s, 4 H), 2.37 (s, 4 H), 2.17 (s, 3 H), 1.34 (d, 6 H),1.28 (t, 3 H); *m*/*z*: 485.

### Intermediates 255-265

The following compounds were prepared by a method similar to Intermediate 254

| **Int** | **Compound** | **M/z** | **SM** |
|---|---|---|---|
| **255** | Ethyl4-[(3,4-dichlorophenyl)amino]-7-isopropoxy-6-(4-methylpiperazin-1-yl)quinoline-3-carboxylate | 517 | Intermediate 266 |
| **256** | Ethyl 4-[(3-chloro-2-fluorophenyl)amino]-7-isopropoxy-6-(4-methylpiperazin-1-yl)quinoline-3-carboxylate | 501 | Intermediate 266 |
| **257** | Ethyl 4-[(2,3-dichlorophenyl)amino]-7-isopropoxy-6-(4-methylpiperazin-1-yl)quinoline-3-carboxylate | 517 | Intermediate 266 |
| **258** | Ethyl 4-[(3-chloro-4-fluorophenyl)amino]-7-isopropoxy-6-(4-methylpiperazin-1-yl)quinoline-3-carboxylate | 501 | Intermediate 266 |
| **259** | Ethyl 4-[(2,4-difluorophenyl)amino]-6-morpholin-4-ylquinoline-3-carboxylate | 415 | Intermediate 271 |
| **260** | Ethyl4-[(3-chloro-4-fluorophenyl)amino]-6-morpholin-4-ylquinoline-3-carboxylate | 432 | Intermediate 271 |
| **261** | Ethyl 4-[(2,3-dichlorophenyl)amino]-6-morpholin-4-ylquinoline-3-carboxylate | 446 | Intermediate 271 |
| **262** | Ethyl 4-[(2,4-difluorophenyl)amino]-6-(4-methylpiperazin-1yl)quinoline-3-carboxylate | 427 | Intermediate 274 |
| **263** | Ethyl 4-[(2,4-dichlorophenyl)amino]-6-(4-methylpiperazin-1-yl)quinoline-3-carboxylate | | Intermediate 274 |
| **264** | Ethyl 4-[(3,4-dichlorophenyl)amino]-6-(4-methylpiperazin-1-yl)quinoline-3-carboxylate | | Intermediate 274 |
| **265** | Ethyl 4-[(2,3-dichlorophenyl)amino]-6-(4-methylpiperazin-1-yl)quinoline-3-carboxylate | | Intermediate 274 |

### Intermediate 266

### Ethyl 4-chloro-7-isopropoxy-6-(4-methylpiperazin-1-yl)quinoline-3-carboxylate

A mixture of diethyl ({[3-isopropoxy-4-(4-methylpiperazin-1-yl)phenyl]amino}methylene)malonate (Intermediate 267; 5.7 g, 13.6 mmol) and phosphorus oxychloride (12.4 g, 20.8 mmol) was heated at 108°C for 16 hours. The reaction mixture was cooled and concentrated under reduced pressure. The resulting dark oil was diluted with acetonitrile (20 mL), added to a stirred solution of NaHCO₃, and extracted with EtOAc (3 x 100 mL). The combined organic extracts were dried (Na₂SO₄), filtered, and concentrated under reduced pressure. Hexanes (30 mL) was added and after 15 minutes of sonication, the mixture was filtered to give 3.1 g (58%) of a brown solid. ¹H NMR: 8.89 (s, 1 H), 7.45 (s, 1 H), 7.42 (s, 1 H), 4.91 (m, 1 H), 4.39 (q, 2 H), 3.16 (s, 4 H), 2.50 (s, 4 H), 2.23 (s, 3 H), 1.37 (m, 9 H); *m*/*z*: 392.

### Intermediate 267

### Diethyl ({[3-isopropoxy-4-(4-methylpiperazin-1-yl)phenyl]amino}methylene)malonate

A solution of [3-isopropoxy-4-(4-methylpiperazin-1-yl)phenyl]amine (Intermediate 268; 5.0 g, 21.5 mmol) and diethyl ethoxymethylenemalonate (4.65 mL, 23.24 mL) in acetonitrile (20 mL) was stirred for 16 hours. The reaction mixture was concentrated under reduced pressure and purified via column chromatography (hexanes/EtOAc) to give 6.7 g (74%) of a dark viscous oil. ¹H NMR: 10.68 (d, 1 H), 8.23 (d, 1 H), 6.99 (s, 1 H), 6.84 (s, 2 H), 4.65 (m, 1 H), 4.21-4.00 (m, 4 H), 2.93 (s, 4 H), 2.42 (s, 4 H), 1.27-1.16 (m, 12 H).

### Intermediate 268

### [3-Isopropoxy-4-(4-methylpiperazin-1-yl)phenyl]amine

1-(2-Isopropoxy-4-nitrophenyl)-4-methylpiperazine (Intermediate 269; 6.0 g, 21.5 mmol) and 10% palladium on carbon (0.6 g) in MeOH (30 ml) was stirred under hydrogen gas (50 psi) for 1 hour, then filtered through a pad of diatomaceous earth. The filtrate was concentrated under reduced pressure to give 5.01 g (94%) of a brown oil. ¹H NMR: 6.59 (d, 1 H), 6.18 (d, 1 H), 6.08 (dd, 1 H), 4.65 (s, 2 H), 4.45 (m, 1 H), 2.79 (s, 4 H), 2.39 (s, 4 H), 2.17 (s, 3 H), 1.21 (d, 6 H).

### Intermediate 269

### 1-(2-Isopropoxy-4-nitrophenyl)-4-methylpiperazine

A mixture of 1-chloro-2-isopropoxy-4-nitrobenzene (Intermediate 270; 5.0 g, 23.2 mmol), 1-methyl piperazine (3.09 mL, 27.82 mmol), and potassium carbonate (3.99 g, 23.19 mmol) in DMF (10 mL) was heated at 135°C for 16 hours. The reaction mixture was cooled, added to water (200 mL) and extracted with EtOAc (3 x 100 mL). The combined organic extracts were dried (Na₂SO₄), filtered, and concentrated under reduced pressure to give 6.0 grams (93%) of a red solid. ¹H NMR: 7.80 (dd, 1 H), 7.65 (d, 1 H), 7.0 (d, 1 H), 4.71 (m, 1 H), 3.20 (m, 4 H), 2.44 (m, 4 H), 2.21 (s, 3 H), 1.32 (d, 6 H); *m*/*z*: 280.

### Intermediate 270

### 1-Chloro-2-isopropoxy-4-nitrobenzene

A mixture of 2-chloro-5-nitrophenol (10.0 g, 57.6 mmol), 2-iodopropane (6.79 mL, 69.1 mmol), caesium carbonate (1.87g, 5.76 mmol), and potassium carbonate (9.93g, 57.6 mmol) in DMF (50 mL) was heated at 35°C for 24 hours. The reaction mixture was cooled, added to water (200 mL) and extracted with EtOAc (3 x 25 mL). The combined organic extracts were dried (Na₂SO₄), filtered, and concentrated under reduced pressure to give 12.1 grams (97%) of a red solid. ¹H NMR: 7.88 (d, 1 H), 7.80 (dd, 1 H), 7.72 (d, 1 H), 4.87 (m, 1 H), 1.32 (d, 6 H).

### Intermediate 271

### Ethyl 4-chloro-6-morpholinoquinoline-3-carboxylate

Ethyl 4-hydroxy-6-morpholin-4-ylquinoline-3-carboxylate (Intermediate 272; 2.2 g, 7.28 mmol) was stirred in refluxing phosphorous oxychloride (6.6 ml, 72.8 mmol) for 3 hours. After cooling, the mixture was concentrated under reduced pressure, and CH₃CN (20 mL) added. This mixture was poured slowly into NaHCO₃ solution (150 mL) and the resulting slurry was extracted with EtOAc. The organic layer was dried (MgSO₄), filtered, and concentrated, and the residue purified by column chromatography (EtOAc/hexanes gradient) to give 1.8 g (77%) white solid. ¹H NMR: 8.86 (s, 1 H), 8.00 (d, 1 H), 7.87 (d, 1 H), 7.38 (s, 1 H), 4.45 (q, 2 H), 3.82 (s, 4 H), 3.38 (s, 4 H), 1.38 (t, 3H); *m*/*z*: 321.

### Intermediate 272

### Ethyl 4-hydroxy-6-morpholin-4-ylquinoline-3-carboxylate

A solution of diethyl {[(4-morpholin-4-ylphenyl)amino]methylene}malonate (Intermediate 273; 5.00 g, 14.35 mmol) in diphenyl ether (30 mL) was heated to 250°C for 2 hours. After cooling, hexane (20 mL) was added, and the resulting precipitate filtered and washed with acetone (20 mL) to give 2.4 g (55%) of a light brown solid. ¹H NMR: 12.21 (s, 1 H), 8.45 (s, 1 H), 7.50 (m, 3 H), 4.21 (m, 2 H), 3.78 (s, 4 H), 3.17 (s, 4 H), 1.27 (t, 3 H); *m*/*z*: 303.

### Intermediate 273

### Diethyl {[(4-morpholin-4-ylphenyl)amino]methylene}malonate

A solution of 4-(4-aminophenyl)morpholine (5.00 g, 28.1 mmol) and diethyl ethoxymethylenemalonate (6.1 mL, 30.5 mmol) in CH₃CN (10 mL) was stirred for 24 hours. The reaction mixture was added to EtOAc (50 mL), washed with brine, dried (MgSO₄), filtered, and concentrated. Hexanes (50 ml) were added to the residue and after 30 minutes sonicaton, the resulting slurry was filtered to give 7.2 g (74 %) of a brown solid. ¹H NMR: 10.75 (d, 1 H), 8.35 (d, 1 H), 7.27 (d, 2 H), 6.98 (d, 2 H), 4.22 (m, 4 H), 3.73 (s, 4 H), 3.08 (s, 4 H), 1.25 (m, 6 H).

### Intermediate 274

### Ethyl 4-chloro-6-(4-methylpiperazin-1-yl)quinoline-3-carboxylate

Ethyl 4-hydroxy-6-(4-methylpiperazin-1-yl)quinoline-3-carboxylate (Intermediate 275; 0.5 g, 1.5 mmol) was stirred in refluxing phosphorous oxychloride (1.5 ml, 15.9 mmol) for 3 hours. After cooling, the mixture was concentrated under reduced pressure, and dichloromethane (10 mL) added. This mixture was poured slowly into NaHCO₃ solution (50 mL) and the resulting slurry extracted with EtOAc. The organic layer was dried (MgSO₄), filtered, and concentrated, and the residue taken up in hexane and filtered to give 0.4 g (76%) black solid. ¹H NMR: 8.84 (s, 1 H), 7.97 (d, 1 H), 7.85 (d, 1 H), 7.35 (s, 1 H), 4.45 (q, 2 H), 3.40 (m, 4 H), 2.47 (m, 4 H), 2.25 (s, 3 H), 1.38 (t, 3 H); *m*/*z*: 334.

### Intermediate 275

### Ethyl 4-hydroxy-6-(4-methylpiperazin-1-yl)quinoline-3-carboxylate

A solution of diethyl ({[4-(4-methylpiperazin-1-yl)phenyl]amino}methylene)malonate (Intermediate 276; 5.00 g, 13.8 mmol) in diphenyl ether (30 mL) was heated to 250°C for 2 hours. After cooling, hexane (20 mL) was added, and the resulting precipitate was filtered and washed with acetone (20 mL) to give 0.52 g (10%) of a light brown solid. ¹H NMR: 12.19 (s, 1 H), 8.43 (s, 1 H), 7.50 (m, 3 H), 4.23 (m, 2 H), 3.19 (m, 4 H), 2.47 (m, 4 H), 2.23 (s, 3 H), 1.27 (t, 3 H).

### Intermediate 276

### Diethyl ({[4-(4-methylpiperazin-1-yl)phenyl]amino}methylene)malonate

A solution of 4-(4-methylpiperazino)aniline (3.0 g, 15.7 mmol) and diethyl ethoxymethylenemalonate (3.4 mL, 16.9 mmol) in CH₃CN (10 mL) was stirred for 24 hours. The reaction mixture was concentrated under reduced pressure, hexane (30 ml) was added to the residue, and the resulting slurry was filtered to give 5.0 g (88 %) of a brown solid. ¹H NMR: 10.74 (d, 1 H), 8.34 (d, 1 H), 7.24 (d, 2 H), 6.97 (d, 2 H), 4.14 (m, 4 H), 3.11 (m, 4 H), 2.44 (m, 4 H), 2.21 (s, 3 H), 1.25 (m, 6 H).

### Intermediate 277

### Ethyl 4-chloro-7-(2-methoxvethoxy)-6-(4-methylpiperazin-1-yl)quinoline-3-carboxylate

A mixture of diethyl ({[3-(2-methoxyethoxy)-4-(4-methylpiperazin-1-yl)phenyl]amino}methylene)malonate (Intermediate 278; 6.6 g,15.15 mmol) and phosphorus oxychloride (23.24 g, 151.5 mmol) was heated at 110°C for 16 hours. The reaction mixture was cooled and concentrated under reduced pressure. The resulting dark oil was diluted with CH₃CN (20 mL) and added to a stirred solution of NaHCO₃. The resulting mixture was extracted with dichloromethane (3 x 25 mL). The combined organic extracts were dried (Na₂SO₄), filtered, and concentrated under reduced pressure. The resulting oil was purified via silica chromatography (EtOAc) to give 1.25 g (20%) of a pale yellow powder. ¹H NMR: 8.92 (s, 1 H), 7.48 (s, 2 H), 4.41 (q, 2 H), 4.39 (q, 2 H), 4.34 (m, 2 H), 3.78 (m, 2 H), 3.37 (s, 3 H), 3.22 (m, 4 H), 2.53 (s, 4 H), 2.26 (s, 3 H), 1.37 (t, 3 H); *m*/*z:* 408.

### Intermediate 278

### Diethyl ({[3-(2-methoxyethoxy)-4-(4-methylpiperazin-1 yl)phenyl]amino}methylene)malonate

1-[2-(2-Methoxyethoxy)-4-nitrophenyl]-4-methylpiperazine (Intermediate 279; 7.0 g, 23.70 mmol), 10% palladium on carbon (0.7g), and MeOH (20 ml) were combined under a nitrogen atmosphere. The nitrogen atmosphere was removed and 50 psi of hydrogen gas was applied for 1 hour while shaking the reaction vessel. The hydrogen gas was removed, and replaced with nitrogen gas. Diethyl ethoxymethylenemalonate (4.84 mL, 24.18 mmol) was added, and the resulting mixture was allowed to stand for 16 hours. The resulting black slurry was filtered through a pad of diatomaceous earth, and the filtrate was concentrated under reduced pressure. The resulting oil was purified via silica chromatography (EtOAc) to give 6.6 grams (64%) of a yellow oil. ¹H NMR: 6.59 (d, 1 H), 6.18 (d, 1 H), 6.08 (dd, 1 H), 4.65 (s, 2 H), (s, 2 H), 4.45 (m, 1 H), 2.79 (s, 4 H), 2.39 (s, 4 H), 2.17 (s, 3 H), 1.22 (s, 3 H), 1.20 (s, 3 H); *m*/*z*: 436.

### Intermediate 279

### 1-[2-(2-Methoxyethoxy)-4-nitrophenyl]-4-methylpiperazine

To a solution of 1-chloro-2-(2-methoxyethoxy)-4-nitrobenzene (Intermediate 280; 5.0 g, 23.19 mmol) in DMF (10 mL) at 60°C was added *N*-methyl piperazine (3.09 mL, 27.82 mmol). After heating at 130°C for 26 hours, the reaction mixture was cooled and added to NaHCO₃ solution (50 mL). The mixture was extracted with EtOAc (3 x 100 mL), and the combined organic extracts were dried (Na₂SO₄), filtered, and concentrated under reduced pressure to give 6.1 grams (92.6%) of a red solid. ¹H NMR: 7.80 (dd, 1 H), 7.69 (d, 1 H), 7.0 (d, 1 H), 4.21 (m, 2 H), 3.71 (m, 2 H), 3.34 (s, 3 H), 3.24 (m, 4 H), 2.44 (m, 4 H), 2.22 (s, 3 H); *m*/*z:* 296.

### Intermediate 280

### 1-Chloro-2-(2-methoxyethoxy)-4-nitrobenzene

A solution of 2-chloro-5-nitrophenol (10.0 g, 57.62 mmol), 1-bromo-2-methoxyethane (8.4 mL, 69.14 mmol), and potassium carbonate (11.92g, 69.14 mmol) in DMF (40 mL) was heated at 40°C for 48 hours. The reaction mixture was cooled and added to NaHCO₃ solution (100 mL) and dichloromethane (100 mL). The resulting mixture was extracted with dichloromethane (3 x 100 mL). The combined organic extracts were dried with (Na₂SO₄), filtered, and concentrated under reduced pressure to give a red solid. The red solid was filtered through a pad of silica gel, eluting with a 9:1 ratio of hexanes:EtOAc. The filtrate was concentrated to give 10.3 g (77%) of a red-orange crystalline solid. ¹H NMR: 7.92 (d, 1 H), 7.85 (dd, 1 H), 7.75 (d, 1 H), 4.35 (m, 2 H), 3.73 (m, 2 H), 3.34 (s, 3 H).

### Intermediate 281

### Ethyl 4,6-dichloro-7-(2-methoxyethoxy)quinoline-3-carboxylate

A solution of ethyl 6-chloro-4-hydroxy-7-(2-methoxyethoxy)quinoline-3-carboxylate (Intermediate 282; 6.7 g, 20.56 mmol), thionyl chloride (4.5 mL, 61.69 mmol), and DMF (1 drop) in CH₃CN (25 mL) was heated at 60°C for 2 hours. The reaction mixture was cooled and added slowly to NaHCO₃ solution (100 mL). The resulting slurry was extracted with EtOAc (3 x 100 mL), the organic extracts were combined and dried (Na₂SO₄). Purification of the residue via silica chromatography (hexanes/EtOAc gradient) gave 5.5 g (78%) of a white solid. ¹H NMR: 9.10 (s, 1 H), 8.34 (s, 1 H), 7.69 (s, 1 H), 4.44 (m, 4 H), 3.81 (m, 2 H), 3.37 (s, 3 H), 1.38 (t, 3 H).

### Intermediate 282

### Ethyl 6-chloro-4-hydroxy-7-(2-methoxyethoxy)quinoline-3-carboxylate

To a solution of Dowtherm A (25 mL) at 250°C, was added diethyl ({[4-chloro-3-(2-methoxyethoxy)phenyl]amino}methylene)malonate (Intermediate 283; 12.7 g, 34.16 mmol) and heating continued for 1 hour. The reaction vessel was cooled, hexanes (100 mL) was added and the resulting slurry was filtered and dried to give 10.5 g (94%) of an insoluble grey solid.

### Intermediate 283

### Diethyl ({[4-chloro-3-(2-methoxyethoxy)phenyl]amino}methylene)malonate

A mixture of 1-chloro-2-(2-methoxyethoxy)-4-nitrobenzene (Intermediate 280; 10.3 g, 44.47 mmol), 5% platinum on carbon (1.0g), and zinc(II)iodide (2.84 g, 8,89 mmol) in EtOAc (40 ml) under nitrogen was evacuated to hydrogen (1 atm) and stirred for 24 hours. The hydrogen was replaced with nitrogen, and diethyl ethoxymethylenemalonate (9.07 mL, 24.18 mmol) and Na₂SO₄ (1 g) were added. After 16 hours, the resulting black slurry was filtered through a pad of diatomaceous earth, and the filtrate was concentrated under reduced pressure. The resulting oil was purified via silica chromatography (hexanes/EtOAc gradient) to give 12.7 grams (77%) of an off-white powder. ¹H NMR: 10.70 (d, 1 H), 8.40 (d, 1 H), 7.40 (d, 1 H), 7.26 (s, 1 H), 6.98 (d, 1 H), 4.24 (m, 6 H), 3.72 (m, 2 H), 3.34 (s, 3 H), 1.28 (m, 6 H); *m*/*z*: 372.

### Intermediate 284

### Ethyl 7-(2-{[tert-butyl(dimethyl)silyl]oxy}ethoxy)-4-chloro-6-(4-methylpiperazin-1-yl)quinoline-3-carboxylate

A solution of diethyl ({[3-(2-{[*tert*-butyl(dimethyl)silyl]oxy}ethoxy)-4-(4-methylpiperazin-1-yl)phenyl]amino}methylene)malonate (Intermediate 285; 2.0 g, 3.73 mmol), triethylamine (9.05 g, 89.59 mmol) and phosphorus oxychloride (1.14 g, 7.47 mmol) in toluene (10 mL) was heated at 100°C for 24 hours. The reaction mixture was cooled, acetonitrile (10 mL) was added, and the resulting mixture was added to NaHCO₃ solution. The mixture was extracted with EtOAc (3 x 100 mL). The combined organic extracts were dried (Na₂SO₄), filtered, and concentrated under reduced pressure. The resulting oil was purified via silica chromatography (EtOAc/MeOH gradient) to give 0.545 g (29%) of a pale yellow solid. ¹H NMR: 8.84 (s, 1 H), 7.40 (s, 2 H), 4.32 (q, 2 H), 4.22 (m, 2 H), 3.94 (m, 2 H), 3.16 (s, 4 H), 2.45 (s, 4 H), 1.28 (t, 3 H), 0.79 (s, 9 H), 0.00 (s, 6 H); *m*/*z:* 508.

### Intermediate 285

### Diethyl({[3-(2-{[tert-butyl(dimethyl)silyl]oxy}ethoxy)-4-(4-methylpiperazin-1-yl)phenyl]amino}methylene)malonate

A mixture of 1-[2-(2-{[*tert*-butyl(dimethyl)silyl]oxy}ethoxy)-4-nitrophenyl]-4-methylpiperazine (Intermediate 286; 1.5 g, 3.79 mmol) and 10% palladium on carbon (0.4g) in EtOAc (10 ml) under nitrogen was evacuated to hydrogen (1 atm) and stirred for 16 hours. The hydrogen gas was removed, and replaced with nitrogen gas. Diethyl ethoxymethylenemalonate (0.774 mL, 3.87 mmol) was added, and after stirring for 24 hours, the resulting black slurry was filtered through a pad of diatomaceous earth. The filtrate was concentrated under reduced pressure to give 2.0 g (99%) of a white solid. ¹H NMR: 10.72 (d, 1 H), 8.37 (d, 1 H), 7.02 (s, 1 H), 6.86 (s, 2 H), 4.23-4.10 (m, 6 H), 3.92 (m, 2 H), 2.97 (m, 4 H), 2.43 (m, 4 H), 2.20 (s, 3 H), 1.25 (m, 6 H), 0.86 (s, 9 H), 0.07 (s, 6 H); *m*/*z:* 536.

### Intermediate 286

### 1-[2-(2-{[tert-Butyl(dimethyl)silyl]oxy}ethoxy)-4-nitrophenyl]-4-methylpiperazine

A solution of *tert*-butyl[2-(2-chloro-5-nitrophenoxy)ethoxy]dimethylsilane (Intermediate 287; 6.0 g, 18.08 mmol) and *N*-methylpiperazine (4.41 mL, 39.77 mmol) in DMF (10 mL) was heated at 100°C for 3 days. After cooling, water (20 mL) was added, the mixture was extracted with EtOAc (3 x 50 mL). The combined organic extracts were dried (Na₂SO₄), filtered, and concentrated, and the residue purified via silica chromatography (hexanes/EtOAc gradient) to give 5.0 g (70 %) of a yellow solid. ¹H NMR: 7.84 (dd, 1 H), 7.70 (d, 1 H), 7.02 (d, 1 H), 4.17 (m, 2 H), 3.96 (m, 2 H), 3.26 (m, 4 H), 2.45 (m, 4 H), 2.21 (s, 3 H), 0.86 (s, 9 H), 0.07 (s, 6 H); *m*/*z:* 396.

### Intermediate 287

### tert-Butyl[2-(2-chloro-5-nitrophenoxy)ethoxy]dimethylsilane

A mixture of 2-chloro-5-nitrophenol (6.0 g, 34.57 mmol), (2-bromoethoxy)-*tert-*butyldimethylsilane (8.6 mL, 41.5 mmol) and potassium carbonate (7.15 g, 41.5 mmol), in DMF (40 mL) was heated at 40°C for 4 days. The reaction mixture was filtered. Brine (200 mL) was added, and the mixture was extracted with EtOAc (3x 50 mL). The combined organic extracts were concentrated, and the residue purified by silica chromatography (hexanes/EtOAc gradient) to give 6.5 g (57%) of a white solid. ¹H NMR: 7.94 (s, 1 H), 7.84 (dd, 1 H), 7.74 (d, 1 H), 4.325 (m, 2 H), 3.96 (m, 2 H), 0.82 (s, 9 H), 0.04 (s, 6 H).

## Claims

1. A compound of formula IA or IB: 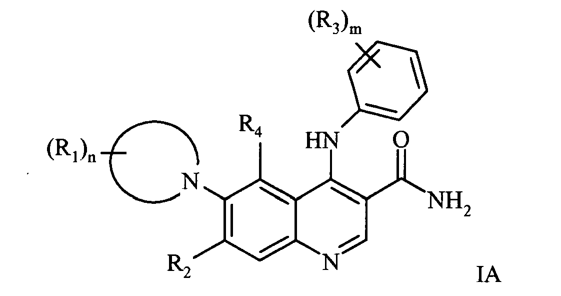 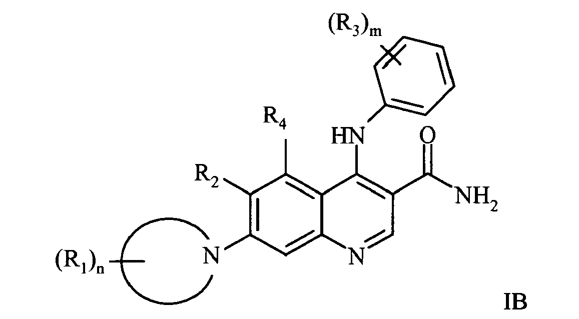 or a pharmaceutically acceptable salt thereof, wherein: 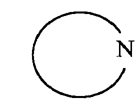 is a 3-10 membered, nitrogen linked, heterocycle or heteroaryl; wherein if said heterocycle or heteroaryl contains an -NH- moiety that nitrogen may be optionally substituted by a group selected from R₅;
**R₁** at each occurrence is independently halo, hydroxy, nitro, formyl, cyano, -CO₂H, -SH, (C₁-C₆)alkyl, (C₂-C₆)alkenyl, (C₂-C₆)alkynyl, (C₃-C₆)cycloalkyl, (C₁-C₆)alkoxy, -OC(O)-(C₁-C₆)alkyl, -C(O)-(C₁-C₆)alkyl, -CO₂(C₁-C₆)alkyl, -NR'R", -NR'-C(O)-(C₁-C₆)alkyl, (C₁-C₆)alkylS(O)ₐ- wherein a is 0 to 2, -NR'-C(O)-O(C₁-C₆)alkyl, -NR'-SO₂-(C₁-C₆)alkyl, -NR'-C(O)NR'R", -SO₂-NR'R",-C(O)-NR'R", carbocyclyl, heterocyclo, heteroaryl or oxo;
**R₂** is hydrogen, halo, hydroxy, nitro, formyl, -CO₂H, -SH, cyano, (C₁-C₆)alkyl, (C₂-C₆)alkenyl, (C₂-C₆)alkynyl, (C₃-C₆)cycloalkyl, -O-(C₃-C₆)cycloalkyl, -OC(O)-(C₁-C₆)alkyl, -C(O)-(C₁-C₆)alkyl, -CO₂(C₁-C₆)alkyl, -NR'R", -NR'-C(O)-(C₁-C₆)alkyl, (C₁-C₆)alkylS(O)ₐ- wherein a is 0 to 2, -NR'-C(O)-O(C₁-C₆)alkyl, -NR'-SO₂-(C₁-C₆)alkyl, -NR'-C(O)NR'R", -SO₂-NR'R", -C(O)-NR'R", -OC(O)-NR'R", carbocyclyl, heterocyclo, heteroaryl or (C₁-C₆)alkoxy;
**R₃** at each occurrence is independently halo, nitro, formyl, cyano, hydroxy, -NR'R", -CO₂H, -C(O)-(C₁-C₆)alkyl, -CO₂(C₁-C₆)alky1, -C(O)-NR'R", -NR'-C(O)-(C₁-C₆)alkyl, -NR'-C(O)NR'R", -NR'-C(O)-O(C₁-C₆)alkyl, -O-C(O)-(C₁-C₆)alkyl, -SH, -SO₂-NR'R", (C₁-C₆)alkyl, (C₂-C₆)alkenyl, (C₂-C₆)alkynyl, (C₁-C₆)alkoxy, (C₁-C₆)alkyIS(O)ₐ- wherein a is 0 to 2, -NR'-SO₂-(C₁-C₆)alkyl, carbocyclyl, heterocyclo, or heteroaryl, wherein if said heterocyclo or heteroaryl contains an -NH- moiety that nitrogen may be optionally substituted by (C₁-C₆)alkyl; or
two R₃ groups on adjacent carbons may optionally form a 5- or 6-membered saturated, partially unsaturated, unsaturated and/or aromatic ring optionally containing 0, 1, or 2 heteroatoms selected from S, O, or NRₐ wherein Rₐ is absent, H or (C₁-C₆)alkyl;
**R₄** is hydrogen or halo;
**m** is 0-3; wherein the values of R₃ may be the same or different;
**n** is 0-3; wherein the values of R₁ may be the same or different;
**p** is independently 1 or 2 at each occurrence; and
**R₅** is selected from (C₁-C₆)alkyl, (C₃-C₆)cycloalkyl, -C(O)-(C₁-C₆)alkyl, -S(O)ₚ(C₁-C₆)alkyl, -CO₂(C₁-C₆)alkyl, -C(O)-NR'R", benzyl, benzyloxycarbonyl, benzoyl and phenylsulphonyl;
**R'** and **R"** independently at each occurrence are H, optionally substituted (C₁-C₆)alkyl, or optionally substituted aryl, or taken together with the nitrogen to which they are attached form an optionally substituted 3-6 membered ring saturated or partially unsaturated containing 0 or 1 additional heteroatom selected from NRₐ; wherein said optional substituents may be selected from one or more R₆;
**R₆** may be independently (C₁-C₆)alkyl, halo(C₁-C₆)alkyl, halo, nitro, cyano, hydroxy, (C₁-C₆)alkoxy, -NR^{x}R^{y}, —COOR^{x} or -CONR^{x}R^{y}; and
**R^{x}** and **R^{y}** are independently of each other hydrogen or (C₁-C₆)alkyl; and wherein
each **Rₐ, R₁, R₂, R₃** and **R₅** may be optionally substituted on carbon by one or more formyl, -SH, azido, halo, nitro, cyano, hydroxy, trifluoromethoxy, -OC(O)-(C₁-C₆)alkyl, -NR'R", -CO₂H, -C(O)-(C₁-C₆)alkyl, -CO₂(C₁-C₆)alkyl, -C(O)-NR'R", -S-(C₁-C₆)alkyl, -SOₚ-(C₁-C₆)alkyl, -SOₚNR'R", (C₁-C₆)alkyl, (C₃-C₆)cycloalkyl, -NR'-C(O)-(C₁-C₆)alkyl, -NR'-C(O)-O(C₁-C₆)alkyl, -NR'-SO₂-(C₁-C₆)alkyl, -NR'-C(O)NR'R", (C₂-C₆)alkenyl, (C₂-C₆)alkynyl, or (C₁-C₆)alkoxy.

2. A compound of formula IA as claimed in claim 1 or a pharmaceutically acceptable salt thereof.

3. A compound of formula IB as claimed in claim 1 or a pharmaceutically acceptable salt thereof.

4. A compound of formula IA or IB or a pharmaceutically acceptable salt thereof as claimed in any one of claims 1-3 wherein: 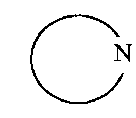 is a 5-7 membered, nitrogen linked, heterocycle; wherein if said heterocycle contains an -NH- moiety that nitrogen may be optionally substituted by a group selected from R₅; wherein
R₅ is selected from (C₁-C₆)alkyl, (C₃-C₆)cycloalkyl, -C(O)-(C₁-C₆)alkyl and -CO₂(C₁-C₆)alkyl; and
each R₅ may be optionally substituted on carbon by one or more cyano, hydroxy, -OC(O)-(C₁-C₆)alkyl, -NR'R", (C₃-C₆)cycloalkyl or (C₁-C₆)alkoxy; wherein
R' and R" independently at each occurrence are (C₁-C₆)alkyl.

5. A compound of formula IA or IB or a pharmaceutically acceptable salt thereof as claimed in any one of claims 1-4 wherein R₁ at each occurrence is hydroxy, -NR'R" or oxo; wherein R' and R" independently at each occurrence are (C₁-C₆)alkyl.

6. A compound of formula IA or IB or a pharmaceutically acceptable salt thereof as claimed in any one of claims 1-5 wherein n is 0 or 1.

7. A compound of formula IA or IB or a pharmaceutically acceptable salt thereof as claimed in any one of claims 1-6 wherein R₂ is hydrogen, halo or (C₁-C₆)alkoxy; wherein R₂ may be optionally substituted on carbon by one or more (C₁-C₆)alkoxy or hydroxy.

8. A compound of formula IA or IB or a pharmaceutically acceptable salt thereof as claimed in any one of claims 1-7 wherein R₃ at each occurrence is independently halo, (C₁-C₆)alkyl or (C₁-C₆)alkoxy; wherein R₃ may be optionally substituted on carbon by halo.

9. A compound of formula IA or IB or a pharmaceutically acceptable salt thereof as claimed in any one of claims 1-8 wherein m is 1-3; wherein the values of R₃ may be the same or different.

10. A compound of formula IA or IB or a pharmaceutically acceptable salt thereof as claimed in any one of claims 1-9 wherein R₄ is hydrogen or fluoro.

11. A compound of formula IA or IB: 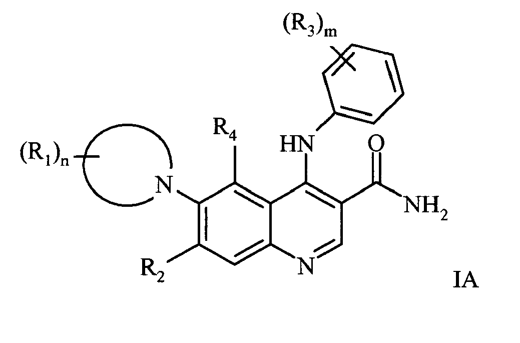 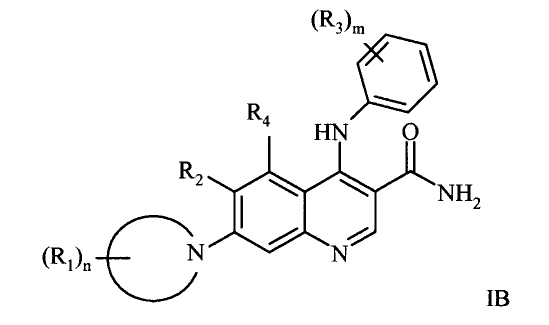 wherein: 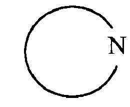 is piperazin-1-yl, *N*-methylpiperazin-1-yl, *N*-ethylpiperazin-1-yl, *N*-isopropylpiperazin-1-yl, *N*-acetylpiperazin-1-yl, *N*-(2-hydroxyacetyl)piperazin-1-yl, *N*-(2-dimethylaminoethyl)piperazin-1-yl, *N*-(2-methoxyethyl)piperazin-1-yl, *N*-(2-cyanoethyl)piperazin-1-yl, *N*-(2-hydroxyethyl)piperazin-1-yl, *N*-(cyclopropylmethyl)piperazin-1-yl, *N-*(cyclopropyl)piperazin-1-yl, *N*-((R)-2-hydroxypropionyl)piperazin-1-yl, *N*-((S)-2-hydroxypropionyl)piperazin-1-yl, *N*-(*t*-butoxycarbonyl)piperazin-1-yl, *N*-(acetoxyacetyl)piperazin-1-yl, piperidin-1-yl, morpholino, homopiperazin-1-yl, *N*-methylhomopiperazin-1-yl, *N*-ethylhomopiperazin-1-yl, *N*-acetylhomopiperazin-1-yl, *N*-isopropylhomopiperazin-1-yl, *N*-cyclopropylhomopiperazin-1-yl and pyrrolidin-1-yl;
R₁ at each occurrence is hydroxy, -NMe₂ or oxo;
n is 0 or 1;
R₂ is hydrogen, fluoro, methoxy, ethoxy, 2-(methoxy)ethoxy, 2-hydroxyethoxy or isopropoxy;
R₃ at each occurrence is independently fluoro, chloro, methyl, trifluoromethyl, ethyl, methoxy or trifluoromethoxy;
m is 1-3; wherein the values of R₃ may be the same or different;
R₄ is hydrogen or fluoro;
or a pharmaceutically acceptable salt thereof.

12. A compound of formula IA or IB: 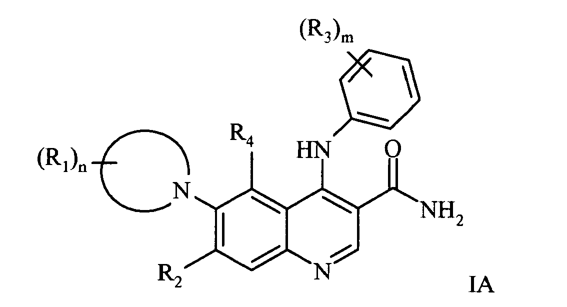 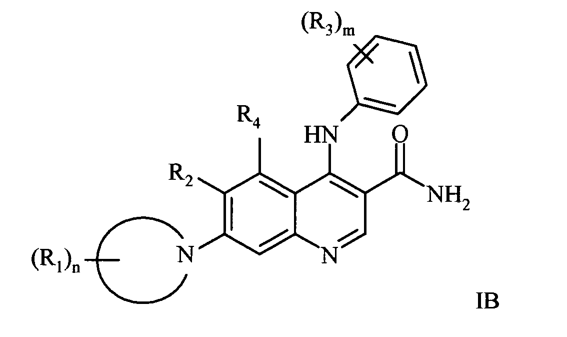 selected from:
4-[(2,4-difluorophenyl)amino]-7-ethoxy-6-(4-methylpiperazin-1-yl)quinoline-3-carboxamide;
4-[(2,3-dichlorophenyl)amino]-7-ethoxy-6-(4-methylpiperazin-1-yl)quinoline-3-carboxamide;
7-ethoxy-4-[(2-fluoro-5-methylphenyl)amino]-6-(4-isopropylpiperazin-1-yl)quinoline-3-carboxamide;
4-[(3-chloro-2-fluorophenyl)amino]-7-ethoxy-6-(4-methylpiperazin-1-yl)quinoline-3-carboxamide;
7-ethoxy-4-[(2-fluoro-5-methylphenyl)amino]-6-(4-methylpiperazin-1-yl)quinoline-3-carboxamide;
7-ethoxy-4-[(2-fluoro-4-methylphenyl)amino]-6-(4-methylpiperazin-1-yl)quinoline-3-carboxamide;
4-[(2,4-difluorophenyl)amino]-7-(2-methoxyethoxy)-6-(4-methylpiperazin-1-yl)quinoline-3-carboxamide;
4-[(2-fluoro-4-methylphenyl)amino]-7-(2-methoxyethoxy)-6-(4-methylpiperazin-1-yl)quinoline-3-carboxamide;
4-[(2-fluoro-5-methylphenyl)amino]-7-(2-methoxyethoxy)-6-(4-methylpiperazin-1-yl)quinoline-3-carboxamide; and
4-[(2-fluoro-4-methylphenyl)amino]-6-(4-isopropylpiperazin-1-yl)-7-(2-methoxyethoxy)quinoline-3-carboxamide.

13. A compound of formula IA or IB according to Claim 12 where the compound is 4-[(2,4-difluorophenyl)amino]-7-ethoxy-6-(4-methylpiperazin-1-yl)quinoline-3-carboxamide.

14. A process for preparing a compound of formula IA or IB or a pharmaceutically acceptable salt thereof, as claimed in claim 1, which process, wherein variable groups are, unless otherwise specified, as defined in claim 1, comprises of:
*Process a)* reacting a compound of formula IIA or IIB: 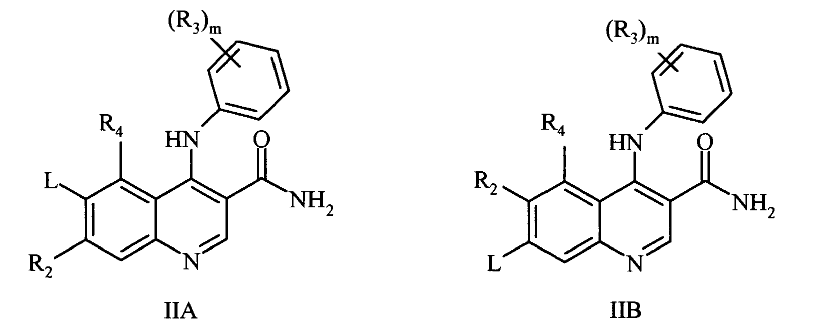 wherein L is a displaceable atom or group; with a compound of formula III: 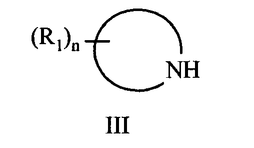 or
*Process b)* reacting a compound of formula IVA or IVB: 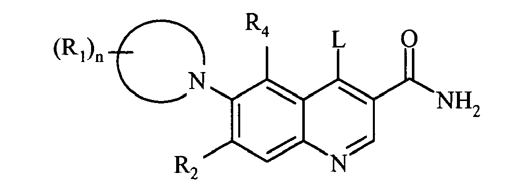 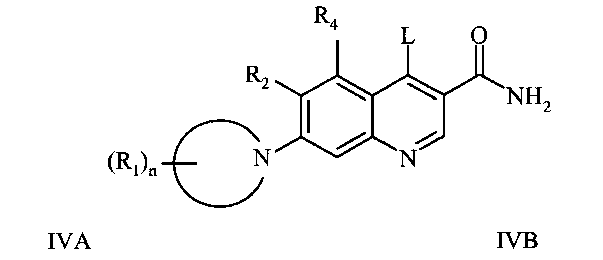 wherein L is a displaceable atom or group; with a compound of formula V: 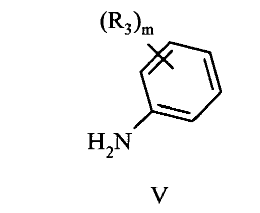 or
*Process c)* reacting a compound of formula VIA or VIB: 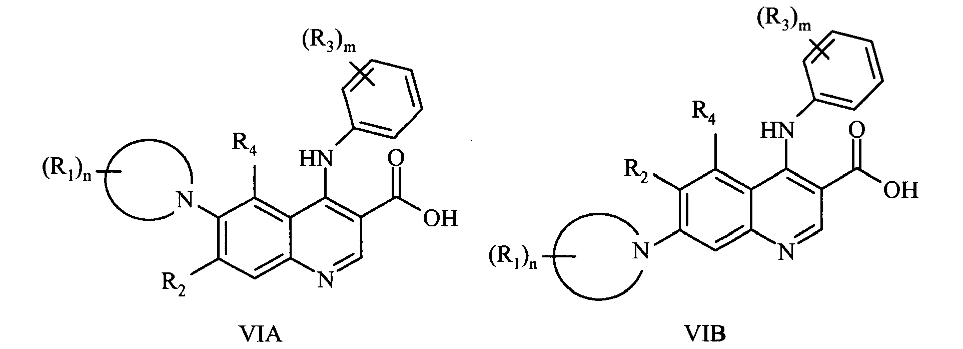 or an activated derivative thereof; with ammonia;
or
*Process d)* reacting a compound of formula VIIA or VIIB: 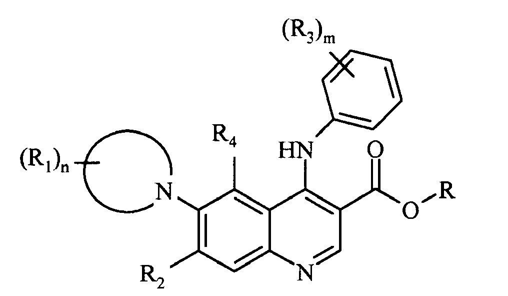 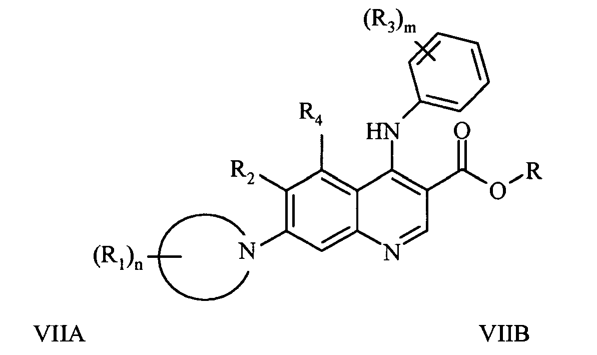 wherein R is (C₁-C₆)alkyl, in particular methyl and ethyl; with formamide and a base;
or
*Process e)* hydrolysis of a compound of formula VIIIA or VIIIB: 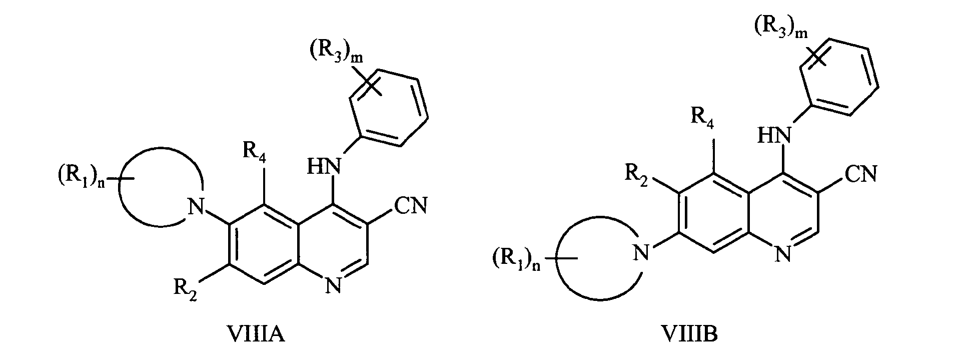 and thereafter if necessary:
i) converting a compound of the formula IA or IB into another compound of the formula IA or IB;
ii) removing any protecting groups;
iii) forming a pharmaceutically acceptable salt.

15. A pharmaceutical composition which comprises a compound of formula IA or IB, or a pharmaceutically acceptable salt thereof, as claimed in any one of claims 1-13, in association with a pharmaceutically-acceptable diluent or carrier.

16. A compound of the formula IA or IB, or a pharmaceutically acceptable salt thereof, as claimed in any one of claims 1-13, for use as a medicament.

## Patentansprüche

1. Verbindung der Formel IA oder IB: 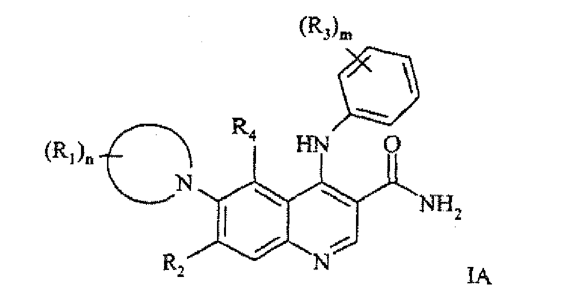 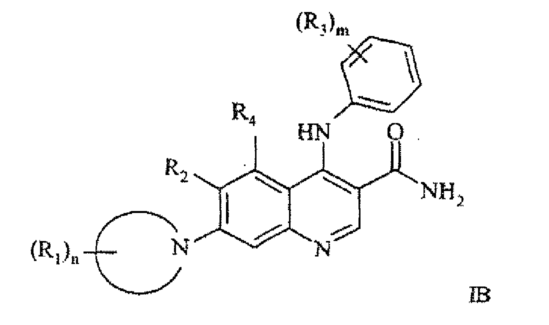 oder ein pharmazeutisch annehmbares Salz davon, wobei: 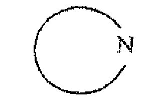 für einen 3- bis 10-gliedrigen, über Stickstoff gebundenen Heterocylus oder ein 3- bis 10-gliedriges, über Stickstoff gebundenes Heteroaryl steht;
wobei dann, wenn der Heterocyclus oder das Heteroaryl eine -NH-Gruppierung enthält, dieser Stickstoff gegebenenfalls durch eine unter R₅ ausgewählte Gruppe substituiert sein kann;
R₁ jeweils unabhängig voneinander für Halogen, Hydroxy, Nitro, Formyl, Cyano, -CO₂H, -SH, (C₁-C₆)-Alkyl, (C₂-C₆) -Alkenyl, (C₂-C₆) -Alkinyl, (C₃-C₆) - Cycloalkyl, (C₁-C₆) -Alkoxy, -OC (O) - (C₁-C₆) -Alkyl, -C(O) - (C₁-C₆) -Alkyl, -CO₂- (C₁-C₆) -Alkyl, -NR'R", -NR'-C(O)-(C₁-C₆)-Alkyl, (C₁-C₆)-Alkyl-S(O)ₐ-, worin a für 0 bis 2 steht, -NR'-C(O)-O(C₁-C₆)-Alkyl, -NR'-SO₂- (C₁-C₆) -Alkyl, -NR'-C(O)NR'R'', -SO₂-NR'R", -C(O)-NR'R", Carbocyclyl, Heterocyclo, Heteroaryl oder Oxo steht;
R₂ für Wasserstoff, Halogen, Hydroxy, Nitro, Formyl, -CO₂H, -SH, Cyano, (C₁-C₆) -Alkyl, (C₂-C₆) - Alkenyl, (C₂-C₆) -Alkinyl, (C₃-C₆) -Cycloalkyl, -O-(C₃-C₆)-Cycloalkyl, -OC(O)-(C₁-C₆)-Alkyl, -C(O)-(C₁-C₆) -Alkyl, -CO₂-(C₁-C₆)-Alkyl, -NR'R", -NR'-C(O)-(C₁-C₆) -Alkyl, (C₁-C₆)-Alkyl-S(O)ₐ-, worin a für 0 bis 2 steht, -NR'-C(O)-O(C₁-C₆)-Alkyl, -NR'-SO₂-(C₁-C₆) -Alkyl, -NR'-C(O)NR'R'', -SO₂-NR'R'',-C(O)-NR'R'', -OC(O)-NR'R'', Carbocyclyl, Heterocyclo, Heteroaryl oder (C₁-C₆) -Alkoxy steht;
R₃ jeweils unabhängig voneinander für Halogen, Nitro, Formyl, Cyano, Hydroxy, -NR'R " , -CO₂H, -C (O) - (C₁-C₆) -Alkyl, -CO₂- (C₁-C₆) -Alkyl, -C (O) - NR'R'', -NR'-C (O) - (C₁-C₆) -Alkyl, -NR'-C (O) NR'R'', -NR'-C(O)-O(C₂-C₆)-Alkyl, -O-C (O) - (C₁-C₆) -Alkyl, -SH, -SO₂-NR'R" , (C₁-C₆) -Alkyl, (C₂-C₆)-Alkenyl, (C₂-C₆) -Alkinyl, (C₁-C₆) -Alkoxy, (C₁-C₆) -Alkyl-S (O) ₐ-, worin a für 0 bis 2 steht, -NR'-SO₂- (C₁-C₆) - Alkyl, Carbocyclyl, Heterocyclo oder Heteroaryl steht, wobei dann, wenn das Heterocyclo oder Heteroaryl eine -NH-Gruppierung enthält, dieser Stickstoff gegebenenfalls durch (C₁-C₆)-Alkyl substituiert sein kann; oder
zwei Gruppen R₃ an benachbarten Kohlenstoffatomen gegebenenfalls einen 5- oder 6-gliedrigen, gesättigten, teilweise ungesättigten, ungesättigten und/oder aromatischen Ring bilden können, der gegebenenfalls 0, 1 oder 2 unter S, O oder NRₐ, worin Rₐ fehlt oder für H oder (C₁-C₆) -Alkyl steht, ausgewählte Heteroatome enthält;
R₄ für Wasserstoff oder Halogen steht;
m für 0-3 steht; wobei die Werte von R₃ gleich oder verschieden sein können;
n für 0-3 steht; wobei die Werte von R₁ gleich oder verschieden sein können;
p jeweils unabhängig voneinander für 1 oder 2 steht und
R₅ unter (C₁-C₆) -Alkyl, (C₃-C₆) -Cycloalkyl, -C (O) - (C₁-C₆) -Alkyl, -S (O)ₚ- (C₁-C₆) -Alkyl, -CO₂- (C₁-C₆) - Alkyl, -C(O)-NR'R" , Benzyl, Benzyloxycarbonyl, Benzoyl und Phenylsulfonyl ausgewählt ist; R' und R " jeweils unabhängig voneinander für H, gegebenenfalls substituiertes (C₁-C₆)-Alkyl oder gegebenenfalls substituiertes Aryl stehen oder zusammen mit dem Stickstoff, an den sie gebunden sind, einen gegebenenfalls substituierten 3-6-gliedrigen, gesättigten oder teilweise ungesättigten Ring bilden, der 0 oder 1 zusätzliches unter NRₐ- ausgewähltes Heteroatom enthalten kann; wobei die fakultativen Substituenten unter einer oder mehreren Gruppen R₆ ausgewählt sein können;
R₆ unabhängig für (C₁-C₆) -Alkyl, Halogen- (C₁-C₆) - alkyl, Halogen, Nitro, Cyano, Hydroxy, (C₁-C₆)-Alkoxy, -NR^{x}R^{y}, -COOR^{x} oder -CONR^{x}R^{y} steht und
R^{x} und R^{y} unabhängig voneinander für Wasserstoff oder (C₁-C₆) -Alkyl stehen und wobei jedes Rₐ, R₁, R₂, R₃ und R₅ gegebenenfalls an Kohlenstoff durch ein oder mehrere Formyl, -SH, Azido, Halogen, Nitro, Cyano, Hydroxy, Trifluormethoxy, -OC (O) - (C₁-C₆) -Alkyl, -NR' R'' , -CO₂H, -C (O) - (C₁-C₆) -Alkyl, -CO₂- (C₁-C₆) -Alkyl, -C (O) - NR'R" , -S- (C₁-C₆) -Alkyl, -SOₚ- (C₁-C₆) -Alkyl, -SOₚNR'R" , (C₁-C₆) -Alkyl, (C₃-C₆)-Cycloalkyl, -NR' - C (O) - (C₁-C₆) -Alkyl, -NR'-C(O)-O-(C₁-C₆)-Alkyl, -NR' - SO₂-(C₁-C₆)-Alkyl, -NR' -C (O) NR' R'', (C₂-C₆) -Alkenyl, (C₂-C₆) -Alkinyl oder (C₁-C₆) -Alkoxy substituiert sein kann.

2. Verbindung der Formel IA nach Anspruch 1 oder ein pharmazeutisch annehmbares Salz davon.

3. Verbindung der Formel IB nach Anspruch 1 oder ein pharmazeutisch annehmbares Salz davon.

4. Verbindung der Formel IA oder IB oder ein pharmazeutisch annehmbares Salz davon nach einem der Ansprüche 1-3, wobei: 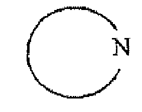 für einen 5-7-gliedrigen, über Stickstoff gebundenen Heterocyclus steht, wobei dann, wenn der Heterocyclus eine -NH-Gruppierung enthält, dieser Stickstoff gegebenenfalls durch eine unter R₅ ausgewählte Gruppe substituiert sein kann; wobei R₅ unter (C₁-C₆) -Alkyl, (C₃-C₆) -Cycloalkyl, -C (O) - (C₁-C₆) -Alkyl und -CO₂- (C₁-C₆) -Alkyl ausgewählt ist und
R₅ jeweils gegebenenfalls an Kohlenstoff durch ein oder mehrere Cyano, Hydroxy, -OC(O)-(C₁-C₆)-Alkyl, -NR'R" , (C₃-C₆) -Cycloalkyl oder (C₁-C₆) -Alkoxy substituiert sein kann, wobei
R' und R" jeweils unabhängig voneinander für (C₁-C₆) -Alkyl stehen.

5. Verbindung der Formel IA oder IB oder ein pharmazeutisch annehmbares Salz davon nach einem der Ansprüche 1-4, wobei R₁ jeweils für Hydroxy, -NR'R " oder Oxo steht; wobei R' und R " jeweils unabhängig voneinander für (C₁-C₆)-Alkyl stehen.

6. Verbindung der Formel IA oder IB oder ein pharmazeutisch annehmbares Salz davon nach einem der Ansprüche 1-5, wobei n für 0 oder 1 steht.

7. Verbindung der Formel IA oder IB oder ein pharmazeutisch annehmbares Salz davon nach einem der Ansprüche 1-6, wobei R₂ für Wasserstoff, Halogen oder (C₁-C₆) -Alkoxy steht; wobei R₂ gegebenenfalls an Kohlenstoff durch ein oder mehrere (C₁-C₆)-Alkoxy oder Hydroxy substituiert sein kann.

8. Verbindung der Formel IA oder IB oder ein pharmazeutisch annehmbares Salz davon nach einem der Ansprüche 1-7, wobei R₃ jeweils unabhängig voneinander für Halogen, (C₁-C₆) -Alkyl oder (C₁-C₆) - Alkoxy steht; wobei R₃ gegebenenfalls an Kohlenstoff durch Halogen substituiert sein kann.

9. Verbindung der Formel IA oder IB oder ein pharmazeutisch annehmbares Salz davon nach einem der Ansprüche 1-8, wobei m für 1-3 steht; wobei die Werte von R₃ gleich oder verschieden sein können.

10. Verbindung der Formel IA oder IB oder ein pharmazeutisch annehmbares Salz davon nach einem der Ansprüche 1-9, wobei R₄ für Wasserstoff oder Fluor steht.

11. Verbindung der Formel IA oder IB: 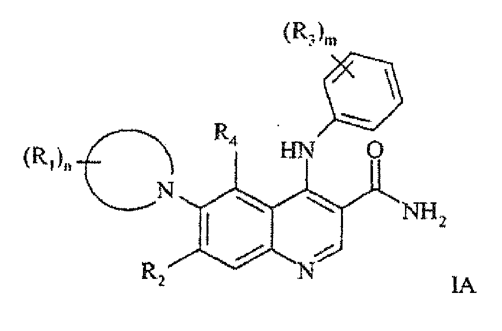 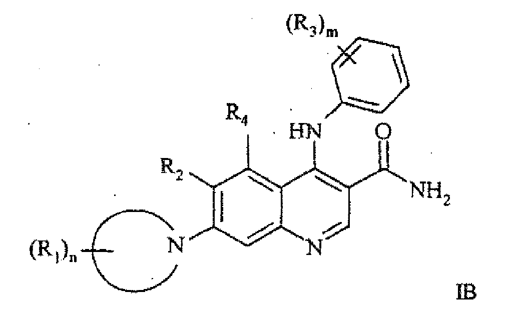 worin: 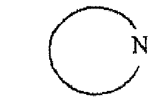 für Piperazin-1-yl, *N*-Methylpiperazin-1-yl, *N*-Ethylpiperazin-1-yl, *N*-Isopropylpiperazin-1-yl, *N*-Acetylpiperazin-1-yl, *N*-(2-Hydroxyacetyl)-piperazin-1-yl, *N*-(2-Dimethylaminoethyl)piperazin-1-yl, *N*-(2-Methoxyethyl)piperazin-1-yl, *N*-(2-Cyanoethyl)piperazin-1-yl, *N*-(2-Hydroxyethyl)-piperazin-1-yl, *N*-(Cyclopropylmethyl)piperazin-1-yl, N-(Cyclopropyl)piperazin-1-yl, *N*-((R)-2-Hydroxypropionyl)piperazin-1-yl, *N*-((*S*)-2-Hydroxypropionyl)piperazin-1-yl, *N*-(*t*-Butoxycarbonyl)piperazin-1-yl, *N*-(Acetoxyacetyl)-piperazin-1-yl, Piperidin-1-yl, Morpholino, Homo-piperazin-1-yl, *N*-Methylhomopiperazin-1-yl, *N-*Ethylhomopiperazin-1-yl, *N*-Acetylhomopiperazin-1-yl, *N*-Isopropylhomopiperazin-1-yl, *N*-Cyclopropylhomopiperazin-1-yl und Pyrrolidin-1-yl steht;
R₁ jeweils für Hydroxy, -NMe₂ oder Oxo steht;
n für 0 oder 1 steht;
R₂ für Wasserstoff, Fluor, Methoxy, Ethoxy, 2-(Methoxy)ethoxy, 2-Hydroxyethoxy oder Isopropoxy steht;
R₃ jeweils unabhängig voneinander für Fluor, Chlor, Methyl, Trifluormethyl, Ethyl, Methoxy oder Trifluormethoxy steht;
m für 1-3 steht; wobei die Werte von R₃ gleich oder verschieden sein können;
R₄ für Wasserstoff oder Fluor steht;
oder ein pharmazeutisch annehmbares Salz davon.

12. Verbindung der Formel IA oder IB: 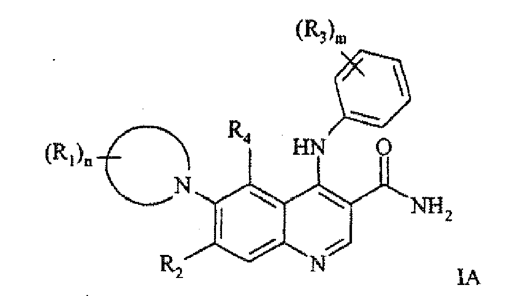 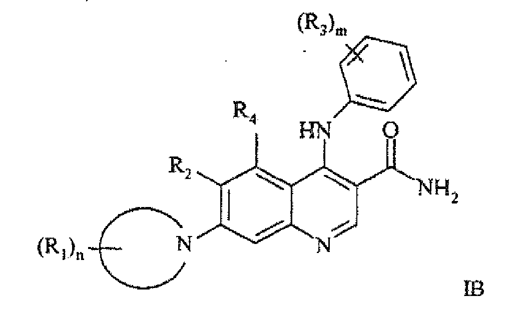 ausgewählt unter:
4-[(2,4-Difluorphenyl)amino]-7-ethoxy-6-(4-methylpiperazin-1-yl)chinolin-3-carboxamid;
4-[(2,3-Dichlorphenyl)amino]-7-ethoxy-6-(4-methylpiperazin-1-yl)chinolin-3-carboxamid;
7-Ethoxy-4-[(2-fluor-5-methylphenyl)amino]-6-(4-isopropylpiperazin-1-yl)chinolin-3-carboxamid;
4-[(3-Chlor-2-fluorphenyl)amino]-7-ethoxy-6-(4-methylpiperazin-1-yl)chinolin-3-carboxamid;
7-Ethoxy-4-[(2-fluor-5-methylphenyl)amino]-6-(4-methylpiperazin-1-yl)chinolin-3-carboxamid;
7-Ethoxy-4-[(2-fluor-4-methylphenyl)amino]-6-(4-methylpiperazin-1-yl)chinolin-3-carboxamid;
4-[(2,4-Difluorphenyl)amino]-7-(2-methoxyethoxy) - 6-(4-methylpiperazin-1-yl)chinolin-3-carboxamid;
4-[(2-Fluor-4-methylphenyl)amino]-7-(2-methoxyethoxy)-6-(4-methylpiperazin-1-yl)chinolin-3-carboxamid;
4-[(2-Fluor-5-methylphenyl)amino]-7-(2-methoxyethoxy)-6-(4-methylpiperazin-1-yl)chinolin-3-carboxamid und
4-[(2-Fluor-4-methylphenyl)amino]-6-(4-isopropylpiperazin-1-yl)-7-(2-methoxyethoxy)chinolin-3-carboxamid.

13. Verbindung der Formel IA oder IB nach Anspruch 12, wobei es sich bei der Verbindung um 4-[(2,4-Difluorphenyl)amino]-7-ethoxy-6-(4-methylpiperazin-1-yl)chinolin-3-carboxamid handelt.

14. Verfahren zur Herstellung einer Verbindung der Formel IA oder IB oder eines pharmazeutisch annehmbaren Salzes davon nach Anspruch 1, bei dem man:
*Verfahren a)* eine Verbindung der Formel IIA oder IIB: 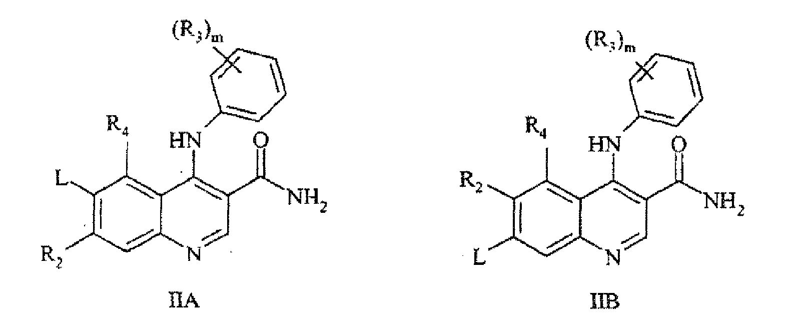 worin L für ein austauschbares Atom oder eine austauschbare Gruppe steht, mit einer Verbindung der Formel III: 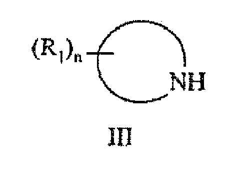 umsetzt oder
*Verfahren b)* eine Verbindung der Formel IVA oder IVB: 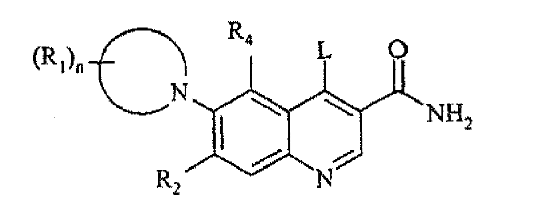 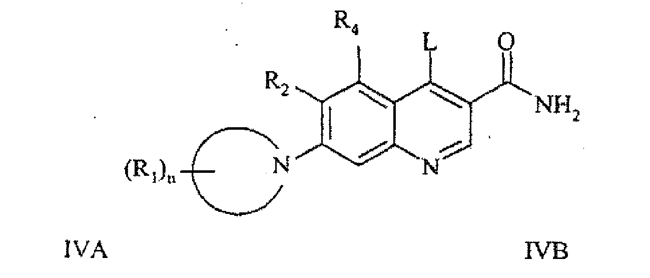 worin L für ein austauschbares Atom oder eine austauschbare Gruppe steht, mit einer Verbindung der Formel V: 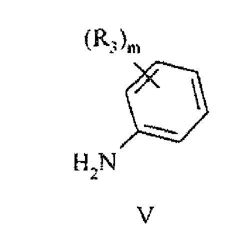 umsetzt oder
*Verfahren* c) eine Verbindung der Formel VIA oder VIB: 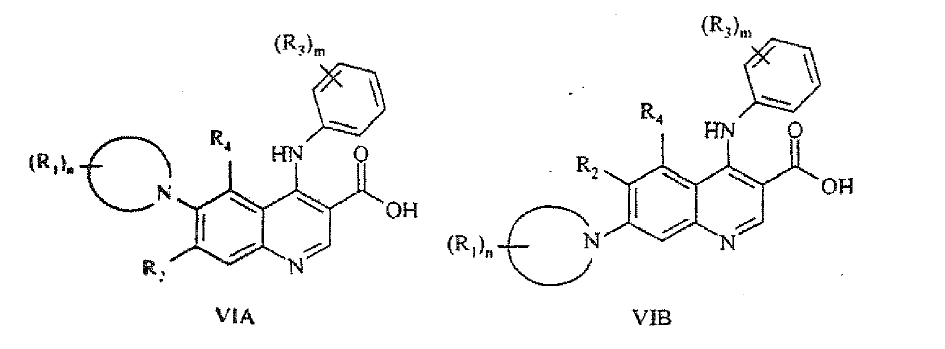 oder ein aktiviertes Derivat davon mit Ammoniak umsetzt oder
*Verfahren d)* eine Verbindung der Formel VIIA oder VIIB: 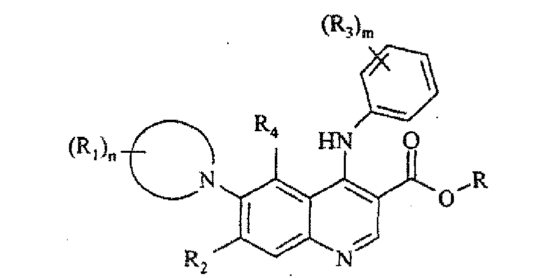 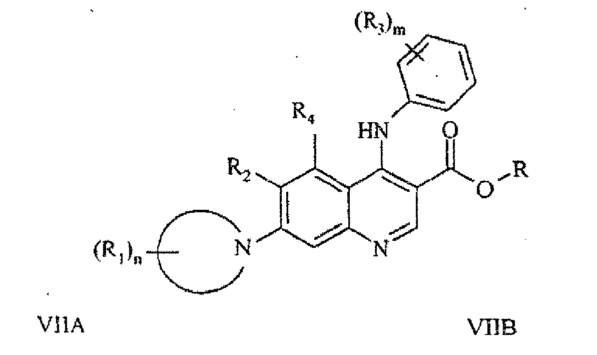 worin R für (C₁-C₆)-Alkyl, insbesondere Methyl und Ethyl, steht, mit Formamid und einer Base umsetzt oder
*Verfahren e)* eine Verbindung der Formel VIIIA oder VIIIB: 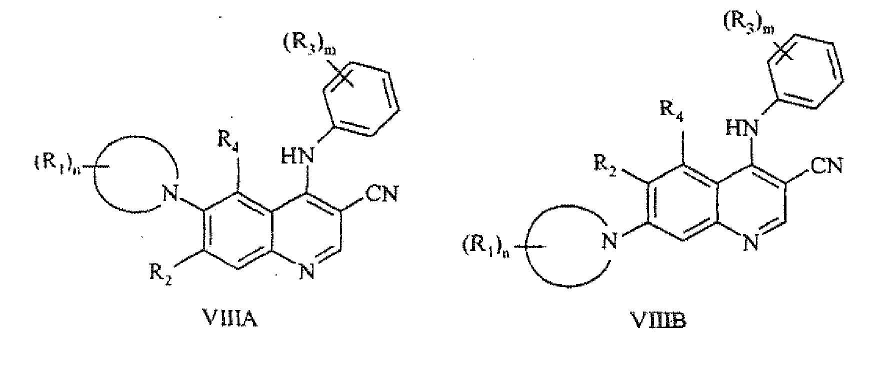 hydrolysiert
und danach gegebenenfalls:
i) eine Verbindung der Formel IA oder IB in eine andere Verbindung der Formel IA oder IB umwandelt;
ii) jegliche Schutzgruppen abspaltet;
iii) ein pharmazeutisch annehmbares Salz bildet;
wobei variable Gruppen wie in Anspruch 1 definiert sind, sofern nicht anders angegeben.

15. Pharmazeutische Zusammensetzung, die eine Verbindung der Formel IA oder IB oder ein pharmazeutisch annehmbares Salz davon nach einem der Ansprüche 1-12 zusammen mit einem pharmazeutisch annehmbaren Verdünnungsmittel oder Träger enthält.

16. Verbindung der Formel IA oder IB oder ein pharmazeutisch annehmbares Salz davon nach einem der Ansprüche 1-12 zur Verwendung als Arzneimittel.

## Revendications

1. Composé de formule IA ou IB : 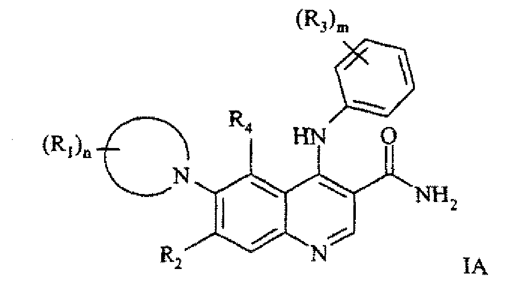 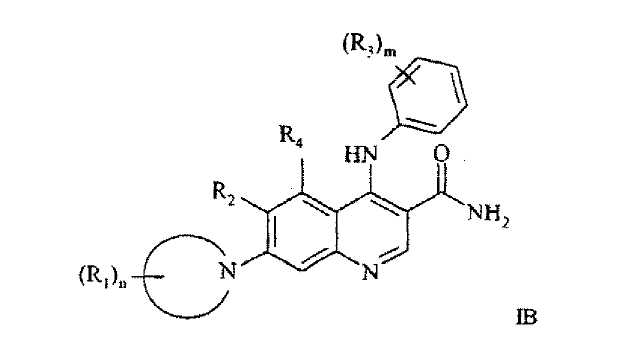 ou un sel pharmaceutiquement acceptable de celui-ci,
dans lesquelles : 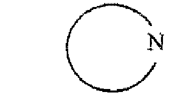 est un hétérocycle ou hétéroaryle lié par l'azote à 3-10 chaînons ; où si ledit hétérocycle ou hétéroaryle contient un motif -NH-, cet azote peut être éventuellement substitué par un groupement choisi parmi R₅ ;
**R₁** à chaque apparition est indépendamment halogéno, hydroxy, nitro, formyle, cyano, -CO₂H, -SH, (C₁-C₆) alkyle, (C₂-C₆) alcényle, (C₂-C₆) alcynyle, (C₃-C₆) cycloalkyle, (C₁-C₆) alcoxy, -OC(O)-(C₁-C₆) alkyle, -C(O)-(C₁-C₆)alkyle, -CO₂(C₁-C₆)alkyle, -NR'R'', -NR'-C(O)-(C₁-C₆)alkyle, (C₁-C₆)alkylS(O)ₐ- où a est 0 à 2, -NR'-C(O)-O(C₁-C₆)alkyle, -NR'-SO₂-(C₁-C₆)alkyle, -NR' - C(O)NR'R", -SO₂-NR'R", -C(O)-NR'R'', carbocyclyle, hétérocyclo, hétéroaryle ou oxo ;
**R₂** est hydrogène, halogéno, hydroxy, nitro, formyle, -CO₂H, -SH, cyano, (C₁-C₆) alkyle, (C₂-C₆) alcényle, (C₂-C₆) alcynyle, (C₃-C₆) cycloalkyle, O-(C₃-C₆) cycloalkyle, -OC(O)-(C₁-C₆)alkyle, -C(O)-(C₁-C₆) alkyle, -CO₂(C₁-C₆)alkyle, -NR'R'', -NR'-C(O)-(C₁-C₆)alkyle, (C₁-C₆)alkylS(O)ₐ- où a est 0 à 2, -NR'-C(O)-O(C₁-C₆)alkyle, -NR'-SO₂-(C₁-C₆) alkyle, -NR'-C(O)NR'R'', -SO₂NR'R", -C(O)-NR'R", -OC(O)-NR'R'', carbocyclyle, hétérocyclo, hétéroaryle ou (C₁-C₆)alcoxy ;
**R₃** à chaque apparition est indépendamment halogéno, nitro, formyle, cyano, hydroxy, -NR'R " , -CO₂H, -C(O)-(C₁-C₆)alkyle, -CO₂(C₁-C₆)alkyle, -C(O)-NR'R'', -NR'-C(O)-(C₁-C₆) alkyle, -NR'-C(O)NR'R'', -NR'-C(O)-O(C₁-C₆)alkyle, -O-C(O) - (C₁-C₆)alkyle, -SH, -SO₂-NR'R'', (C₁-C₆)alkyle, (C₂-C₆)alcényle, (C₂-C₆)alcynyle, (C₁-C₆) alcoxy, (C₁-C₆)alkylS(O)ₐ- où a est 0 à 2, -NR'-SO₂-(C₁-C₆)alkyle, carbocyclyle, hétérocyclo, ou hétéroaryle, où si ledit hétérocyclo ou hétéroaryle contient un motif -NH-, cet azote peut être éventuellement substitué par (C₁-C₆)alkyle ; ou
deux groupements R₃ sur des carbones adjacents peuvent éventuellement former un cycle saturé, partiellement insaturé, insaturé et/ou aromatique à 5 ou 6 chaînons contenant éventuellement 0, 1 ou 2 hétéroatomes choisis parmi S, O ou NRₐ où Rₐ est absent, H ou (C₁-C₆) alkyle ;
**R₄** est hydrogène ou halogéno ;
**m** est 0-3 ; où les valeurs de R₃ peuvent être identiques ou différentes ;
**n** est 0-3 ; où les valeurs de R₁ peuvent être identiques ou différentes ;
**p** est indépendamment 1 ou 2 à chaque apparition ;
et
**R₅** est choisi parmi (C₁-C₆) alkyle, (C₃-C₆)cycloalkyle, -C(O)-(C₁-C₆)alkyle, -S(O)ₚ(C₁-C₆)alkyle, -CO₂(C₁-C₆)alkyle, -C(O)-NR'R'', benzyle, benzyloxycarbonyle, benzoyle et phénylsulfonyle ;
**R'** et **R''** indépendamment à chaque apparition sont H, (C₁-C₆)alkyle éventuellement substitué, ou aryle éventuellement substitué ou pris ensemble avec l'azote auquel ils sont liés forment un cycle à 3-6 chaînons éventuellement substitué, saturé ou partiellement insaturé, contenant 0 ou 1 hétéroatome supplémentaire choisi parmi NRₐ ; où lesdits substituants éventuels peuvent être choisis parmi un ou plusieurs R₆ ;
**R₆** peut être indépendamment (C₁-C₆)alkyle, halogéno(C₁-C₆) alkyle, halogéno, nitro, cyano, hydroxy, (C₁-C₆)alcoxy, -NR^{x}R^{y}, -COOR^{x} ou -CONR^{x}R^{y} ; et
**R^{x}** et **R^{y}** sont indépendamment l'un de l'autre hydrogène ou (C₁-C₆) alkyle ; et où
chaque **Rₐ, R₁, R₂, R₃** et **R₅** peut être éventuellement substitué sur le carbone par un ou plusieurs formyle, -SH, azido, halogéno, nitro, cyano, hydroxy, trifluorométhoxy, -OC(O)-(C₁-C₆)alkyle, -NR'R'', -CO₂H, -C(O)-(C₁-C₆) alkyle, -CO₂(C₁-C₆)alkyle, -C(O)-NR'R'', -S-(C₁-C₆)alkyle, -SOₚ- (C₁-C₆) alkyle, -SOₚNR'R'', (C₁-C₆)alkyle, (C₃-C₆) cycloalkyle, -NR'-C(O) - (C₁-C₆) alkyle, -NR'-C(O)-O(C₁-C₆)alkyle, -NR'-SO₂-(C₁-C₆) alkyle, -NR' - C(O)NR'R'', (C₂-C₆)alcényle, (C₂-C₆) alcynyle ou (C₁-C₆)alcoxy.

2. Composé de formule IA selon la revendication 1 ou un sel pharmaceutiquement acceptable de celui-ci.

3. Composé de formule IB selon la revendication 1 ou un sel pharmaceutiquement acceptable de celui-ci.

4. Composé de formule IA ou IB ou un sel pharmaceutiquement acceptable de celui-ci selon l'une quelconque des revendications 1-3, **caractérisé en ce que** : 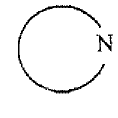 est un hétérocycle lié par l'azote, à 5-7 chaînons ; où si ledit hétérocycle contient un motif -NH-, cet azote peut être éventuellement substitué par un groupement choisi parmi R₅ ; où
R₅ est choisi parmi (C₁-C₆) alkyle, (C₃-C₆) cycloalkyle, -C(O)-(C₁-C₆) alkyle et -CO₂ (C₁-C₆) alkyle ; et
chaque R₅ peut être éventuellement substitué sur le carbone par un ou plusieurs cyano, hydroxy, -OC(O)-(C₁-C₆) alkyle, -NR'R'', (C₃-C₆)cycloalkyle ou (C₁-C₆)alcoxy ; où
R' et R'' indépendamment à chaque apparition sont (C₁-C₆) alkyle.

5. Composé de formule IA ou IB ou un sel pharmaceutiquement acceptable de celui-ci selon l'une quelconque des revendications 1-4, **caractérisé en ce que** R₁ à chaque apparition est hydroxy, -NR'R'' ou oxo ; où R' et R'' indépendamment à chaque apparition sont (C₁-C₆) alkyle.

6. Composé de formule IA ou IB ou un sel pharmaceutiquement acceptable de celui-ci selon l'une quelconque des revendications 1-5, **caractérisé en ce que** n est 0 ou 1.

7. Composé de formule IA ou IB ou un sel pharmaceutiquement acceptable de celui-ci selon l'une quelconque des revendications 1-6, **caractérisé en ce que** R₂ est hydrogène, halogéno ou (C₁-C₆)alcoxy ; où R₂ peut être éventuellement substitué sur le carbone par un ou plusieurs (C₁-C₆) alcoxy ou hydroxy.

8. Composé de formule IA ou IB ou un sel pharmaceutiquement acceptable de celui-ci selon l'une quelconque des revendications 1-7, **caractérisé en ce que** R₃ à chaque apparition est indépendamment halogéno, (C₁-C₆) alkyle ou (C₁-C₆) alcoxy ; où R₃ peut être éventuellement substitué sur le carbone par halogéno.

9. Composé de formule IA ou IB ou un sel pharmaceutiquement acceptable de celui-ci selon l'une quelconque des revendications 1-8, **caractérisé en ce que** m est 1-3 ; où les valeurs de R₃ peuvent être identiques ou différentes.

10. Composé de formule IA ou IB ou un sel pharmaceutiquement acceptable de celui-ci selon l'une quelconque des revendications 1-9, où R₄ est hydrogène ou fluoro.

11. Composé de formule IA ou IB : 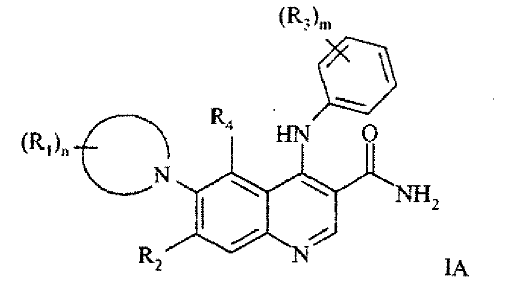 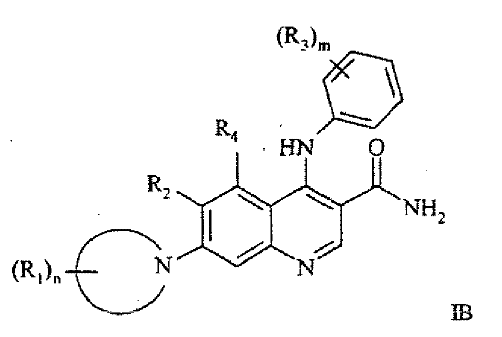 dans lesquelles : 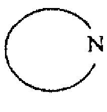 est pipérazin-1-yle, *N*-méthylpipérazin-1-yle, *N*-éthylpipérazin-1-yle, *N*-isopropylpipérazin-1-yle, *N*-acétylpipérazin-1-yle, *N*-(2-hydroxyacétyl)pipérazin-1-yle, *N*-(2-diméthylaminoéthyl)pipérazin-1-yle, *N*-(2-méthoxyéthyl)pipérazin-1-yle, *N*-(2-cyanoéthyl)pipérazin-1-yle, *N*-(2-hydroxyéthyl)pipérazin-1-yle, *N*-(cyclopropylméthyl)pipérazin-1-yle, *N*-(cyclopropyl)pipérazin-1-yle, *N*-((R)-2-hydroxypropionyl)pipérazin-1-yle, *N*-((*S*)-2-hydroxypropionyl)pipérazin-1-yle, *N*-(*t-*butoxycarbonyl)pipérazin-1-yle, *N*-(acétoxyacétyl)pipérazin-1-yle, pipéridin-1-yle, morpholino, homopipérazin-1-yle, *N*-méthylhomopipérazin-1-yle, *N*-éthylhomopipérazin-1-yle, *N*-acétylhomopipérazin-1-yle, *N*-isopropylhomopipérazin-1-yle, *N*-cyclopropylhomopipérazin-1-yle et pyrrolidin-1-yle ;
R₁ à chaque apparition est hydroxy, -NMe₂ ou oxo ;
n est 0 ou 1 ;
R₂ est hydrogène, fluoro, méthoxy, éthoxy, 2-(méthoxy)éthoxy, 2-hydroxyéthoxy ou isopropoxy ;
R₃ à chaque apparition est indépendamment fluoro, chloro, méthyle, trifluorométhyle, éthyle, méthoxy ou trifluorométhoxy ;
m est 1-3 ; où les valeurs de R₃ peuvent être identiques ou différentes ;
R₄ est hydrogène ou fluoro ;
ou un sel pharmaceutiquement acceptable de celui-ci.

12. Composé de formule IA ou IB : 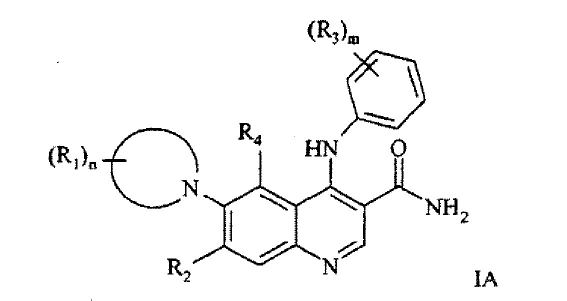 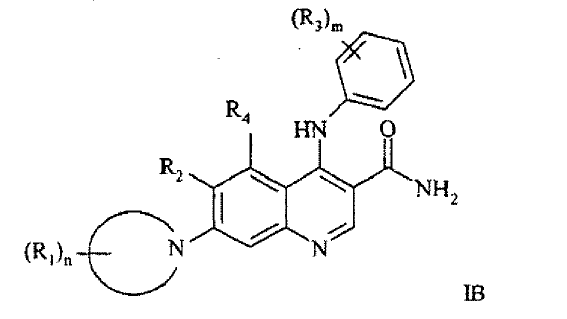 choisi parmi :
le 4-[(2,4-difluorophényl)amino]-7-éthoxy-6-(4-méthylpipérazin-1-yl)quinoléine-3-carboxamide ;
le 4-[(2,3-dichlorophényl)amino]-7-éthoxy-6-(4-méthylpipérazin-1-yl)quinoléine-3-carboxamide ;
le 7-éthoxy-4-[(2-fluoro-5-méthylphényl)amino]-6-(4-isopropylpipérazin-1-yl)quinoléine-3-carboxamide;
le 4-[(3-chloro-2-fluorophényl)amino]-7-éthoxy-6-(4-méthylpipérazin-1-yl)quinoléine-3-carboxamide ;
le 7-éthoxy-4-[(2-fluoro-5-méthylphényl)amino]-6-(4-méthylpipérazin-1-yl)quinoléine-3-carboxamide ;
le 7-éthoxy-4-[(2-fluoro-4-méthylphényl)amino]-6-(4-méthylpipérazin-1-yl)quinoléine-3-carboxamide ;
le 4-[(2,4-difluorophényl)amino]-7-(2-méthoxyéthoxy)-6-(4-méthylpipérazin-1-yl)quinoléine-3-carboxamide ;
le 4-[(2-fluoro-4-méthylphényl)amino]-7-(2-méthoxyéthoxy)-6-(4-méthylpipérazin-1-yl)quinoléine-3-carboxamide ;
le 4-[(2-fluoro-5-méthylphényl)amino]-7-(2-méthoxyéthoxy)-6-(4-méthylpipérazin-1-yl)quinoléine-3-carboxamide ; et
le 4-[(2-fluoro-4-méthylphényl)amino]-6-(4-isopropylpipérazin-1-yl)-7-(2-méthoxyéthoxy)quinoléine-3-carboxamide.

13. Composé de formule IA ou IB selon la revendication 12, **caractérisé en ce que** le composé est le 4-[(2,4-difluorophényl)amino]-7-éthoxy-6-(4-méthylpipérazin-1-yl)quinoléine-3-carboxamide.

14. Procédé de préparation d'un composé de formule IA ou IB ou d'un sel pharmaceutiquement acceptable de celui-ci, selon la revendication 1, lequel procédé, où les groupements variables sont, sauf précision contraire, tels que définis dans la revendication 1, comprend :
*Procédé a)* la réaction d'un composé de formule IIA ou IIB : 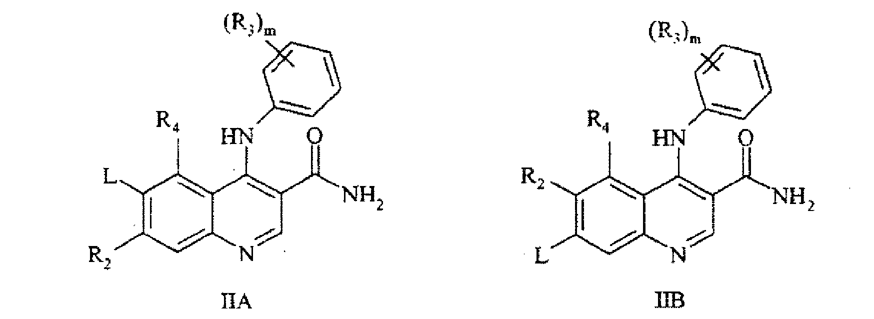 dans lesquelles L est un atome ou groupement déplaçable ; avec un composé de formule III : 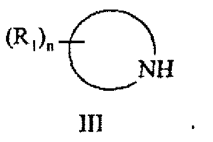 ou
*Procédé b)* la réaction d'un composé de formule IVA ou IVB : 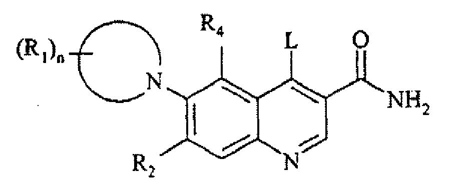 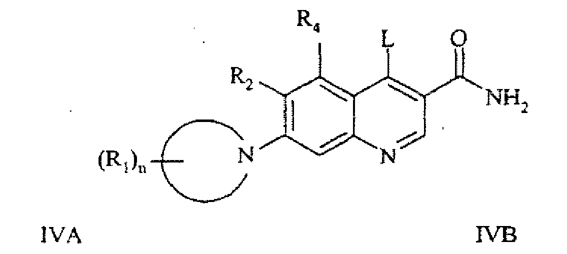 dans lesquelles L est un atome ou groupement déplaçable ; avec un composé de formule V : 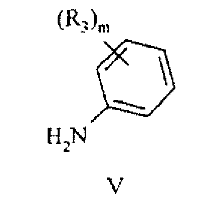 ou
*Procédé c)* la réaction d'un composé de formule VIA ou VIB : 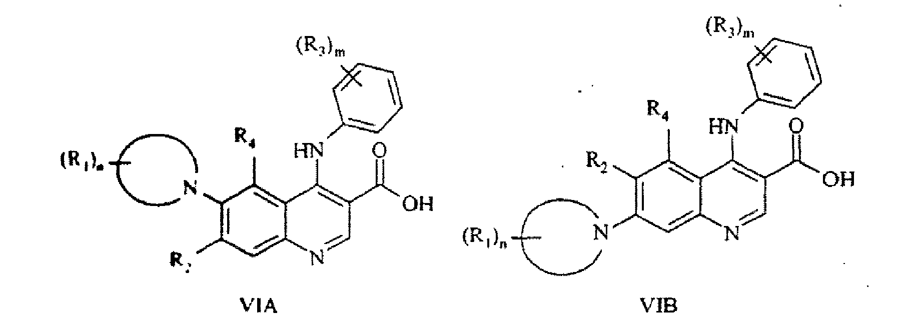 ou d'un dérivé activé de celui-ci ; avec de l'ammoniac ;
ou
*Procédé d)* la réaction d'un composé de formule VIIA ou VIIB : 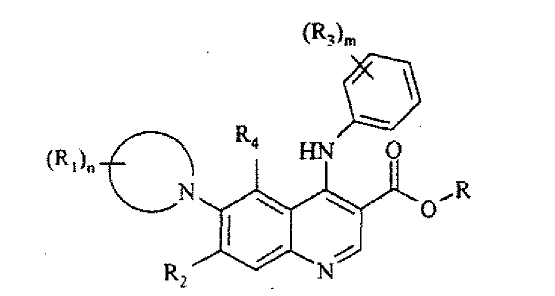 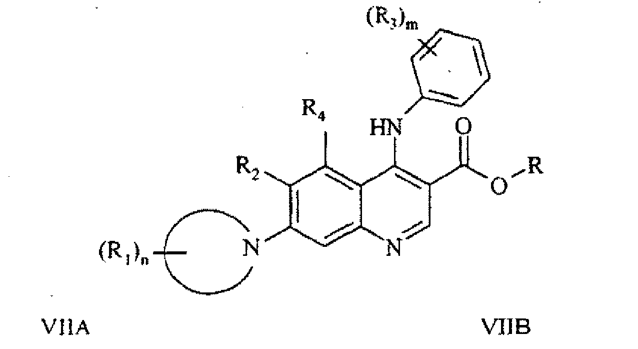 dans lesquelles R est (C₁-C₆)alkyle, en particulier méthyle ou éthyle ; avec du formamide et une base ;
ou
*Procédé e)* l'hydrolyse d'un composé de formule VIIIA ou VIIIB : 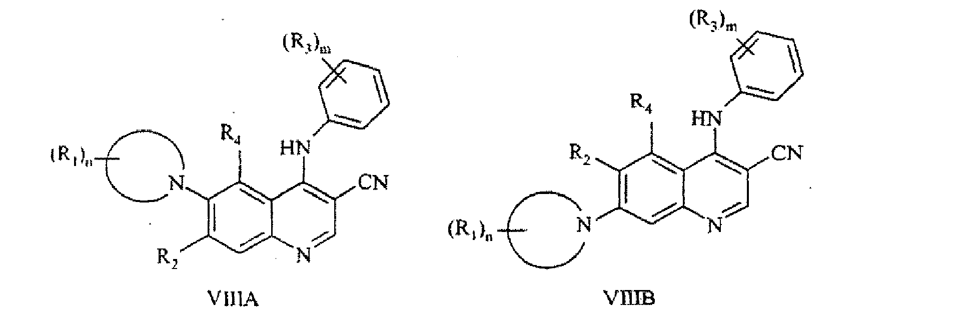 et ensuite le cas échéant :
i) la transformation d'un composé de formule IA ou IB en un autre composé de formule IA ou IB ;
ii) l'élimination de tous groupements protecteurs ;
iii) la formation d'un sel pharmaceutiquement acceptable.

15. Composition pharmaceutique, **caractérisée en ce qu'**elle comprend un composé de formule IA ou IB, ou un sel pharmaceutiquement acceptable de celui-ci, selon l'une quelconque des revendications 1-13, en association avec un diluant ou un support pharmaceutiquement acceptable.

16. Composé de formule IA ou IB, ou un sel pharmaceutiquement acceptable de celui-ci, selon l'une quelconque des revendications 1-13, destiné à être utilisé comme médicament.
